# EUROPEAN PATENT SPECIFICATION

(11) **EP 4 003 158 B1**
(45) Date of publication and mention of the grant of the patent: **11.06.2025**
(21) Application number: 20757995.4
(22) Date of filing: 24.07.2020
(51) Int. Cl.: A61B 5/0205, A61B 5/11, A61B 5/00, A61B 5/08, A61N 1/36

(54) **SLEEP DETECTION FOR SLEEP DISORDERED BREATHING (SDB) CARE**
SCHLAFERKENNUNG ZUR BEHANDLUNG VON SCHLAFATMUNGSSTÖRUNGEN (SDB)
DÉTECTION DU SOMMEIL POUR LA PRISE EN CHARGE D'UN TROUBLE RESPIRATOIRE DU SOMMEIL (TRS)

(30) Priority: 25.07.2019 US 201962878568 P
(43) Date of publication of application: 01.06.2022
(60) Divisional application: 25174255.7
(73) Proprietor: Inspire Medical Systems, Inc., Golden Valley, MN 55416 (US)
(72) Inventor: RONDONI, John, Golden Valley, Minnesota 55416 (US); DIEKEN, David, Golden Valley, Minnesota 55416 (US); THORP, Christopher, Golden Valley, Minnesota 55416 (US)
(74) Representative: Zimmermann & Partner Patentanwälte mbB
(86) International application number: PCT/US2020/043493
(87) International publication number: WO 2021/016558

(56) References cited:
- EP-A2- 1 938 862
- US-A1- 2005 061 320
- US-A1- 2015 224 307

## Description

A significant portion of the population suffers from various forms of sleep disordered breathing SDB. Devices for monitoring and treating sleep disordered breathing are known for example from US2015/224307, which describes systems and methods of detecting and treating obstructive sleep apnea and from US2005/061320 and EP1938862, which both describe a coordinated use of respiratory and cardiac therapies for sleep disordered breathing. In some patients, external breathing therapy devices and/or mere surgical interventions may fail to treat the sleep disordered breathing behavior.

### Brief Description of the Drawings

FIG. 1 is a flow diagram schematically representing an example method of determining a sleep-wake status.
FIG. 2A is a diagram schematically representing an example method of sensing physiologic information via sensing motion.
FIG. 2B is a diagram schematically representing an example method including sensing motion regarding a blood vessel.
FIGS. 3A, 3B are diagrams schematically representing an example method of determining a sleep-wake status relative to posture information.
FIG. 3C is a diagram schematically representing an example method of determining a sleep-wake status regarding different example sensed physiologic parameters.
FIG. 4A is a flow diagram schematically representing an example method of detecting sleep and/or maintaining stimulation therapy.
FIG. 4B is a diagram schematically representing an example method of detecting sleep.
FIG. 4C is a diagram schematically representing an example method including distinguishing body motion, posture, etc.
FIGS. 5, 6, 7, 8 are diagrams schematically representing an example method of determining a sleep-wake status relative to respiratory phase information.
FIG. 9 is a flow diagram schematically representing an example method of determining a sleep-wake status regarding variability in respiratory signals and/or cardiac cycles.
FIGS. 10-14 are diagrams schematically representing an example method of determining a sleep-wake status relative to various example cardiac cycle features, cardiac waveform morphology, etc..
FIGS. 15A-15B are diagrams schematically representing an example method of determining a sleep-wake status relative to example motion information.
FIGS. 15C-15F are diagrams schematically representing an example method of determining a sleep-wake status relative to various example cardiac cycle information.
FIGS. 16A-16B are diagrams schematically representing an example method of determining a sleep-wake status via identifying variability in sensed physiologic information relative to a threshold.
FIGS. 17 and 18 are diagrams schematically representing an example method of determining a sleep-wake status via tracking parameters relating to time, activity, non-movement parameters, etc.
FIG. 19 is a diagram schematically representing an example method of determining a sleep-wake status according to a probability of sleep and/or a probability of wakefulness.
FIG. 20A is a diagram schematically representing an example method of determining a sleep-wake status regarding taking an action based on a probability of sleep and/or a probability of wakefulness.
FIGS. 20B-20H are diagrams schematically representing an example taking an action, in relation to a method of determining a sleep-wake status, including initiating or terminating stimulation, relative to various example boundaries regarding time, temperature, sleep stages, etc.
FIG. 20I is a diagram schematically representing an example method of receiving inputs regarding some of the example boundaries represented in at least FIGS. 20B-20H.
FIG. 21 is a diagram schematically representing an example method of determining a sleep-wake status including dividing sense signal(s) to enable assessing different sleep-wake determination parameters.
FIG. 22 is a diagram schematically representing an example method of determining a sleep-wake status according to a probability of sleep and/or a probability of wakefulness.
FIG. 23A, 23B are diagrams schematically representing an example method of determining a sleep-wake status according to wakefulness information and snoring information, respectively.
FIG. 24 is a block diagram schematically representing an example sensing portion of an example device and/or used as part of example method for determining a sleep-wake status.
FIG. 26 is a block diagram schematically representing an example processing portion, which may form part of and/or be in communication with the example sensing portion.

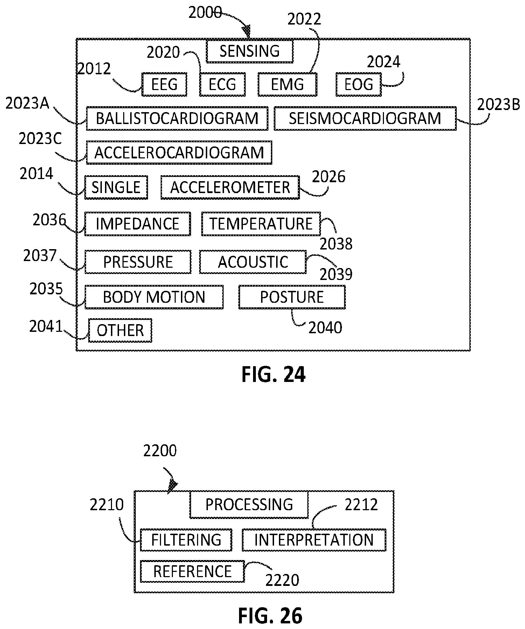

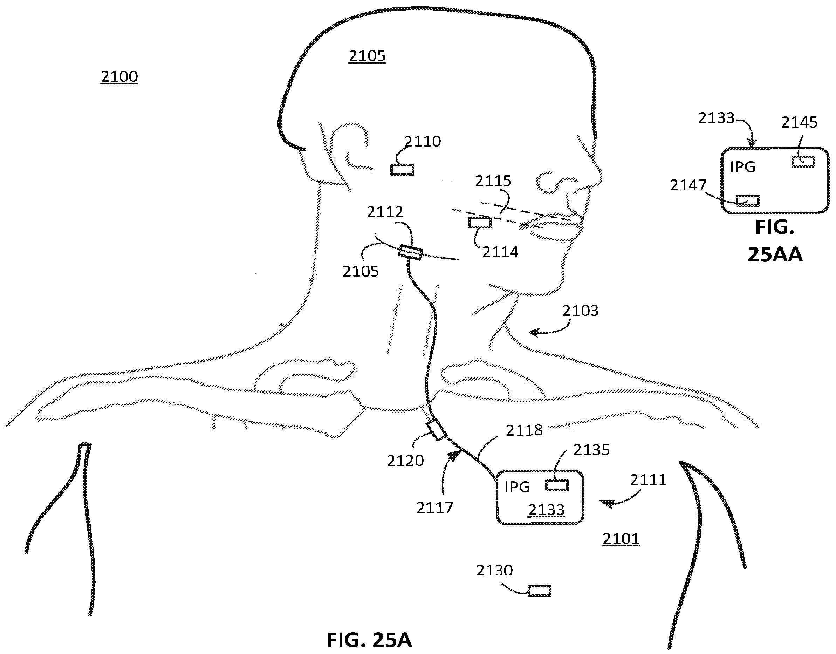

FIG. 25A is a diagram including a front view schematically representing a patient's body and example implanted medical device for treating sleep disordered breathing and/or determining a sleep-wake status.
FIG. 25AA is a diagram including a front view schematically representing an example implantable medical device with sensors.

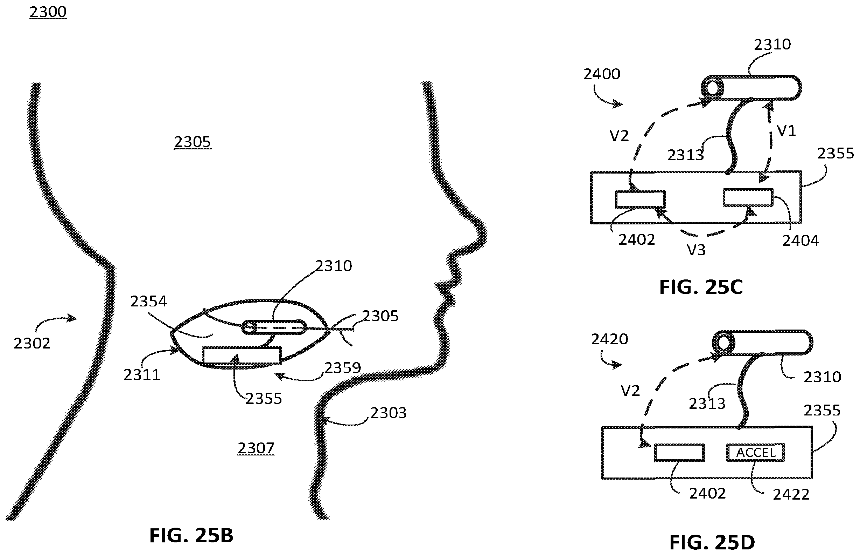

FIG. 25B-25F is a diagram schematically representing different example implementations of an example implantable medical device as a microstimulator implanted in a head-and-neck region.

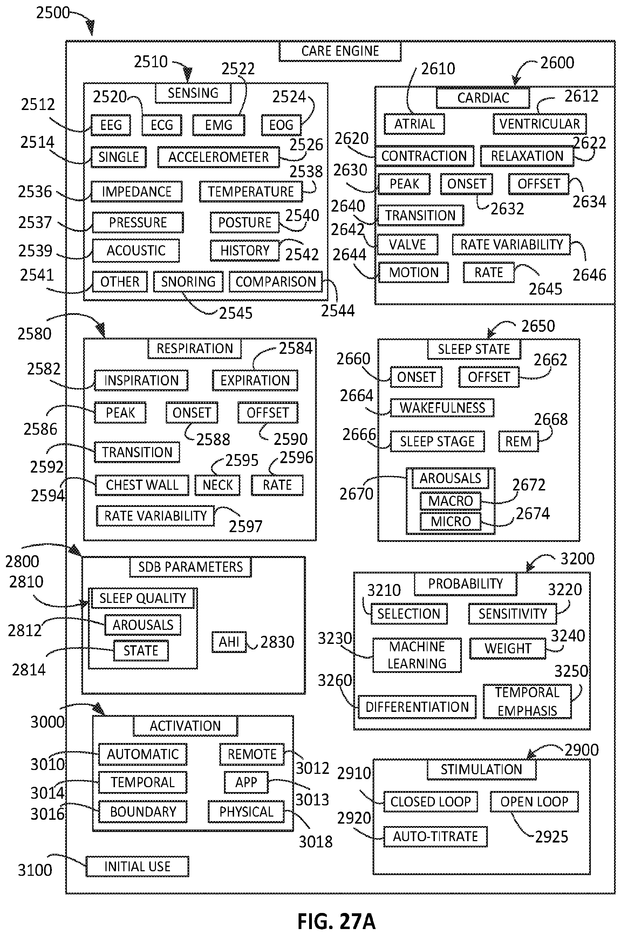

FIG. 27A is a block diagram schematically representing an example care engine.

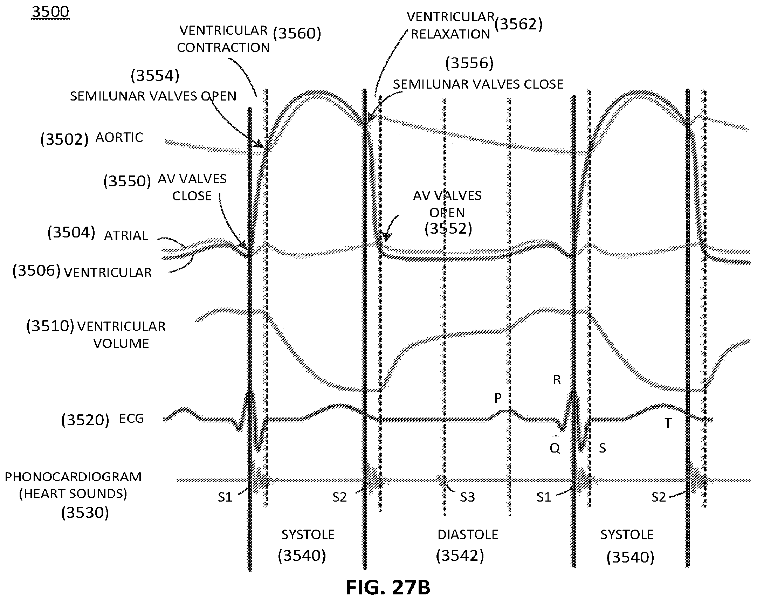

FIG. 27B is a diagram including a graph schematically representing example cardiac waveform morphology.

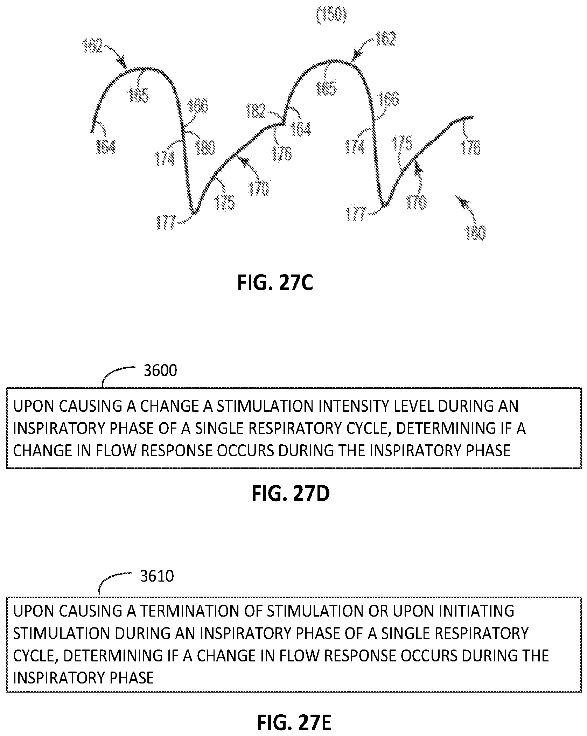

FIG. 27C is a diagram including a graph schematically representing example respiratory waveform morphology.
FIGS. 27D and 27E are diagrams schematically representing an example method of determining a sleep-wake status including determining a change in flow response in relation to initiating, changing, or terminating stimulation.

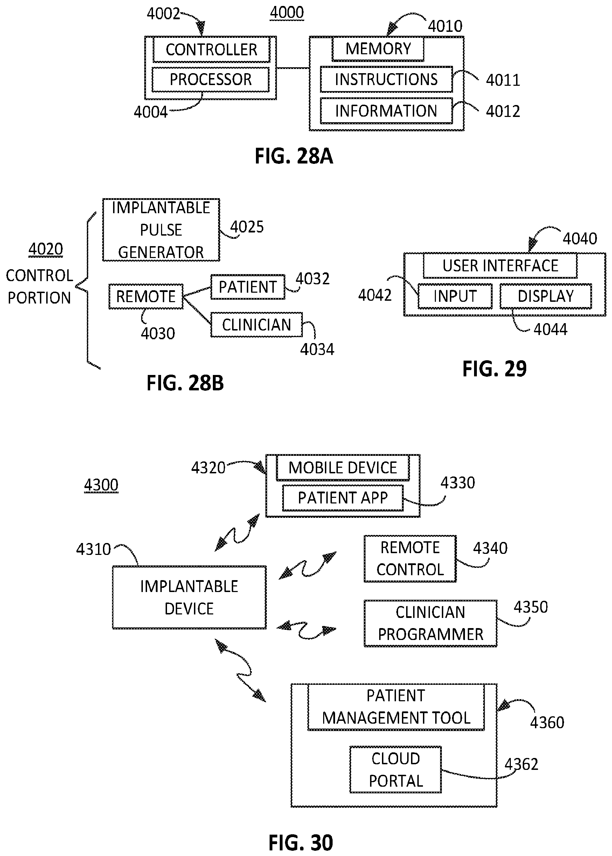

FIGS. 28A and 28B are block diagrams schematically representing an example control portions.
FIG. 29 is a block diagram schematically representing an example user interface.
FIG. 30 is a block diagram schematically representing example communication arrangements between an implantable medical device and external devices.

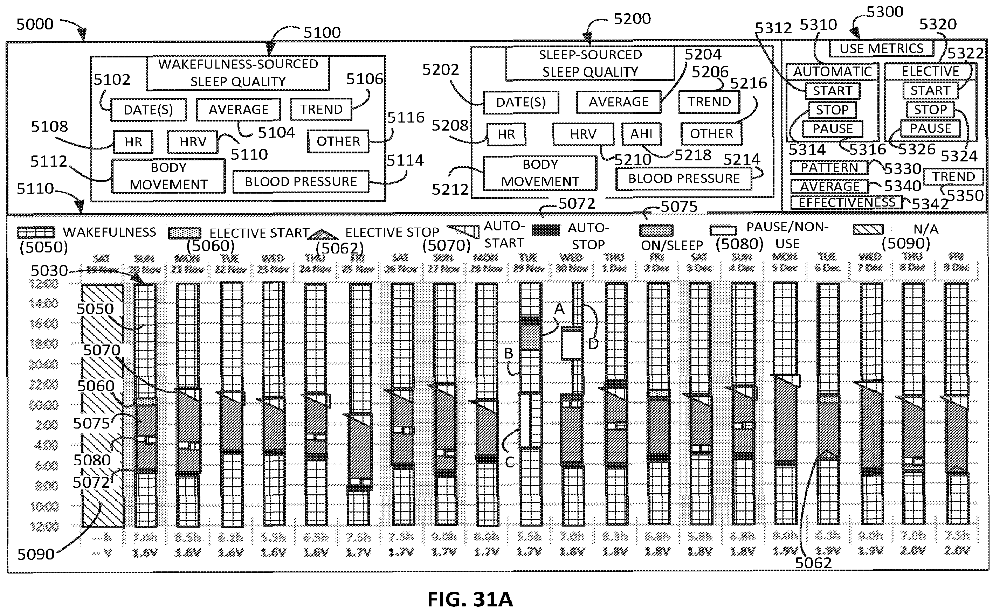

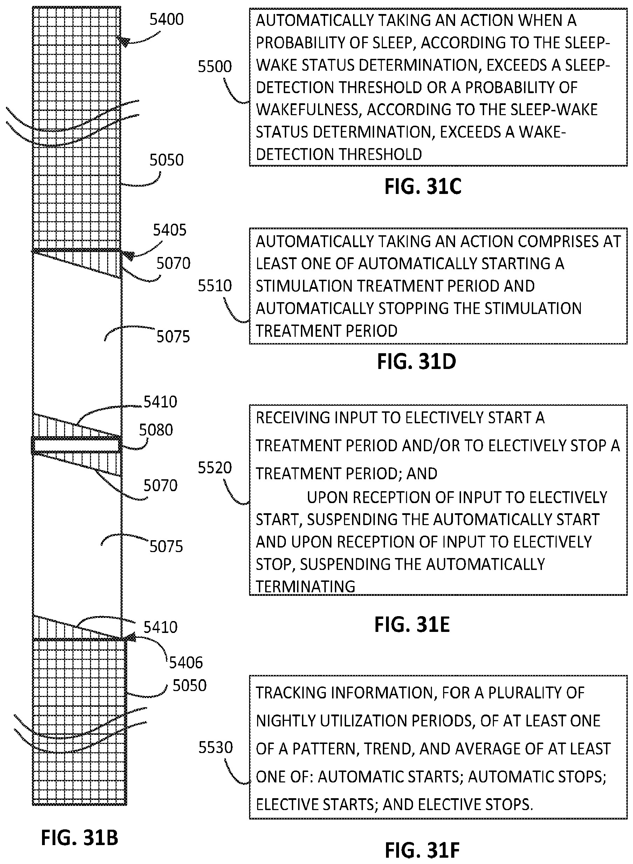

FIGS. 31A, 31B are diagrams schematically representing an example user interface including example therapy usage patterns, sleep-wake status, sleep quality portions, use metrics, etc., which may be used in association with example method and/or example device for determining sleep-wake status.
FIGS. 31C-31D are diagrams schematically representing an example methods including taking an action in relation to a sleep-wake status determination.
FIG. 31E is a diagram schematically representing an example method of receiving input in relation to starting and/or stopping therapeutic treatment.
FIG. 31F is a diagram schematically representing an example method including tracking information regarding usage, starting, stopping, etc. of a therapy.

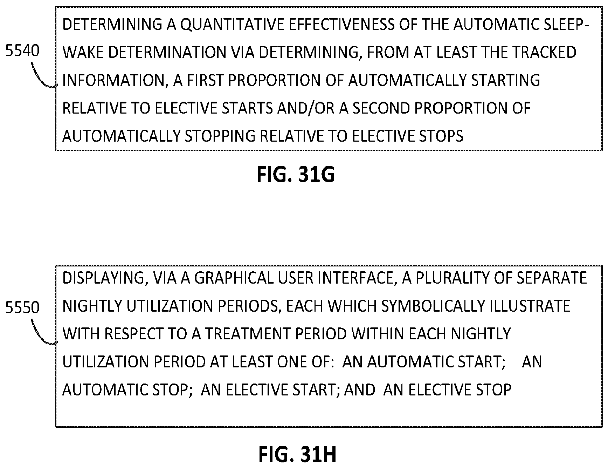

FIG. 31G is a diagram schematically representing an example method of determining an effectiveness of automatic sleep-wake determination.
FIG. 31H is a diagram schematically representing an example method and/or example device for displaying, via a graphical user interface, regarding usage, starting, stopping, etc. of a therapy.

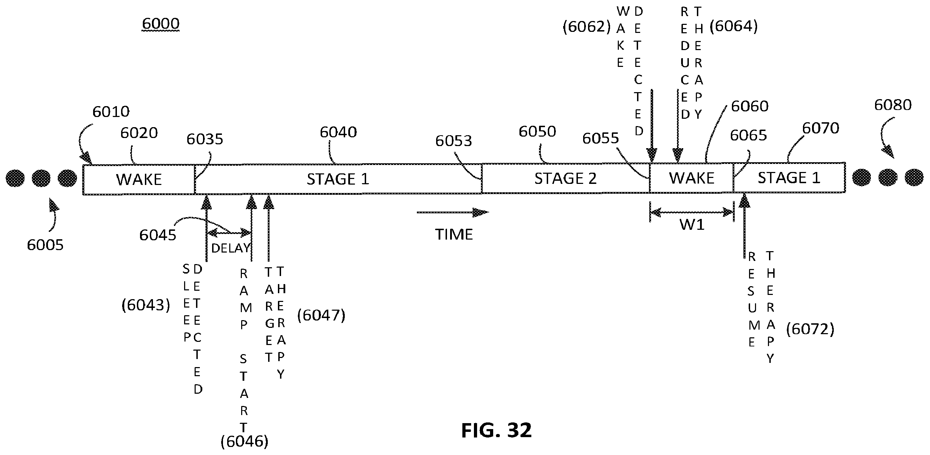

FIG. 32 is a diagram schematically representing an example timeline of sleep-wake-related events according to an example method of sleep-wake determination.

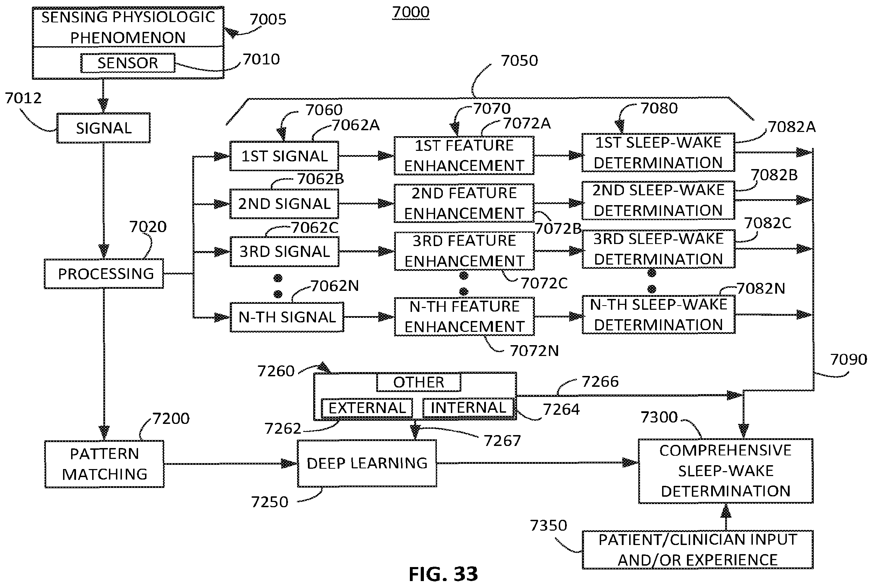

FIG. 33 is a diagram schematically representing an example method and/or example device for determining sleep-wake status.

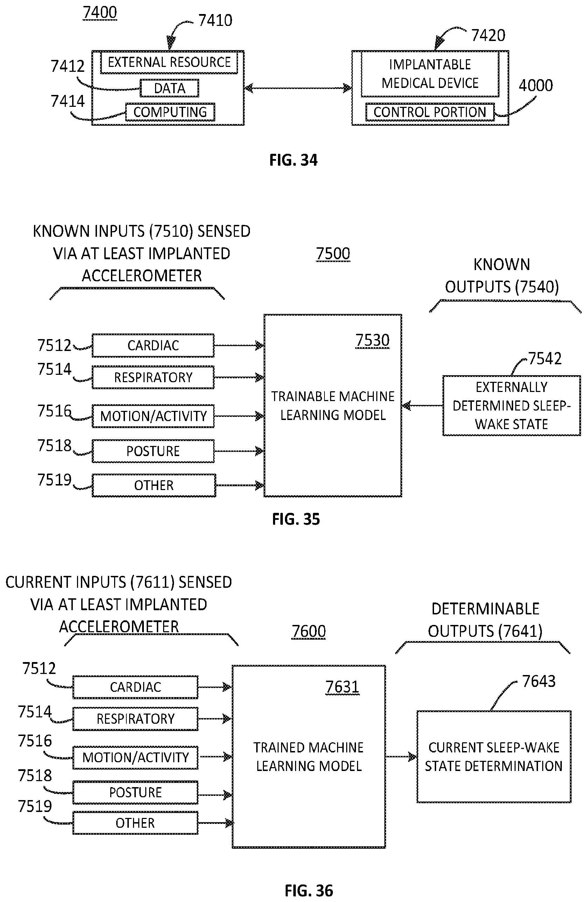

FIG. 34 is a diagram schematically representing an example method and/or example device including an implantable medical device in relation to an external resource for determining a sleep-wake status, including training a machine learning model, etc.
FIG. 35 is a diagram schematically representing an example method and/or example device for training a machine learning model regarding determining a sleep-wake status.
FIG. 36 is a diagram schematically representing an example method and/or example device for determining a sleep-wake status according to a trained machine learning model.

### Detailed Description

In the following detailed description, reference is made to the accompanying drawings which form a part hereof, and in which is shown by way of illustration specific examples in which the disclosure may be practiced. It is to be understood that other examples may be utilized and structural or logical changes may be made without departing from the scope of the present disclosure. The following detailed description, therefore, is not to be taken in a limiting sense. It is to be understood that features of the various examples described herein may be combined, in part or whole, with each other, unless specifically noted otherwise. The scope of the invention is defined by claim 1.

At least some examples of the present disclosure are directed to devices for diagnosis, therapy, and/or other care of medical conditions. At least some examples may comprise implantable devices and/or methods comprising use of implantable devices.

At least some of the example devices and/or example methods may relate to sleep disordered breathing (SBD) care, which may comprise monitoring, diagnosis, and/or stimulation therapy. In some such examples, SDB care may comprise automatically determining a sleep-wake status, which may in turn comprise detecting sleep and/or detecting wakefulness. In some examples, detecting sleep comprises detecting an onset of sleep and/or detecting on-going sleep after the onset of sleep. In some such examples, the sleep-wake determination may be used to initiate (and/or maintain) a treatment period in which neurostimulation therapy is used to treat sleep disordered breathing. In some examples, such automatic detection of wakefulness (as part of an automatic sleep-wake determination) may be used to terminate a treatment period. In this way, a patient may be able to forego and/or significantly reduce use a patient remote control on a daily basis such that the patient remote control may be used rarely to initiate and/or terminate treatment periods during which stimulation may be applied. In one aspect, such automatic detection of sleep onset, which may be used to initiate stimulation therapy within a treatment period, is distinct from mere sleep staging for diagnostic purposes.

In some such examples, such automatic sleep detection may be used instead of (or in conjunction with) other methods of identifying a beginning and/or end of a treatment period, such as selectable predetermined time of day (e.g. 10 p.m. to 6 p.m.) or a patient turning a device on (at sleep initiation) and the device off (at sleep termination). Various example methods, elements, and/or devices which may be used with, or instead of, automatic sleep detection in order to identify a beginning or end of a treatment period will be described later.

With at least some of the previously described examples in mind, in some examples a method of determining sleep-wake status comprises detecting sleep upon: (1) a time of day; and (2) detection of a lack of bodily motion indicative of sleep over a selectable, predetermined period of time. The time-of-day may be selectable and/or based on patient data. In at least some examples, the time-of-day corresponds to a time period when the patient is likely sleeping or attempting to sleep. Once at least these two criteria are met, then the method comprises initiating stimulation therapy at a low intensity and gradually increasing the intensity of the therapy to a target intensity level. As long as sensed physiologic information indicates that sleep is continuing, then then stimulation at the target intensity level continues. However, upon the detection of body motion by the patient (which is indicative of wakefulness) or upon detection of the patient mechanically indicating wakefulness, then the method terminates any stimulation therapy and remains in a no-stimulation mode (or lower stimulation mode in some examples) for a selectable predetermined of time (e.g. 15 minutes). In other words, after the interruption, the method delays resumption of stimulation therapy (or a change from low stimulation to target stimulation) for set period of time (e.g. 15 minutes). The length of the delay period is programmable. In some examples, the method may be performed using additional parameters, such as sensing additional physiologic phenomenon (e.g. respiration, cardiac, posture, etc.), implementing additional conditions, and/or enhancing sensitivity or specificity to the physiologic phenomenon being sensed. For instance, in one non-limiting example, the method may comprise detecting posture and comprising detection of sleep for certain postures (but not others) and/or for certain changes in posture. In some examples, the certain postures and/or changes in posture may be selectable by a patient and/or clinician. For example, in the method the specified posture for which sleep is detectable may comprise a lying down posture (e.g. supine, left side, right side) while a specified posture for which is sleep is not automatically detectable may comprise the patient being in a sitting-up posture.

In one aspect, to the extent that some sleep periods are unintended or occur due to an irregular schedule, at least some example methods of automatic sleep detection (and/or wakefulness detection) may enhance SDB care. In contrast, initiating SDB care solely according to preset times and/or according to manual control may miss opportunities to provide SDB care, whereas automatic sleep detection may increase the number and/or type of situations in which SDB care may be implemented. For instance, in situations in which the patient may desire sleep when sitting up (where lying down is the only permitted posture for which automatic sleep-detection is authorized), per the method the patient may activate a treatment period (e.g. via remote control or tapping of chest near IPG) or may modify the sleep detection feature to implement auto-detection of sleep for a sitting up posture.

In some examples, determination of the sleep-wake status also may comprise determining sleep stages of the patient, which may in turn enable modifying stimulation (e.g. increasing, reducing, etc.) within a treatment period.

In some examples, the automatic determination of a sleep-wake status may be customized automatically according to a particular patient's breathing patterns, activity patterns (e.g. ways and times in which they are active), sleeping patterns (e.g. time of day, days of week, etc.), and the like.

In some examples, determining a sleep-wake status may be implemented without posture or body position information. Accordingly, in some examples, sleep in a particular posture or particular position is not used to determine sleep-wake status. For instance, even though a patient may be in positions other than a supine position or side-laying position, example methods and/or devices of the present disclosure may still determine a sleep-wake status. Accordingly, example methods and/or devices may provide more robust and more accurate determination of sleep-wake status, and therefore provide more useful automatic initiation and/or termination of treatment periods regardless of sleep posture. In some examples, the automatic initiation, automatic pause features, etc. (e.g. arising from automatically detecting a sleep-wake status) may be selectively activated or deactivated by a clinician or patient, such as via a clinician programmer or a patient remote control.

At least some examples of determining a sleep-wake status also may relate to cardiac care, drug delivery, and/or other forms of care, whether standing alone or in association with sleep disordered breathing (SDB) care.

As part of determining sleep-wake status, in some examples, sleep time is collected and provided to a clinician and the patient for diagnostic, adherence monitoring, and patient engagement purposes. In some examples, the sleep time may comprise duration of sleep for each day, week, etc., as well as a time-of-day that sleep commences and/or that sleep terminates for each weekday, weekend day, average, etc. The collected sleep time also may comprise additional information regarding pauses in sleep, such as their frequency, duration, etc., as well as other sleep time information.

In some examples, when determining a sleep-wake status, the detection of sleep may differ from detecting wakefulness at least because at least some of the particular sensing modalities for best detecting each (sleep vs. wakefulness) may be different and/or the particular value of sensed parameters may be different in sleep than in wakefulness. For instance, during sleep the example methods and/or devices of the present disclosure can successfully distinguish REM sleep from wakefulness.

In some examples, at least some of the substantially the same above-described features and attributes used to determine a sleep-wake state may be used to detect a non-sleep state and/or a non-wake state. In some such examples, the term "non-sleep" may correspond to a probability of sleep remaining below a sleep detection threshold, while in some such examples, the term "non-wake" may correspond to a probability of wakefulness remaining below a wake detection threshold." In some examples, detection of a non-sleep state and/or a non-wake state may enhance comprehensive tracking, monitoring, etc. of the patient's sleep-wake state, which may in turn enhance care for sleep disordered breathing.

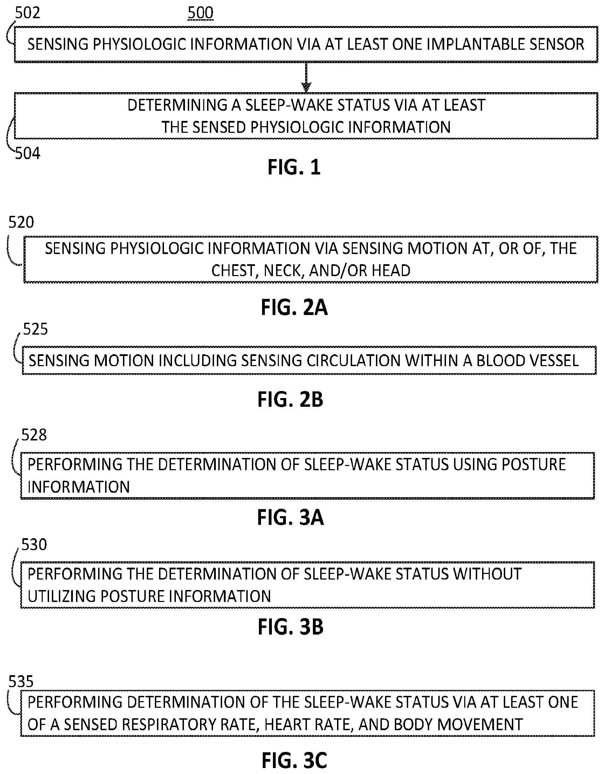

These examples, and additional examples, are further described in association with at least FIGS. 1-36.

As schematically represented at 500 in FIG. 1, in some examples a method comprises sensing physiologic information via at least one implantable sensor (502), and determining a sleep-wake status via the sensed physiologic information (504). As noted above, determining the sleep-wake status comprises sleep detection by which a treatment period for SDB care (e.g. neurostimulation therapy) may be initiated automatically. Conversely, determining the sleep-wake status may comprise wakefulness detection by which the treatment period for SDB care may be terminated automatically. In some examples in which a brief awakening is detected (versus prolonged wakefulness) and after which sleep is expected to be resumed, the treatment period is not terminated. Rather, the brief awakening may be deemed as a pause in the treatment period. Some example methods may comprise a time-based threshold (which may be just one factor of multiple factors) to determine whether the duration of wakefulness comprises a brief awakening or prolonged wakefulness.

In some examples, detecting wakefulness may comprise detecting a trend from NREM sleep toward wakefulness. In some such examples, upon detecting such a trend some example methods may comprise decreasing stimulation intensity gradually, such as via a ramp, which may increase patient comfort upon termination of the treatment period and/or during brief awakenings. In addition, upon resuming stimulation after a brief awakening, the stimulation intensity may be increased gradually (e.g. via a ramp). As noted elsewhere, a change in stimulation intensity may comprise a change in an amplitude, frequency, pulse width, etc. of electrical stimulation signal.

More specific example methods, devices, and/or arrangements of determining a sleep-wake status are described and illustrated in association with least FIGS. 2A-36.

As schematically represented at 520 in FIG. 2A, in some examples sensing the physiologic information may comprise sensing motion at, or of, the chest, neck, and/or head, which in turn may be used to determine the sleep-wake status. At least some aspects of such determination are further described in association with FIGS. 24-27B. For instance, sensing portion 2000 in FIG. 24 and/or sensing portion 2510 (of care engine 2500) in FIG. 27A comprises multiple sensor types, modalities, etc., at least some of which may be used to sense motion at , or of, the chest, neck, and/or head, and to utilize such sensed motion to determine a sleep-wake status (e.g. detecting sleep). One such example modality may comprise employing an accelerometer to sense motion at the chest, neck, and/or head, as further described later. In some examples, the accelerometer may be implanted at the chest, neck, and/or head, while in some examples, the accelerometer may be secured externally on the patient's body at such locations.

The sensed motion at the chest, neck, and/or head may comprise motion of the chest, neck, and/or head or may comprise motion phenomenon at those respective locations without necessarily involving gross motion of the chest, neck, and/or head, as further described later in association with at least FIGS. 25A-25F. In one non-limiting example, sensing the motion phenomenon at the neck or other location may comprise sensing circulation of blood within a blood vessel/vasculature (e.g. arterial motion within a vessel), as shown at 525 in FIG. 2B. In some such examples, the sensing element (e.g. accelerometer, impedance, other) may be at least partially incorporated in a microstimulator (or other implantable pulse generator) sized and shaped to be implantable within a blood vessel. The example method may comprise sensing ballistic motion of the blood vessel caused by the heartbeat of the patient. In some examples, the blood vessel may comprise an external jugular vein and hence the sensing of motion may occur at the neck in some examples without necessarily being motion of the neck (e.g. bending, titling, twisting, etc.).

As schematically represented at 528 in FIG. 3A, in some examples, a method of determining a sleep-wake status may be performed using sensed posture information and/or body position information. The sensed posture information may comprise a static posture or may comprise a change in posture, which may be considered a form of gross body motion mentioned above. As noted elsewhere, the sensed posture may be used to help confirm whether the patient is likely sleeping (e.g. lying down) or awake (e.g. sitting up) which may be in combination with other sensed information (e.g. heart rate, respiratory rate, etc.).

As schematically represented at 530 in FIG. 3B, in some examples, a method of determining a sleep-wake status may be performed without utilizing posture information and/or body position information.

For instance, a patient may sometimes intentionally (or unintentionally) sleep when sitting in a chair or an airline seat, and would benefit from SDB care (e.g. neurostimulation therapy). In such instances, determining sleep-wake status without using posture information may enhance quicker or more accurate detection of sleep for the patient sleeping in a sitting position because the example method may avoid a false negative indication (by a posture-based determination) that the patient is awake.

Conversely, a patient may sometimes intentionally be awake when lying horizontally, and accordingly, does not wish to receive SDB care. In such instances, determining sleep-wake status without using posture information may enhance quicker or more accurate detection of sleep for the patient who is awake in a lying-down position because the example method avoids a false positive indication (by a posture-based determination) that the patient is asleep because they are laying in the horizontal position typically associated with sleep.

As schematically represented at 535 in FIG. 3C, in some examples, a method of determining a sleep-wake status comprises sensing at least one of a respiratory rate, a heart rate, and body movement, and performing determination of the sleep-wake status at least via at least one of the respective sensed respiratory rate, heart rate, and body movement. In some examples, the sensed body movement may correspond to the sensed motion in FIGS. 2A and/or 2B. Various aspects of determination the sleep-wake status based on such sensed physiologic information is further described in association with at least FIGS. 24-27B and elsewhere throughout the various examples of the present disclosure.

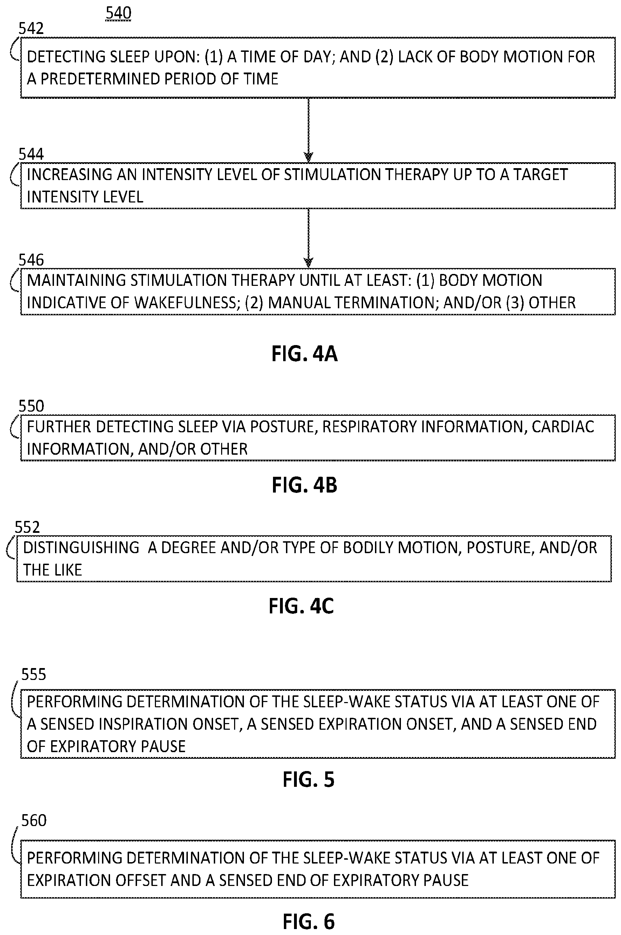

With these examples of FIGS. 1-3C in mind, in some examples detecting sleep (and/or wakefulness) in association with delivering a stimulation therapy may comprise the method shown at 540 in FIG. 4A. As shown at 542 in FIG. 4A, the method 540 may comprise detecting sleep upon: (1) a time of day; and (2) detection of a lack of bodily motion indicative of sleep over a selectable, predetermined period of time. The time-of-day may be selectable and/or based on patient data. Once at least these two criteria are met, then as shown at 544 in FIG. 4A, the method comprises increasing the intensity of the stimulation therapy from a lower initial intensity level to a target intensity level, such as in a ramped manner. As long as sensed physiologic information indicates that sleep is continuing, then stimulation at the target intensity level continues. However, upon the detection of body motion by the patient (which is indicative of wakefulness) or upon detection of the patient mechanically indicating wakefulness (e.g. physically tapping on chest near IPG), then the method may terminate any stimulation therapy and may remain in a no-stimulation mode for a selectable predetermined of time (e.g. 15 minutes). In other words, after the interruption, the method may delay therapy onset for set period of time (e.g. 15 minutes). The length of the delay period is programmable.

As shown 550 in FIG. 4B, in some examples the method 540 may further comprise sensing onset of sleep via additional parameters, such as sensing posture, respiratory information (e.g. stability regarding period, depth, etc.), cardiac information (e.g. stability per R-R interval, HR, etc.), and/or other information. For instance, in one non-limiting example, portion 542 of method 540 may comprise detecting posture (550) and comprise detection of sleep for some particular postures (but not others) and/or for some particular changes in posture (but not others). In some examples, the particular postures and/or particular changes in posture may be selectable by a patient and/or clinician. For example, the specified posture for which sleep is detectable may comprise a lying down posture (e.g. supine, left side, right side) but the method not permitting auto-detection of sleep when the a patient is sitting up.

In some instances, the example methods may detect (e.g. recognize) REM sleep and thereby avoid a false positive detection of wakefulness. In particular, while respiration during REM sleep does not exhibit the same stability as in non-REM sleep, such sensed less-stable respiration may be confirmed as occurring during REM sleep (and not wakefulness) based upon the patient having been asleep for some extended period of time (e.g. passage through multiple sleep stages, S1-S4) and upon the patient exhibiting a lack of bodily motion (e.g. of the type of bodily motion one would observe in wakefulness).

Implementation of method 540 also may comprise enhancing sensitivity to and/or specificity regarding the physiologic phenomenon being sensed.

In some examples, the detection of sleep (e.g. at 542) in method 540 in FIG. 4A also may comprise distinguishing a degree and/or type of bodily motion, posture, and the like as shown at 552 in FIG. 4C. This distinguishing may be performed in association with ramping up stimulation (e.g. at 544), ramping down stimulation, terminating stimulation (e.g. 546), etc. For instance, via aspect 552 of method 540, the method may distinguish voluntary bodily motion as opposed to the jostling of the patient caused by vehicle motion (e.g. airplane, car, etc.) or by a bed partner. In some such examples, upon detecting such jostling, the method 540 may comprise temporarily decreasing stimulation therapy or pausing therapy, and then resuming the method at 544 to cause a quick return to target (e.g. therapeutic) intensity stimulation levels. In contrast, via aspect 552, the method 540 may identify physical tapping of the chest (near the IPG) as a voluntary bodily motion/cause or may identify a significant change to posture (e.g. change from lying down to sitting up) as being voluntary (e.g. not inadvertent) and then terminating therapy (or causing a longer pause) as at 546 in FIG. 4A because such detected behavior is indicative of wakefulness, whether temporary or longer term.

At least FIGS. 5-8 provide at least some example methods by which the determination of sleep-wake status may be made according to respiratory morphologic features. Moreover, at least some aspects of such sensing and related determination (of the sleep-wake status) relating to respiratory morphology features are further described in association with at least FIGS. 24 and 27A-27C.

In one aspect, the various features of respiration morphologies addressed below in FIGS. 5-8 (e.g. inspiration onset, inspiration offset, magnitude, etc.) may enhance determining the sleep-wake status (e.g. at least sleep detection). In one aspect, these features of the respiratory morphology are readily identifiable and therefore beneficial to use in tracking a respiratory rate, which may be indicative of sleep (vs. wakefulness) according to the value of the respiratory rate, trend, and/or variability of the respiratory rate. In some examples, at least some of these features of respiration morphology may exhibit stability, which may be characteristic of sleep (vs. wakefulness). Some examples of such stability, which may be used to detect sleep/wake transitions, may include a stable respiratory rate, stability in an amplitude of the respiratory signal, stability of the percentage of the respiratory period corresponding to inspiration, and/or stability of the percentage of the respiratory period corresponding to expiration.

As schematically represented at 555 in FIG. 5, in some example methods, determining a sleep-wake status, such as via tracking at least some of the above-identified respiratory rate information, may comprise sensing at least one of an inspiration onset(s), an expiration onset(s), and end of expiratory pause, and performing determination of the sleep-wake status at least via at least one of the sensed inspiration onset(s), sensed expiration onset(s), and sensed end of expiratory pause.

As schematically represented at 560 in FIG. 6, in some example methods, determining a sleep-wake status, such as via tracking at least some of the above-identified respiratory rate information, may comprise sensing at least one of an expiration offset(s) and an end of expiratory pause(s) and performing determination of the sleep-wake status via at least one of the sensed expiration offset(s) and end of expiratory pause(s).

It will be understood that other combinations may be employed such as combining different combinations of fiducials (e.g. inspiration onset, end of expiratory pause, etc.) from FIGS. 5-8 or using just one of these fiducials from FIGS. 5-8 in determining a sleep wake status.

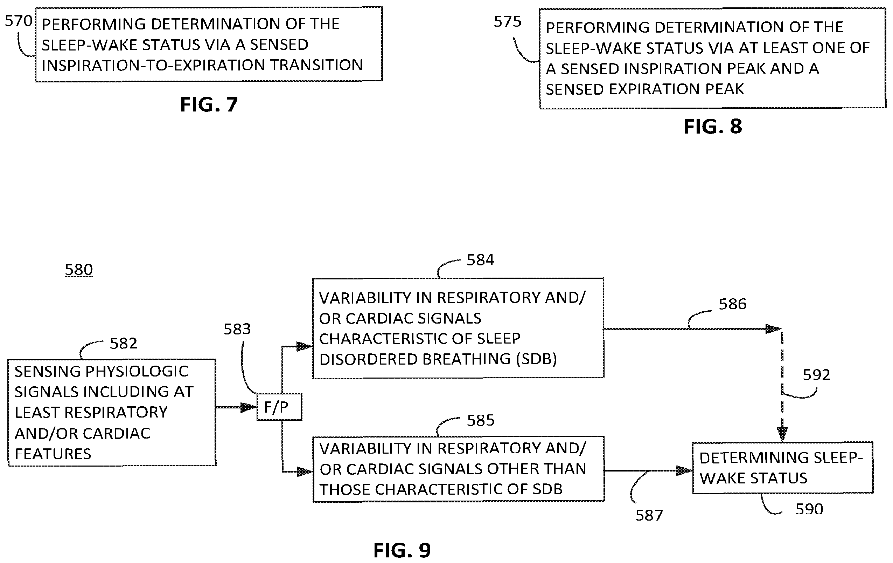

As schematically represented at 570 in FIG. 7, in some example methods, determining a sleep-wake status (such as via tracking at least some of the above-identified respiratory rate information) may comprise sensing an inspiration-to-expiration transition(s), and performing determination of the sleep-wake status at least via the sensed inspiration-to-expiration transition(s). Conversely, in some examples, sensing the physiologic information comprises sensing an expiration-to-inspiration transition(s), and determination of the sleep-wake status is performed via the sensed expiration-to-inspiration transition(s).

As schematically represented at 580 in FIG. 8, in some example methods, determining a sleep-wake status (such as via tracking at least some of the above-identified respiratory rate information) may comprise sensing at least one of an inspiration peak(s) and an expiration peak(s), and performing determination of the sleep-wake status via at least one of the sensed inspiration peak(s) and sensed expiration peak(s).

In some examples, at least some of the sensing of respiratory features, morphologies, etc. may be detected via sensing bioimpedance, as further described later in association with at least impedance parameter 2536 in FIG. 27A. Of course, as noted elsewhere, sensing of such respiratory features, etc. may be implemented via sensing modalities other than, or in addition to, sensing bioimpedance. For instance, in some examples, at least some of the sensing of respiratory features, morphologies, etc. may be detected via sensing an electrocardiographic (ECG) information, as further described later in association with at least ECG parameter 2520 in FIG. 27A and/or 2020 in FIG. 24.

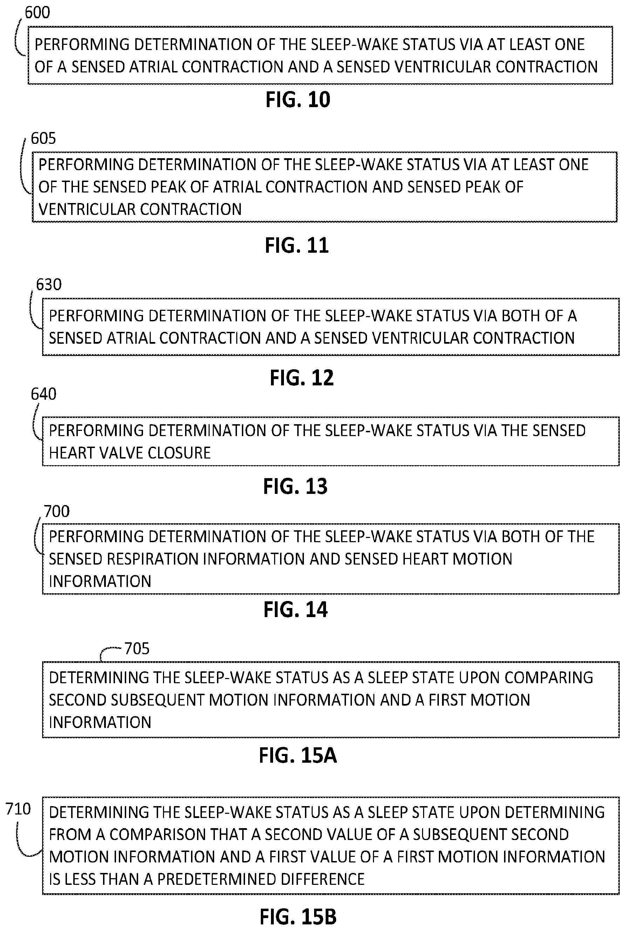

In some such examples associated with at least FIGS. 5-8 and/or at least FIGS. 10-13, a method and/or device for determination of sleep-wake status via sensing variability in respiratory behavior and/or cardiac behavior may comprise identifying some features of such variability which are indicative of sleep disordered breathing (SDB) and differentiating the identified SDB-indicative features from other features of respiratory behavior and/or cardiac behavior, such as those which are indicative of a sleep or wakefulness.

For instance, as schematically represented in the block diagram of FIG. 9, in some examples a method 580 (or device for) determining a sleep-wake status may comprise sensing physiologic signals including at least respiratory features and/or cardiac features as shown at 582. At 583, method 580 may comprise applying filtering and processing (F/P) of the sensed signals to produce: (1) filtered/processed signal information at 584 comprising variability in respiratory features and/or cardiac features which are characteristic of sleep disordered breathing (SDB); and (2) filtered/processed signal information at 585 comprising variability in respiratory features and/or cardiac features other than those characteristic of sleep disordered breathing (SDB). Via the signal information at 585, at 590 the method may comprise determining sleep-wake status. In some such examples, the sleep-wake status determination may comprise at least some of substantially the same features as described in association with at least FIGS. 5-8 and/or FIGS. 10-13, or other examples described throughout the present disclosure.

In some examples, the output 586 of the information 584 may be used in monitoring, diagnosing, treating, etc. sleep disordered breathing (SDB). However, in some examples, via path 592 this respiratory and/or cardiac information characteristic of sleep disordered breathing (SDB) may be used to confirm determination of a sleep-wake status, such as confirming that the patient is in a sleep state by confirming the occurrence of sleep disordered breathing. In making this confirmation, the method may identify characteristics of sleep disordered breathing including (but not limited to) at least some of the periodic nature of SDB, such as the reoccurring sequence of a flow limitation, an apnea (or hypopnea), and recovery. This identification also may comprise identifying similar periodic changes in heart rate occurring without detecting any gross changes in posture.

Alternatively, the method may comprise at least partially confirming that the patient is in a wake state (which is primarily determined by other information) via confirming the absence of sleep disordered breathing, such as due to the periodic nature of changes to respiratory patterns and heart rate without gross posture changes.

In some examples, determination of a sleep-wake status may be performed via sensed cardiac morphological features. Accordingly, at least FIGS. 10-13 provide at least some example methods by which the determination of a sleep-wake status may be made according to such example cardiac morphologic features. Moreover, at least some aspects of such sensing and related determination (of the sleep-wake status) relating to cardiac morphology features are further described in association with at least FIGS. 24 and 27A-27B.

In one aspect, the various features of cardiac morphologies addressed below in at least FIGS. 10-13 (e.g. atrial contraction, ventricular contraction, etc.) may enhance determining the sleep-wake status (e.g. at least sleep detection) at least because these features of the cardiac morphology are readily identifiable and therefore beneficial to use in tracking a heart rate, which may be indicative of sleep (vs. wakefulness) according to value of, trend of, and/or the variability of the heart rate (HRV). In some examples, at least some of these features of cardiac morphology may exhibit increasing stability, which may be characteristic of sleep (vs. wakefulness). In some examples, at least some sleep stages may exhibit more or less variability in heart rate variability (HRV) and/or more or less variability in respiratory features, as noted above. For instance, more variability in cardiac features (e.g. heart rate, etc.) and respiratory features (e.g. respiratory rate, etc.) can be expected in REM sleep. At least some examples of determining a sleep-wake status may identify such variability in cardiac and respiratory signals characteristic of a REM sleep stage in a manner which can be distinguished from variability (or lack thereof in some instances) of cardiac and respiratory signals characteristic of wakefulness. For instance, when the sensing of a moderate increase in variability of respiratory and/or cardiac features follows other sleep stages (e.g. S3, S4) coupled with sensing a lack of body motion, then the example methods may identify that the patient in in REM sleep.

In some examples, at least some of the sensing of cardiac features, morphologies, etc. may be detected via sensing bioimpedance, as further described later in association with at least impedance parameter 2036 in FIG. 24 and 2536 in FIG. 27A. In some examples, at least some of the sensing of cardiac features, morphologies, etc. may be detected via sensing an electrocardiograph (ECG) information, as further described later in association with at least ECG parameter 2020, 2520 in FIGS. 24 and 27A, respectively. The bioimpedance and/or ECG which is used to sense cardiac features, morphologies, etc., also may be used to sense respiratory features, morphologies, etc. (as previously noted), or may be used to sense both cardiac and respiratory features, morphologies, etc. It will be further understood that FIGS. 24, 27A provide additional example sensing types, modalities, etc. by which cardiac information (including but not limited to heart rate and/or heart rate variability) may be sensed, and which then may be used in determining sleep-wake status.

As schematically represented at 600 in FIG. 10, in some example methods, determining a sleep-wake status may comprise sensing at least one of an atrial contraction or a ventricular contraction, and performing determination of the sleep-wake status via at least one of the sensed atrial contraction and sensed ventricular contraction.

As schematically represented at 605 in FIG. 11, in some examples, determining a sleep-wake status may comprise sensing at least one of a peak of the atrial contraction or a peak of the ventricular contraction, and performing determination of the sleep-wake status via at least one of the sensed peak of atrial contraction and sensed peak of ventricular contraction. In some example methods, determining a sleep-wake status may comprise sensing onset of at least one of atrial contraction and of ventricular contraction, and performing determination of the sleep-wake status via the sensed onset of at least one of atrial contraction and ventricular contraction. In some example methods, determining a sleep-wake status may comprise sensing onset of at least one of atrial relaxation and of ventricular relaxation, and performing determination of the sleep-wake status via sensed onset of at least one of atrial relaxation and ventricular relaxation.

As schematically represented at 630 in FIG. 12, in some example methods, determining a sleep-wake status may comprise sensing both atrial contraction and ventricular contraction, and performing determination of the sleep-wake status via both of the sensed atrial contraction and ventricular contraction. By sensing both of these features of cardiac morphology, a more robust tracking of heart rate (and heart rate variability) may be performed, which in turn may provide a more robust determination of sleep-wake status.

As schematically represented at 640 in FIG. 13, in some example methods, determining a sleep-wake status may comprise sensing a heart valve closure, and performing determination of the sleep-wake status via the sensed heart valve closure. It will be understood that in some examples, the sensed heart valve closure may relate to closure of the semilunar valves and/or the atrioventricular (AV) valves. Moreover, in some such examples, via use of timing information, the sensed heart valve closure(s) may act as surrogates for other cardiac cycle morphologies, such as ventricular contractions, etc. For instance, because ventricular contractions begins just prior to closure of the AV valves and before the semilunar valves opening, heart sounds (e.g. S1, S2) may be used to track ventricular contractions and/or other cardiac morphologic features.

At least some of these relationships in cardiac morphology are further described in association with FIG. 27B and cardiac portion 2600 of care engine 2500 in FIG. 27A.

As schematically represented at 700 in FIG. 14, in some example methods, determining a sleep-wake status may comprise sensing respiration information and sensing heart motion information, and performing determination of the sleep-wake status via both of the sensed respiration information and sensed heart motion information.

As schematically represented at 705 in FIG. 15A, in some example methods, determining a sleep-wake status (e.g. onset of sleep, etc.) may comprise comparing subsequent second motion information to first motion information. As further shown at 710 in FIG. 15B, in some examples, method 705 may comprise determining the sleep-wake status (e.g. onset of sleep, etc.) upon determining from the comparison that a second value of the subsequent second motion information and a first value of the first motion information is less than a predetermined difference. The value of the predetermined difference may be selectable.

In some examples of methods 705, 710, each of the respective first and second motion information comprises at least one of sensed respiratory information, sensed cardiac information, and sensed body motion.

In some examples of methods 705, 710, the subsequent second information comprises information obtained in the most recent sensed respiratory cycle and the first information comprises information obtained in a prior respiratory cycle. In some examples, the subsequent second information comprises information for respiratory activity in at least the last 30 seconds. In some examples, this information may relate to respiratory activity in at least the last 60 seconds. In some examples, this information may relate to respiratory activity in at least the last 7 breaths.

In some examples, the prior respiratory cycle comprises a respiratory cycle immediately preceding the most recent sensed respiratory cycle. In some examples, the prior respiratory cycle(s) comprise respiratory activity in the 30 seconds (or 60 seconds, or 7 breaths) preceding the most recent sensed respiratory cycle. In some examples, the first information comprises respiratory information over at least one respiratory cycle or at least 30 seconds or at least 60 seconds.

In some examples of methods 705, 710, recent motion information is compared to objective values indicative of sleep. In some examples, lower and/or more stable respiration rates and heart rates are more likely to be associated with sleep. As previously noted in association with at least FIG. 9, determining a sleep-wake status may comprise separating out (e.g. filtering, rejection) of respiratory features characteristic of sleep disordered breathing (SDB) and/or of respiratory features characteristic of particular sleep stages which do not necessarily contribute to general sleep detection (e.g. detecting onset of sleep).

However, as previously noted with respect to at least aspects 584, 592 in the method of FIG. 9, the detection of sleep disordered breathing (SDB) also may be used to sense or confirm the presence of sleep, or may be used to sense or confirm the onset of sleep in some instances.

In some examples, the method (at 705, 710 in FIGS. 15A-15B) may comprise determining the subsequent second motion information from a second average value of motion information in the respiratory cycles of the sensed second respiratory period and determining the first motion information from a first average value of motion information in the respiratory cycles of the first respiratory period. In some such examples, the second average value of motion information corresponds to an average of a parameter, such as but not limited to: an average amplitude of the sensed second respiratory period; an average respiratory rate of the sensed second respiratory period; and/or an average ratio of an inspiratory period relative to an expiratory period for the sensed second respiratory period.

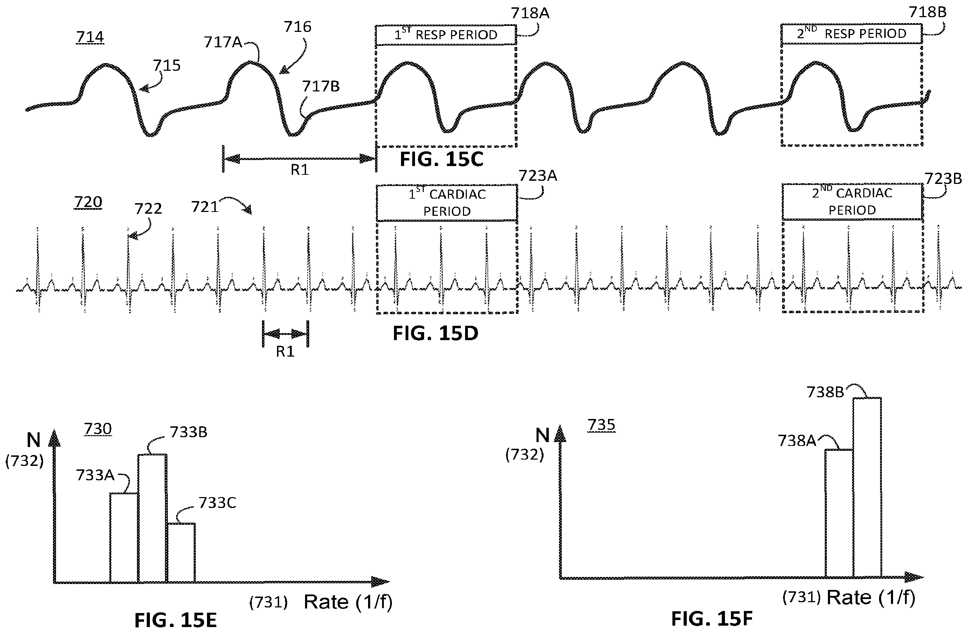

In some examples, at least some of the example methods previously described in association with FIGS. 15A-15B may be implemented via, or in association with, the examples shown and described in association with FIGS. 15C-15F.

In one example, as shown in FIG. 15C, a diagram 714 includes a respiratory signal 715 with a series of respiratory cycles 716 with an inspiratory phase 717A, active expiratory phase 717B, and expiratory pause 717C. Each respiratory cycle 716 may define a period R1, such as a duration of respiratory cycle 716. However, period R1 may be defined by other aspects of a cardiac cycle or cycles. As further shown in diagram 714, in some example implementations of the example methods described above in association with FIGS. 15A-15B, a second capture window 718B (e.g. 2^{nd} respiratory period) may be used to obtain the sensed subsequent motion information and a first capture window 718A may be used to obtain the sensed first motion information. The capture windows 718A, 718B may correspond to a sample of a single biologic period (e.g. respiratory cycle) or may correspond to a sample of multiple biologic periods (e.g. a series of several respiratory cycles). In some examples, the first and second capture windows may have the same size, i.e. duration. In some examples, the capture window may sample a biologic period other than a full respiratory cycle, such as sampling an inspiratory peak to inspiratory peak, which may be expressed as 30 second epochs, 3 breath sets, 10 heartbeat sets, start of expiration to start of expiration, and the like.

By comparing the respiratory motion information in the second capture window 718B to the respiratory motion information in the first capture window 718A, one may implement the example methods described above (in association with FIGS. 15A-15B) in comparing a second (i.e. subsequent) motion information with a first (i.e. earlier) motion information to at least partially determine sleep-wake status.

Similarly, as shown in the diagram 720 in FIG. 15D, in some examples a cardiac waveform signal 721 may be the signal of interest, which includes a series of cardiac cycles 722. Each cardiac cycle 722 may define a period R2, such as a duration of cardiac cycle 722. However, period R2 may be defined by other aspects of a cardiac cycle or cycles. In a manner similar to FIG. 15C, a first capture window 723A may be used to obtain first (i.e. earlier) cardiac-related motion information and a second capture window 723B may be used to obtain second (i.e. subsequent) cardiac-related motion information which may be compared to the first motion information (as described above) to at least partially determine sleep-wake status (e.g. onset of sleep, etc.). The capture windows 723A, 723B may correspond to a sample of a single biologic period (e.g. cardiac cycle) or may correspond to a sample of multiple biologic periods (e.g. a series of several cardiac cycles). In some examples, the first and second capture windows have the same size, i.e. duration.

With regard to FIGS. 15C and/or 15D, it will be further understood that more than two capture windows may be applied as part of obtaining and comparing motion information from a sensed signal of interest as part of determining sleep-wake status.

In some examples, the information obtained via use of the respective first and second capture windows (e.g. in FIGS. 15C, 15D) may be analyzed or plotted in various forms to graphically highlight the comparison. For instance, in just one example and as shown in diagram 730 in FIG. 15E, the cardiac motion information capture windows 723A, 723B (FIG. 15D) may be analyzed in histogram form by placing values of a particular parameter (e.g. heart rate) in groups 733A, 733B, 733C to assess values of the cardiac parameter (e.g. heart rate) in a first period of time (e.g. first capture window) and optionally with values of the same cardiac parameter (e.g. heart rate) in a second period of time (e.g. second capture window). In this way, it can be determined whether a change in the biological values of the parameter may be at least partially indicative to a change in sleep-wake status (e.g. onset of sleep) because shifts in the rates of respiration and cardiac rhythm are associated with the transition between sleep and wakefulness. This indication may be supplemented by other indicators of sleep/wake status such a body motion, position, time of day, time since potential onset of sleep, and other parameters than affect the confidence in the determination of sleep/wake status. It will be understood that the formation and/or use of the results of such example histograms may be implemented via control portion 4000 (FIG. 28A).

Similarly, in another example shown in diagram 735 in FIG. 15F, the respiratory motion information from capture windows 718A, 718B may be assessed in histogram form by which values of a particular parameter (e.g. respiratory rate) may be placed in groups 738A, 738B to graphically illustrate the respiratory motion information (e.g. values of the particular parameter) in a first period of time (e.g. first capture window 718A) juxtaposed with respiration motion information in a second period of time (e.g. second capture window 718B).

Some example methods which at least partially determine a sleep-wake status via comparing a subsequent motion information with a first motion information may use solely the respiration motion information alone (FIGS. 15C and 15 E), may use solely the cardiac information alone (FIGS. 15D and 15F), or may use both the respiratory information and cardiac information as described throughout various examples of the present disclosure. In addition, the cardiac and/or respiratory motion may be augmented with other physiologic information.

It will be understood that for illustrative simplicity the waveforms in FIG. 15C and FIG. 15D do not show dramatically different respiratory rates and heart rates among the different capture windows, but it will be understood that one capture window (e.g. 718A) may correspond to a significantly different respiratory rate or other waveform features from the respiratory rate (or other waveform features) in the other capture window (e.g. 718B).

It will be further understood that in some examples, the example implementations associated with FIGS. 15A-15E may be used for any biologic signal of interest which may contribute to determining sleep-wake status throughout the various examples of the present disclosure.

In some examples, at least some of the aspects described above with respect to FIGS. 15A-15E may be implemented via a history parameter 2542 and/or comparison parameter in sensing portion 2510 of care engine 2500, as later described in association with at least FIG. 27A.

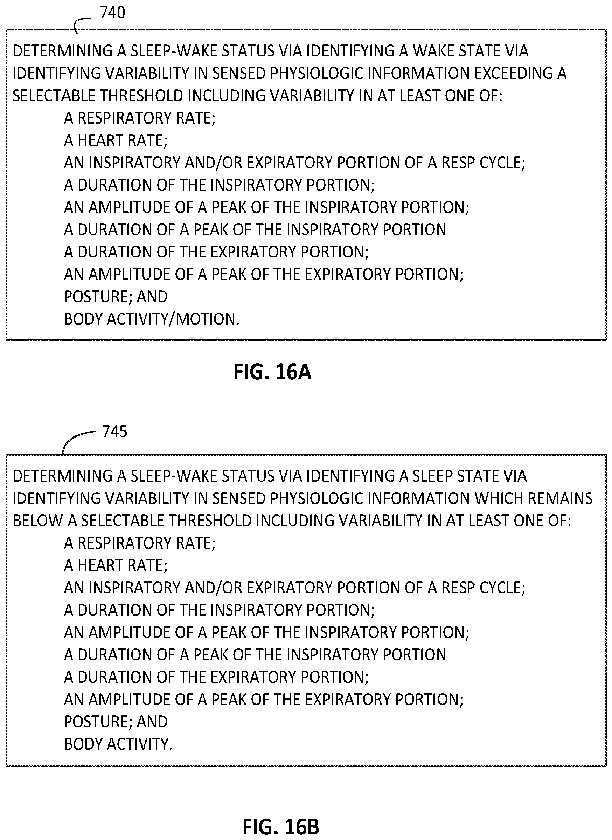

As schematically represented at 720 in FIG. 16A, in some example methods, determining a sleep-wake status may comprise identifying a wakefulness state (or lack thereof) via identifying variability in sensed physiologic information including variability in at least one of: a respiratory rate; a heart rate; an inspiratory and/or expiratory portion of a respiratory cycle; a duration of the inspiratory portion; an amplitude of a peak of the inspiratory portion; a duration of a peak of the inspiratory portion; a duration of the expiratory portion; body activity; and an amplitude of a peak of the expiratory portion. In some examples, for at least some parameters, the variability may be evaluated relative to a threshold, which may be fixed in some examples.

As schematically represented at 730 in FIG. 16B, in some example methods, determining a sleep-wake status may comprise identifying a sleep state (or lack thereof) via identifying variability in sensed physiologic information including variability in at least one of: a respiratory rate; a heart rate; an inspiratory and/or expiratory portion of a respiratory cycle; a duration of the inspiratory portion; an amplitude of a peak of the inspiratory portion; a duration of a peak of the inspiratory portion; a duration of the expiratory portion; body activity; and an amplitude of a peak of the expiratory portion. In some examples, for at least some parameters, the variability may be evaluated relative to a threshold, which may be fixed in some examples.

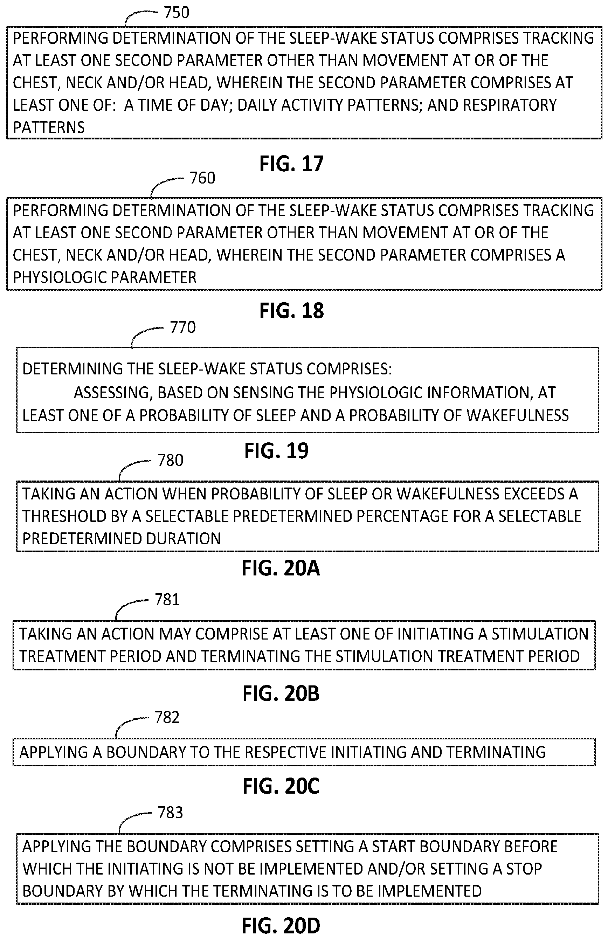

As schematically represented at 750 in FIG. 17, in some examples, performing determination of the sleep-wake status comprises tracking at least one second parameter other than movement at (or of) the chest, neck, and/or head, wherein the second parameter comprises at least one of: a time of day; daily activity patterns; and (typical) respiratory patterns.

As schematically represented at 760 in FIG. 18, in some examples, performing determination of the sleep-wake status comprises tracking at least one second parameter other than movement at (or of) the chest, neck, and/or head, wherein the second parameter comprises a physiologic parameter. For examples, one such physiological parameter may comprise temperature (e.g. 2038 in FIG. 24, 2538 in FIG. 27A).

As schematically represented at 770 in FIG. 19, in some examples, determining the sleep-wake status comprises assessing, based on sensing the physiologic information, at least one of a probability of sleep and a probability of wakefulness.

As schematically represented at 780 in FIG. 20A, some example methods (and/or devices) comprise taking an action when a probability of sleep or a probability of wakefulness exceeds a threshold. In some such examples, some example methods (and/or devices) comprise taking an action when a probability of sleep or a probability of wakefulness exceeds a threshold by a selectable predetermined percentage for a selectable predetermined duration.

In some examples of method 780 (FIG. 20A), taking an action may comprise at least one of initiating a stimulation treatment period and terminating the stimulation treatment period as shown at 781 in FIG. 20B. In some such examples, the taking an action (when a probability of sleep exceeds the threshold as in 780 in FIG. 20A) may comprise initiating a therapy treatment period (e.g. applying stimulation), resuming stimulation within a treatment period after a pause or suspension of stimulation, and/or other actions. In some examples, taking an action (when a probability of wakefulness exceeds the threshold) may comprise terminating a therapy treatment period, suspending stimulation within a treatment period, and/or other actions.

In some such examples, the initiating and/or resuming stimulation therapy may comprise employing a stimulation ramp in which an initial stimulation intensity is lower and then increased to a target intensity level. In some examples, terminating therapy may comprise employing a stimulation ramp in which a stimulation intensity is decreased gradually from a target therapy intensity level until stimulation is no longer applied (i.e. stimulation intensity equals zero).

With further reference to at least FIG. 20A, in some examples taking an action in method 780 may comprise use of an observer for an additional period of time to ensure the patient is asleep and/or using a start timer to initiate counting a selectable, predetermined period of time (e.g. delay) until stimulation is initiated as part of a treatment period.

In some such examples as method 780 (FIG. 20A), the method further comprises applying a boundary to the respective initiating and terminating as shown at 782 in FIG. 20C. At least some aspects of such a boundary are further described in association with boundary parameter 3016 of activation portion 3000 in FIG. 27A.

In some example methods associated with method 780, applying the boundary comprises setting a start boundary before which the initiating is not be implemented and/or setting a stop boundary by which the terminating is to be implemented, as shown at 783 in FIG. 20D.

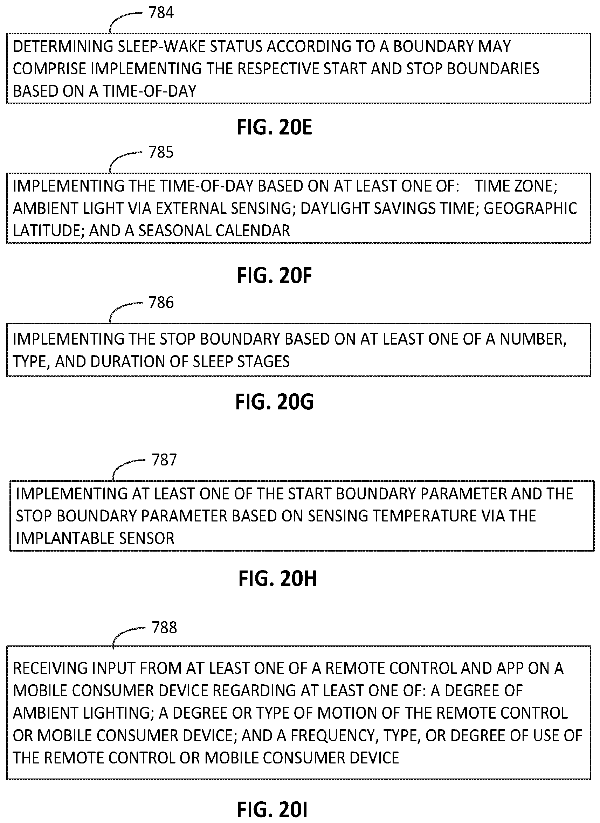

In some examples, the method (e.g. 782, 783) of determining sleep-wake status according to a boundary may comprise implementing the respective start and stop boundaries based on a time-of-day, as shown at 784 in FIG. 20E. In some examples, as shown at 785 in FIG. 20F, the method may comprise implementing the time-of-day based on at least one of: time zone; ambient light via external sensing; daylight savings time; geographic latitude; and a seasonal calendar.

In some examples, as shown at 786 in FIG. 20G, the method (e.g. 783) may comprise implementing the stop boundary based on at least one of a number, type, and duration of sleep stages.

In some examples, as shown at 787 in FIG. 20H, the method (e.g. 783) may comprise implementing at least one of the start boundary parameter and the stop boundary parameter based on sensing temperature via the implantable sensor. In some examples, the method may comprise implementing, at least one of the initiating of the stimulation treatment period and the terminating of the stimulation treatment period, based on sensing body temperature via the implantable sensor. In some examples, the method may comprise arranging the implantable sensor within an implantable pulse generator and the implantable sensor comprises a temperature sensor. In some such examples, method 787 may be implemented via, and/or is further described later in association with, at least temperature sensor 2038 in FIG. 24, temperature parameter 2538 in FIG. 27A, and/or at least boundary parameter 3016 in FIG. 27A.

In some examples, as shown at 788 in FIG. 20I, the method (including determining the sleep-wake status such as at 770 in FIG. 19 and/or in 780 at FIG. 20A) may comprise receiving input from at least one of a remote control and app on a mobile consumer device regarding at least one of: a degree of ambient lighting; a degree or type of motion of the remote control or mobile consumer device; and a frequency, type, or degree of use of the remote control or mobile consumer device.

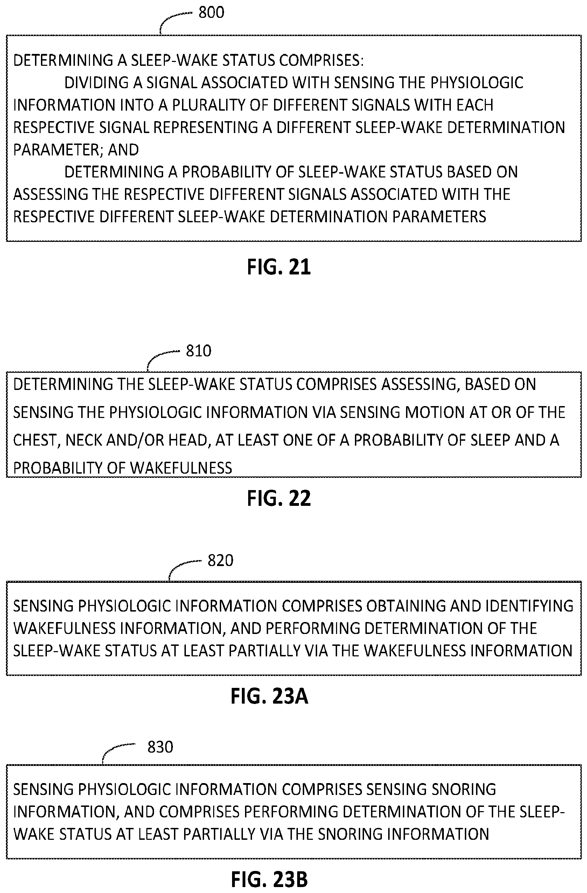

As schematically represented at 800 in FIG. 21, some examples of determining a sleep-wake status may comprise: dividing a signal associated with sensing the physiologic information into a plurality of different signals with each respective signal representing a different sleep-wake determination parameter; and determining a probability of sleep-wake status based on assessing the respective different signals associated with the respective different sleep-wake determination parameters. In some examples, this example method may comprise voting, by which each signal provides input to the overall probability of sleep. In some such examples, the various separate signals may be weighted differently so as to apply each respective sleep-wake determination parameter relatively more or relatively less in comparison to the other respective sleep-wake determination parameters.

In some examples, at least some aspects of method 800 (FIG. 21) may be implemented via at least some of the features and attributes of the arrangement described in association with at least FIGS. 33-36.

As schematically represented at 810 in FIG. 22, In some examples determining the sleep-wake status comprises at least one of: assessing, based on sensing the physiologic information via sensing motion at (or of) the chest, neck, and/or head, at least one of a probability of sleep and a probability of wakefulness.

As schematically represented at 820 in FIG. 23A, in some examples, sensing physiologic information comprises obtaining and identifying wakefulness information (e.g. during normal wake periods), and comprising performing determination of the sleep-wake status at least partially via the wakefulness information. In some such examples, the wakefulness information is used to better characterize sleep and therefore more readily determine a sleep-wake status (e.g. such as detecting sleep or lack thereof). However, in this context, the identified wakefulness information is not used to adjust therapy (e.g. stimulation parameters, etc.) and/or not used to characterize a respiratory disorder. In some examples, the identification of wakefulness may be performed via sensing at least one of gross body motion and movement. In some examples, sensing physiologic information comprises obtaining sleep information, and comprising performing determination of the sleep-wake status via the sleep information.

As schematically represented at 830 in FIG. 23B, in some examples, a method comprises sensing snoring and using the snoring information as part of determining sleep-wake status. In some such examples, the method(s) may comprise quantifying the sensed snoring, and reporting snoring information to at least one of a patient, physician, or caregiver. In some examples, the snoring may be differentiated from normal speech. In some examples, snoring may be sensed, tracked, etc. in association with acoustic sensor 2039 (FIG. 24) and/or acoustic parameter 2539 (FIG. 27A).

In some examples, various features and attributes of the example methods (and/or care devices) described in association with at least FIGS. 1-23B for determining sleep-wake status may be combined and implemented in a complementary or additive manner.

These, and additional features and attributes associated with FIGS. 1-23B will be further described in association with at least FIGS. 24-36. Moreover, at least some of the examples described in association with FIGS. 24-36 may comprise example implementations of the examples described in association with FIGS. 1-23B.

FIG. 24 is a block diagram schematically representing an example sensing portion. In some examples, an example method may employ and/or an example SDB care device may comprise the sensing portion 2000 to sense physiologic information and/or other information, with such sensed information relating to sleep-awake detection, among other uses. The sensed information may be used to implement at least some of the example methods and/or examples devices described in association with at least FIGS. 1-23 and/or FIGS. 25A-36.

It will be understood that the sensing portion 2000 may be implemented as single sensor or multiple sensors, and may comprise a single type of sensor or multiple types of sensing. In addition, it will be further understood that the various types of sensing schematically represented in FIG. 24 may correspond to a sensor and/or a sensing modality.

For instance, in one non-limiting example, an electrocardiogram (ECG) sensor 2020 in FIG. 24 may comprise a sensing element (e.g. electrode) or multiple sensing elements arranged relative to a patient's body (e.g. implanted in the transthoracic region) to obtain ECG information. In some examples, the ECG information may comprise one example implementation to obtain cardiac information, including but not limited to, heart rate and/or heart rate variability (HRV), which may be used (with or without other information) in determining sleep-wake status as described throughout the examples of the present disclosure.

However, in some instances, the ECG sensor 2020 may represent ECG sensing element(s) in general terms without regard to a particular manner in which sensing ECG information may be implemented.

In some examples in which multiple electrodes are employed to obtain an ECG signal, an ECG electrode may be mounted on or form at least part of a case (e.g. outer housing) of an implantable pulse generator (IPG), such as further described later in association with at least FIG. 25A. In such instances, other ECG electrodes are spaced apart from the ECG electrode associated with the IPG. In some examples, such as further described in association with FIG. 25A, at least some ECG sensing electrodes also may be employed to deliver stimulation to a nerve or muscle, such as but not limited to, an upper airway patency-related nerve (e.g. hypoglossal nerve) or other nerves or muscles.

In some examples, multiple ECG sensing electrodes may be mounted on or form different portions of a case of an IPG, such as later described in association with at least FIGS. 25B, 25C, 25D. In such examples, the respective ECG electrodes are arranged on the case of the IPG to be electrically independent of each other so that a suitable ECG signal may be obtained.

In some examples, an ECG sensing electrode may be used solely for sensing (e.g. single purpose) but is located along a lead body of a stimulation lead, as further described later in association with FIG. 25A. It will be understood that such dedicated ECG sensing electrode is positioned along the stimulation lead in a manner to avoid contact with a case of the IPG, particularly in examples in which an exposed electrically conductive portion of the case of the IPG may act as an electrode and by which a sensing vector may be obtained via a combination of the sensor electrode along the lead and the conductive portion of the IPG. Similarly, the same/similar electrode arrangement may be used to sense bioimpedance, as also described more fully later in association with FIGS. 25A-25F, 27A.

In some examples, other types of sensing may be employed to obtain cardiac information (including but not limited to heart rate and/or heart rate variability), such as via ballistocardiogram sensor(s) 2023A, seismocardiogram sensor(s) 2023B, and/or accelerocardiogram sensor(s) 2023C as shown in FIG. 24. In some examples, such sensing is based on and/or implemented via accelerometer-based sensing such as further described below in association with accelerometer 2026.

In one aspect, in some examples the ballistocardiogram sensor 2023A senses cardiac information caused by cardiac output, such as the forceful ejection of blood from the heart into the great arteries that occurs with each heartbeat. The sensed ballistocardiogram information may comprise heart rate (HR), heart rate variability (HRV), and/or additional cardiac morphology. As noted above in context with at least FIG. 4A, in some examples such ballistocardiogram-type information may be sensed from within a blood vessel in which the sensor (e.g. accelerometer) senses the movement of the vessel wall caused by pulsations of blood moving through the vessel with each heartbeat. This phenomenon may sometimes be referred to as arterial motion.

In one aspect, the seismocardiogram sensor 2023B may provide cardiac information which is similar to that described for ballistocardiogram sensor 2023A, except for being obtained via sensing vibrations, per an accelerometer (e.g. single or multi-axis), in or along the chest wall caused by cardiac output. In particular, the seismocardiogram measures the compression waves generated by the heart (e.g. per heart wall motion and/or blood flow) during its movement and transmitted to the chest wall. Accordingly, the sensor 2023B may be placed in the chest wall.

In some such examples of sensing per sensors 2023A, 2023B, such methods and/or devices also may comprise sensing a respiratory rate and/or other respiratory information.

As further shown in FIG. 24, in some examples the sensing portion 2000 may comprise an electroencephalography (EEG) sensor 2012 to obtain and track EEG information. In some examples, the EEG sensor 2012 may also sense and/or track central nervous system (CNS) information in addition to sensing EEG information. In some examples, the EEG sensor(s) 2012 may be implanted subdermally under the scalp or may be implanted in a head-neck region otherwise suitable to sense EEG information. Accordingly, the EEG sensor(s) 210 are located near the brain and may detect frequencies associated with electrical brain activity.

In some examples, a sensing element used to sense EEG information is chronically implantable, such as in a subdermal location (e.g. subcutaneous location external to the cranium skull), rather than an intracranial position (e.g. interior to the cranium skull). In some examples, the EEG sensing element is placed and/or designed to sense EEG information without stimulating a vagus nerve at least because stimulating the vagal nerve may exacerbate sleep apnea, particularly with regard to obstructive sleep apnea. Similarly, the EEG sensing element may be used in a device in which a stimulation element delivers stimulation to a hypoglossal nerve or other upper airway patency nerve without stimulating the vagus nerve in order to avoid exacerbating the obstructive sleep apnea.

In some examples the sensing portion 2000 may comprise an electromyogram (EMG) sensor 2022 to obtain and track EMG information. In some such examples, the EMG sensor may comprise an electrode positioned near the tongue to detect signals indicative of voluntary control of the tongue, which in turn may be indicative of wakefulness. In some examples, the sensed EMG signals may be used to identify sleep and/or obstructive events. At least some additional aspects regarding EMG sensing is described in association with at least FIG. 25A.

In some examples, as shown in FIG. 24, the sensing portion 2000 may comprise an EOG sensor 2024 to obtain and track EOG information, which may be used to a determine sleep-wake status and/or different sleep stages. In some instances, such sensed EOG information may be used to distinguish REM sleep from non-REM sleep or from wakefulness. In some examples, a sensing element for obtaining EOG information may be implanted in the head-and-neck portion, such as adjacent the eyes, eye muscles, and/or eye nerves, etc. In some examples, the sensing element may communicate the EOG information wirelessly, or via an implanted lead, to a control element (e.g. monitor, pulse generator, and the like) implanted within the head-and-neck region. In some such examples, the sensing element may comprise an electrode implanted near one or both eyes of the patient.

However, in some examples, the EOG information may be obtained via external sensing elements which are worn on the head or which may observe the eye movement, position, etc. such as via a mobile phone, monitoring station within proximity to the patient, and the like. Such externally-obtained EOG information may be communicated wirelessly to an implanted monitor, pulse generator and the like which controls sensing elements and/or stimulation elements implanted within the patient's body. Some aspects of sensing via EOG sensor are further described later in association with at least FIG. 27A.

In some examples, any one or a combination of the various sensing modalities (e.g. EEG, EMG, etc.) described in association with FIG. 24 may be implemented via a single sensing element 2014.

In some examples, the sensing portion 2000 may comprise an accelerometer 2026. In some examples, the accelerometer 2026 and associated sensing (e.g. motion at (or of) the chest, neck, and/or head, respiratory, cardiac, posture, etc.) may be implemented according to at least some of substantially the same features and attributes as described in Dieken et al., ACCELEROMETER-BASED SENSING FOR SLEEP DISORDERED BREATHING (SDB) CARE, published as U.S. 2019-0160282 on May 30, 2019, and which is incorporated by reference herein in its entirety. In some examples, the accelerometer may comprise a single axis accelerometer while in some examples, the accelerometer may comprise a multiple axis accelerometer.

Among other types and/or ways of sensing information, the accelerometer sensor(s) 2026 may be employed to sense or obtain a ballistocardiogram (2023A), a seismocardiogram (2023B), and/or an accelerocardiogram (2023C), which may be used to sense (at least) heart rate and/or heart rate variability (among other information such as respiratory rate in in some instances), which may in turn may be used as part of determining sleep-wake status as described throughout the examples of the present disclosure.

In some examples, the accelerometer 2026 may be used to sense activity, posture, and/or body position as part of determining a sleep-wake status, the sensed activity, posture, and/or body position may sometimes be at least partially indicative of a sleep-wake status.

In some examples, the sensing portion 2000 may comprise an impedance sensor 2036, which may sense transthoracic impedance or other bioimpedance of the patient. In some examples, the impedance sensor 2036 may comprise a plurality of sensing elements (e.g. electrodes) spaced apart from each other across a portion of the patient's body, such as electrodes 2120, 2135, 2130 in FIG. 25A, and/or example electrodes (e.g. 2310, 2402, 2404) in FIGS. 25B-25F. In some such examples, one of the sensing elements (e.g. electrode 2135 in FIG. 25A) may be mounted on or form part of an outer surface (e.g. case) of an implantable pulse generator (IPG) or other implantable sensing monitor, while other sensing elements (e.g. electrodes 2120, 2130 in FIG. 25A) may be located at a spaced distance from the sensing element of the IPG or sensing monitor. In at least some such examples, the impedance sensing arrangement integrates all the motion/change of the body (e.g. such as respiratory effort, cardiac motion, etc.) between the sense electrodes (including the case of the IPG when present). Some examples implementations of the impedance measurement circuit will include separate drive and measure electrodes to control for electrode to tissue access impedance at the driving nodes

In some examples, the sensing portion 2000 may comprise a pressure sensor 2037, which senses respiratory information, such as but not limited to respiratory cyclical information. In some such examples, the respiratory pressure sensor may comprise at least some of substantially the same features and attributes as described in Ni et al., US Patent Publication US2011/0152706, METHOD AND APPARATUS FOR SENSING RESPIRATORY PRESSURE IN AN IMPLANTABLE STIMULATION SYSTEM, published on June 23, 2011, and which is incorporated herein by reference in its entirety. In some examples, the pressure sensor 2037 may be located in direct or indirect continuity with respiratory organs or airway or tissues supporting the respiratory organs or airway in order to sense respiratory information.

In some examples, one sensing modality within sensing portion 2000 may be at least partially implemented via another sensing modality within sensing portion 2000.

In some examples, sensing portion 2000 may comprise an acoustic sensor 2039 to sense acoustic information, such as but not limited to cardiac information (including heart sounds), respiratory information, snoring, etc.

In some examples, sensing portion 2000 may comprise body motion parameter 2026 by which patient body motion may be detected, tracked, etc. The body motion may be detected, tracked, etc. via a single type of sensor or via multiple types of sensing. For instance, in some examples, body motion may be sensed via accelerometer 2026 and in some examples, body motion may be sensed via EMG 2022 and/or other sensing modalities, as described throughout various examples of the present disclosure.

In some examples, the sensing portion 2000 in FIG. 24 may comprise a posture parameter 2040 to sense and/or track sensed information regarding posture, which also may comprise sensing of body position, activity, etc. of the patient. This sensed information may be indicative of an awake or sleep state of the patient in some examples. In some such examples, such information may be sensed via accelerometer 2026 as mentioned above, and/or other sensing modalities. In some examples, such posture information (and/or body position, activity) may be used sometimes alone and/or in combination with other sensing information to determine sleep-wake status. As described elsewhere herein, in some examples posture may be considered as one of several parameters when determining a probability of sleep (or awake).

For instance, sensing an upright posture typically is associated with a wakeful state, such as standing or walking. However, as noted elsewhere, a person could be in an upright sitting position and still be in a sleep state (e.g. sleeping a chair). Accordingly, posture may be just one parameter used in determining a sleep-wake state, along with at least some other parameters described in association with sensing portion 2000 of FIG. 24 and/or care engine 2500 in FIG. 27A. Conversely, sensing a supine or lateral decubiitis (i.e. laying on a side) posture typically is associated with a sleep state. However, a patient might be in such a position without being asleep, such that other parameters (e.g. FIGS. 24, 27A) in addition to, or instead of, posture may significantly enhance determination of sleep-wake status.

Moreover, sensing posture may not be limited to sensing a static posture but extend to sensing simple changes in posture (or body position), which may be indicative of a sleep-wake state at least because certain changes in posture (e.g. from supine to upright) are mostly likely indicative of a wake state. Similarly, more complex or frequent changes in posture and/or body position may be further indicative of a wake state, whereas maintaining a single stable posture for an extended period time may be indicative of a sleep state.

In some examples, the sensing portion 2000 of care engine 2500 (FIG. 27A) comprises other parameter 2041 to direct sensing of, and/or receive, track, evaluate, etc. sensed information other than the previously described information sensed via the sensing portion 2510.

In some example methods and/or devices, via sensing portion 2000, a sleep-wake status may be determined without using posture information or body position information. In some such examples, a determination of a sleep-wake status without regard to posture information (or body position) may permit the device to provide efficacious sleep disordered breathing (SDB) care even when the patient may be sleeping in a vertical position, such as sitting in a chair, in a zero-gravity environment, etc. in contrast to a conventional assumption of sleep occurring in a horizontal body position. Such implementations may permit SDB care when a patient is sleeping during travel, such as sitting in an airplane seat, automobile seat, train seat, etc. In some such examples, a SDB care method and/or SDB care device may sometimes be referred to as being posture-insensitive.

As further shown in FIG. 24, in some examples the sensing portion 2000 may comprise a temperature sensor 2038. In some examples, such sensed temperature may be tracked, evaluated, etc. in association with temperature parameter 2538 in sensing portion 2510 of care engine 2500 in FIG. 27A.

In some examples, the sensed temperature may be used as one factor in making a sleep-wake status determination according to the examples of the present disclosure. In one aspect, the temperature sensor 2038 may sense and track a patient's normal fluctuation (e.g. temperature profile) in body temperature within a 24 hour daily period, which may exhibit on the order of a 2 degree F change. For most patients, their body temperature may reach and remain at the high end (e.g. 99.5 F) of its range during the middle of the day and evening (e.g. 7pm) before falling throughout late evening and overnight to the low end (e.g. 97.5 F) of its range by early morning (e.g. 5 or 6 am). In some examples, the temperature sensor may 2038 may sense a change in the sensed temperature which occurs within a selectable time window of a 24 hour daily period and which exceeds a selectable threshold. In some examples, the selectable time window may comprise one hour, two hours, or other time periods. In some such examples, one method comprises selecting that a change of a predetermined number of degrees within the selectable time window will correspond to either a wake-to-sleep state transition or a sleep-to-wake state transition.

In some example methods, sensing a change in temperature (such as via sensor 2038) during a treatment period may be used to identify sleep disordered breathing behavior. In some such examples, additional sensed information (as described in examples of the present disclosure) may be used in addition to sensed temperature to identify sleep disordered breathing (SDB) behavior.

In some examples, this temperature fluctuation information se sensed via temperature sensor 2038 may be used in association with boundary parameter 3016 to automatically implement a boundary or limit on the beginning and end of the treatment period, such that the lowest sensed body temperature may be used to at least partially mark a boundary of an end of a treatment period for a typical patient which sleeps at night. Similarly, the highest sensed body temperature (e.g. held for an extended period) may be used to at least partially implement a boundary at a beginning of a treatment period. In some such examples, these features may be used to implement method 787 in FIG. 20H.

In some examples, these same temperature-based boundaries may be used as one factor (among other factors) to determine a sleep-wake status. At least some other factors, which may be used with this sensed temperature fluctuation information to determine a sleep-wake status, may comprise a time of day parameter, accelerometer information, cardiac information, respiratory information, etc.

In some examples, smaller yet detectable temperature changes within a treatment period may be used to at least partially determine a sleep-wake status. For instance, a detectable temperature change may be sensed as a result of patient exertion to breathe in response to an apnea event, given the greater muscular effort in attempting to breathe.

Moreover, in some examples, such sensed temperature fluctuation information may provide a more distinctive or characteristic indication of a sleep or wake period when compared with heart rate or body position, which may exhibit more changes, some of which are not necessarily indicative of a sleep period or wake period, at least in some instances.

In some examples, at least some of the sensors and/or sensor modalities described in association with FIG. 24 (and/or FIG. 27A) may be incorporated within or on a pulse generator (IPG 2133 in FIG. 25A), or within or on a microstimulator (e.g. FIGS. 25A-25E).

FIG. 25A is a diagram schematically representing several example implementations of sensing elements and a neurostimulation device 2113 implanted with a patient. The sensing elements and/or neurostimulation device may be employed in at least some of the example methods and/or example devices described throughout the present disclosure. As shown in FIG. 25A, the neurostimulation device 2113 may comprise an implantable pulse generator (IPG) 2133 and stimulation lead 2117, which comprises a lead body 2118 and a stimulation electrode 2112. The stimulation electrode 2112 is subcutaneously implanted and engaged relative to an upper airway patency-related nerve 2105, such as the hypoglossal nerve. In some examples, the IPG 2133 is implanted in the pectoral region 2101 with stimulation lead 2117 extending upward into the head-and-neck region 2103. In some examples, the stimulation electrode 2112 is chronically implantable, and may comprise a cylindrical arrangement to be at least partially wrapped about a nerve, may comprise a paddle-style electrode, may comprise a non-cuff configuration, or other configuration by which electrode may be chronically implanted in nerve-stimulating relation to the nerve.

In some examples, the stimulation electrode 2112 may comprise at least some of substantially the same features and attributes as described in Bonde et al. U.S. 8,340,785, SELF EXPANDING ELECTRODE CUFF, issued on December 25, 2102 and Bonde et al. U.S. 9,227,053, SELF EXPANDING ELECTRODE CUFF, issued on January 5, 2016, both which are hereby incorporated by reference in their entirety. In some examples, the stimulation electrode 2112 may comprise at least some of substantially the same features and attributes as described in Johnson, U.S. 8,934,992 NERVE CUFF, issued on January 13, 2015, and/or in Rondoni, CUFF ELECTRODE, published as WO 2019/032890 on February 14, 2019 (and filed as U.S. application Serial Number 16/485,954 on August 14, 2019), both which are hereby incorporated by reference in their entirety. Moreover, in some examples the stimulation lead 2117 may comprise at least some of substantially the same features and attributes as the stimulation lead described in U.S. Patent No. 6,572,543 to Christopherson et al., and which is incorporated herein by reference.

However, it will be understood that in some examples the IPG 2133 also may take the form of a microstimulator, which is sized and placed, in the head-and-neck region 2103 in close proximity to the upper airway patency-related nerve 2105 to be stimulated. In some such examples, the microstimulator 2133 also may incorporate and/or include the stimulation electrode 2112 and/or sensing electrodes. In some example implementations in which the IPG 2133 may comprise a microstimulator, then placement of the microstimulator in the head-and-neck region 2103, such as in close proximity to the upper airway patency-related nerve would also place any exposed electrodes (e.g. 2135) on microstimulator in closer proximity to the nerve 2105, as well as in closer proximity to the head portion 2105 from which EEG information (including sleep information) may be determined via such electrode 2135. In some examples, such example microstimulators may comprise at least some of substantially the same features and attributes as described in association with at least MICROSTIMULATION SLEEP DISORDERED BREATHING (SDB) THERAPY DEVICE, published on May 26, 2017 as PCT Publication WO 2017/087681 from application PCT/US2016/062546 filed on November 17, 2016, and filed as U.S. application Serial Number 15/774,471 on May 8, 2018, both of which are which is incorporated herein by reference. In such example arrangements including a microstimulator as the IPG 2133, the stimulation lead 2117 may be omitted (while still retaining stimulation electrode 2112) or the stimulation lead 2117 may be significantly shortened.

Via such neurostimulation device(s) (2133, 2112), delivery of a stimulation signal to the upper airway patency-related nerve 2105 may cause contraction of at least some upper airway patency muscles (e.g. the genioglossus muscle) to cause at least protrusion of the tongue to maintain or restore upper airway patency, and thereby provide therapeutic treatment of obstructive sleep apnea. At least some further example implementations regarding such stimulation are described in association with at least FIGS. 27A-36.

In some examples, the example microstimulator may be implanted in the head-and-neck region (e.g. 2103) of a patient to sense at least some of the desired sleep-wake-related information, which may be used to perform sleep-wake determination. In some examples, sleep-wake determination may be used to implement, control, adjust, etc. therapy of sleep disordered breathing per neurostimulation of upper-airway-patency related nerves, muscles, tissues, etc. At least some example implementations of a head-and-neck implanted microstimulator may take the form described later in association with at least FIGS. 25B-25F.

In some examples, such as described later in FIGS. 25B-25F, the device implanted within the head-and-neck region may comprise a sensing element forming a part of and/or associated with the microstimulator. In some such examples, the sensing element(s) may be used to determine sleep-wake via detection of cardiac signals such as heart rate based on ECG or arterial motion. In some examples, the sensing element(s) may be used to determine sleep-wake via detection of respiratory signals such as respiratory motion or the subset of such motions that could be considered sounds including, but not limited to, snoring. In some such examples, the sensing element(s) may detect both cardiac signals and respiratory signals.

In some examples, whether involving microstimulation or involving other implantable pulse generators (IPG 2133 in FIG. 25A), changes to sensed signals after and/or during stimulation may be used to quantify therapy effectiveness and/or may be used to implement auto-titration of the stimulation, as further described later in association with at least FIGS. 27A-27E.

In some examples, the stimulation electrode 2112 also may serve as a sensing element to sense physiologic information. In some such examples, the electrode 2112 may act as the sole sensing element to sense the physiologic information, such as a single channel EEG electrode or a single channel ECG electrode or other sensing modalities per sensing portion 2000 (FIG. 24) or sensing portion 2500 (FIG. 27A). As noted below, in some examples the stimulation electrode 2112 may be used for sensing in combination with other sensing elements and/or sensing modalities.

In some examples, the stimulation lead body 2118 may comprise a sensing element (e.g. electrode) 2120, which may act as the sole sensing element to sense the physiologic information, such as cardiac information, EEG information, EMG information, movement information, etc. in accordance with sensing portion 2000 (FIG. 24), 2500 (FIG. 27A). Accordingly, in some examples, the sensing element 2120 may comprise an accelerometer. However, in some examples a sensing element (e.g. electrode) 2120 may be considered the sole sensing element when used in association an electrically conductive exterior portion (e.g. at least part of a case/housing) of an implantable stimulator (e.g. IPG or microstimulator).

In some examples, a single/sole sensor may comprise a pressure sensor (e.g. 2037 in FIG. 24), and in some examples, pressure sensed via sensor 2037 may being tracked, evaluated, etc. via pressure parameter 2537 in sensing portion 2510 of care engine 2500 in FIG. 27A.

In some examples, the EMG information sensed via one of the electrodes (e.g. 2120, 2112, etc.) may comprise detecting upper airway patency to assess obstruction (e.g. degree, location, etc.) and/or assess stimulation effectiveness, as well as detecting (and/or assessing) inhalation/exhalation during respiration. In some examples, the sensed EMG information may comprise sensing intercostal muscle activity in order to identify respiratory cyclical information (e.g. inspiration, expiration, expiratory pause) and/or identify or differentiate between central sleep apnea and obstructive sleep apnea.

However, in some examples, one or both of electrode 2112 and electrode 2120 may be used in association with other sensing elements (e.g. electrodes) to sense physiologic information.

In some examples, IPG 2133 comprises a sensing element(s) 2135. In some such examples, the sensing element 2135 is located on a surface of (or forms) a case of IPG 2133, and one of both of electrodes 2112 and 2120 may be used in association with electrode 2135 to measure bioimpedance (2036 in FIG. 24; 2536 in FIG. 27A), to obtain an ECG signal, an EMG signal, etc. sense cardiac information (including cardiac morphology), respiratory information (including respiratory morphology), motion/movement of the chest, neck, and/or head, etc.

In some such examples, the sensing element 2135 of the IPG 2133 may comprise an accelerometer, which may comprise a single axis or multiple-axis accelerometer. The accelerometer may be located internally within the IPG 2133, may be located externally on the IPG 2135, or may extend a short distance from the IPG 2133 via a small lead body.

As discussed in association with at least parameters 2026, 2526 in FIGS. 24 and 27A, respectively, the accelerometer may be employed to sense motion at (or of) the chest, neck, and/or head, cardiac information, respiratory information, etc. In some examples, the accelerometer may be used to sense body activity/movement/motion, such as gross body motion (e.g. walking, talking), which may be indicative of activity associated with wakefulness. Alternatively, sensing a lack of activity via an accelerometer may be indicative of a sleep state, in some examples. In some such examples, the accelerometer may be used to sense physiologic information for use in at least some of the example methods of determining sleep-wake status without being used to sense posture or body position, as previously described herein. However, in some examples, the accelerometer may be used to sense such posture or body position.

With further reference to FIG. 25A, in some examples of determining a sleep-wake status, an electrode 2110 may be implanted subdermally in a head portion 2105 of a head-and-neck region 2103 (e.g. above the shoulder) to sense electrical brain activity and obtain EEG information and/or other central nervous system (CNS) information, with such sensed information being used to determine the sleep-wake status. Among other aspects, sleep onset, sleep termination, and/or various sleep stages may be determined via the sensed EEG information. In some examples, multiple electrodes 2110 may be placed subdermally about the head portion 2015 to sense such EEG information. In some examples, a single electrode 2110 may be used in combination with another electrode, such as the stimulation electrode 2112, to sense such EEG information. In some examples, the electrode 2110 may comprise the sole sensing element used to determine sleep-wake status.

In some example methods and/or devices of determining a sleep-wake status, an electrode 2114 may be implanted in or in close proximity to a tongue 2115 to sense electromyography (EMG) information. This sensed EMG information may include signals which are indicative of voluntary control of the tongue (e.g. talking, eating, etc.), which in turn may be indicative of wakefulness. In addition, this sensed EMG information may include signals which are indicative of sleep and/or sleep disordered breathing (e.g. obstructive events) such as when the tongue may relax into a position obstructing the upper airway.

It will be understood that just some of the various electrodes shown in FIG. 25A may be implanted or present in a particular example implementation. Moreover, while some of the electrodes (if present) may be used in combination with each other, some of the electrodes may be used to implement a particular sensing modality periodically or selectively rather than all of the time. For instance, there may be periods of time in which some electrodes are used to sense one modality (e.g. cardiac information, such as an ECG or other), while some electrodes are used to sense another modality (e.g. impedance) during some periods of time, with such periods of time being overlapping, coincidental, or independent of each other.

With this in mind, in some examples, one or multiple sensing modalities for determining wake-sleep status may be implemented in some instances while another, a different sensing modality (or a different combination of sensing modalities) may be implemented in other instances. For example, certain sensing modalities may be employed solely or less significantly during a portion of the daily period (e.g. normal wake period, such as 6 a.m. to 10 p.m.) and then not employed at all or less significantly during another portion of the daily period (e.g. normal sleep period), or vice versa.

In some example methods and/or devices, the normal wake period may be identified via at least one of clinician input, patient input, machine learning, and other observational criteria. In the example of clinician input or patient input, a user may directly specify the start time and/or end time of the normal wake period (and conversely the normal sleep period). In some examples, the normal wake period (or conversely the normal sleep period) may be at least partially determined via historical data for a particular patient and/or historical data regarding multiple patients or the general population. In some such examples, machine learning (e.g. 3230 in FIG. 27A) may be applied to the historical data to make the determination. In some such examples, the machine learning may be on-going on a daily basis using at least the most recent historical data (e.g. last 30 days).

In some examples, as described later, a probability of sleep (or the sleep-wake status) may be determined from among a plurality of sleep-wake status parameters in which different sleep-wake status parameters may be weighted differently. Such different weighting for a given sleep-wake status parameter may depend on a time-of-day, clinician/patient input, etc. These aspects and/or other aspects of determining a probability of sleep are further described in association with at least FIGS. 33-36.

As schematically represented in FIG. 25AA, in some examples the IPG 2133 of FIG. 25A may comprise a plurality of sensing elements (e.g. electrodes 2145, 2147) mounted on, or formed as part of, an outer surface (e.g. case) of the IPG 2133. As previously described elsewhere, this arrangement may be used to sense cardiac information (e.g. ECG, other), impedance, etc.

In some examples, whether mounted on a single housing (e.g. IPG 2133 in FIG. 25AA) or placed in multiple different location (or on different components), the electrode(s) shown in FIG. 25A, 25AA may be used to sense cardiac information (including cardiac morphology), respiratory information (including respiratory morphology), motion/movement of the chest and/or neck, etc., as described in association with sensing portion 2000 (FIG. 24) and/or sensing portion 2510 (FIG. 27A). In some examples, this sensed information may comprise a respiratory rate and/or a heart rate. In some such examples, the sensed respiratory information and/or cardiac information may comprise at least some of substantially the same features and attributes as described in association with respiration portion 2580, cardiac portion 2600 in FIG. 27A, cardiac diagram 3500 in FIG. 27B, and/or respiratory diagram 150 in FIG. 27C.

In some examples, the respiratory information is obtained via measuring trans-thoracic impedance solely via the electrodes on the IPG or via electrodes in addition to those present on the surface of the IPG. However, in some examples, the respiratory information may be derived from the ECG information.

With this in mind, in some examples described elsewhere in the present disclosure, the respiratory information and/or cardiac information may be obtained via an accelerometer (2026 in FIG. 24), which may be located in the IPG 2133. As previously noted, there may be times at which the accelerometer is used to sense respiratory information, cardiac information, and/or other information in order to determine sleep-wake status while at other times, sensing modalities (e.g. ECG electrodes, EMG electrodes, etc.) other than an accelerometer may be used to sense respiration information, cardiac information, and/or other information to determine sleep-wake status.

Unless noted specifically otherwise, it will be understood that the electrodes described in FIG. 25A comprise an exposed electrically conductive portion to engage bodily tissues, etc. within the patient.

In some examples, a single SDB care device comprises a single housing. In some examples, the single device comprises an on-board power source. In some examples, a single device comprises a plurality of sensing elements (e.g. electrodes). In some examples, at least one sensing element (e.g. electrode) is located on two separate portions of a device. For instance, one electrode may be located on IPG 2133 while one electrode may be located on a stimulation lead body 2118.

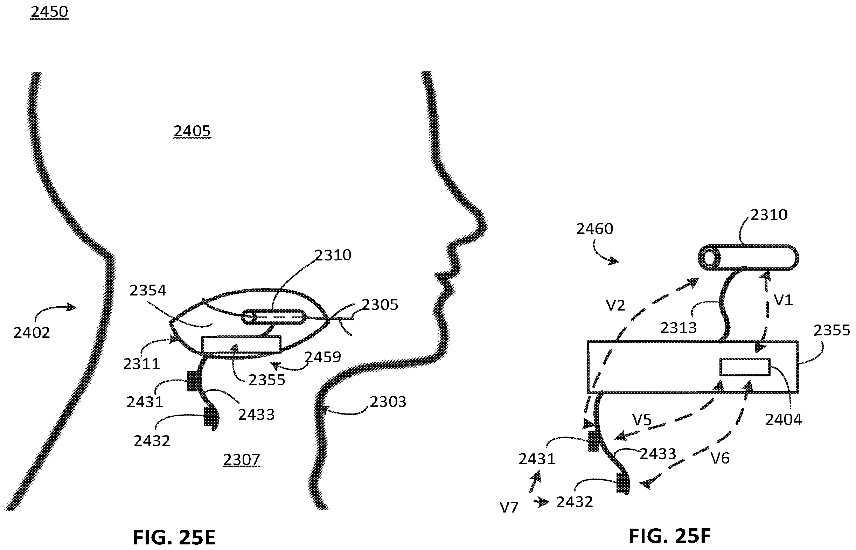

As previously described in association with at least FIGS. 25A, 25AA, in some examples an implantable pulse generator (IPG) may take the form of a microstimulator, and may be used to implement various sensing modalities as previously described. At least some example implementations of such a microstimulator are shown in at least FIGS. 25B and 25E.

As shown in FIG. 25B, an example device 2359 including an example microstimulator 2355 may be implanted in a head-and-neck region 2302 of a patient, and in particular in the neck region 2303 in this example. The microstimulator 2355 is implanted subcutaneously via access-incision 2311. In the particular illustrated example, a stimulation electrode 2310 is electrically connected to and extends from the microstimulator 2355, with stimulation electrode 2310 coupled to nerve 2305 to stimulate the nerve, which causes contraction of musculature (e.g. tongue) maintain or restore upper airway patency to treat sleep disordered breathing. In some examples, the stimulation electrode 2301 may comprise at least some of substantially the same features and attributes as stimulation electrode 2112 in FIG. 25A, including acting as a sensing electrode in some examples.

As further shown in the schematic representation of an example device 2400 in FIG. 25C, in some examples the microstimulator 2355 may comprise at least one electrode (e.g. 2402 and/or 2404) relative to which sensing vectors V1, V2, and/or V3 among electrodes 2310, 2402, 2404 may be established to sense physiologic phenomenon (e.g. ECG, bioimpedance, motion at (or of) the neck 2303, etc.) as previously described. This sensed physiologic information may be used to determine a sleep-wake status, among other things, such as implementing stimulation therapy. It will be further understood that in some examples, additional sensing modalities (e.g. EMG) described in association with FIG. 25A, 24, and 27A may be implemented via at least a portion of the microstimulation devices of FIGS. 25B-25F. While not fully shown in FIG. 25B, FIG. 25C illustrates that electrode 2310 may be arranged on a lead 2313 extending from microstimulator 2355.

As further shown in the schematic representation of an example device 2420 in FIG. 25D, in some examples the microstimulator 2355 may comprise an accelerometer 2422 and by which sensing physiologic information (e.g. via sensing motion at or of the neck, etc.) may be implemented as previously described throughout the present disclosure. In addition, the microstimulator 2355 in FIG. 25D also may comprise an electrode 2402 (as in FIG. 25C) by which at least some of the previously described sensing (e.g. cardiac, ECG, bioimpedance, motion, etc.) may be implemented via sensing vector V2. This sensed physiologic information may be used to determine a sleep-wake status, among other things, such as implementing stimulation therapy.

FIG. 25E provides a schematic representation 2450 of an example device 2459 which comprises at least some of substantially the same features as the devices of FIGS. 25B-25D, except further comprising a dedicated sensing lead 2433 extending (subcutaneously) into tissue from microstimulator 2355 to support at least one electrode (e.g. 2431, 2432) spaced apart from microstimulator 2355 and/or other electrodes (e.g. 2431, 2432 or 2404 in FIG. 25F). This arrangement may be used to sense physiologic information (e.g. ECG, bioimpedance, motion at or of neck 2303) via vectors V1, V2, V3, V5, V6, and/or V7 (FIG. 7) in a manner similar to that described for at least example arrangement in FIG. 25B-25D.

In some examples, the microstimulator devices of FIGS. 25B-25F facilitate SDB care, including sleep-wake determination, in a compact arrangement in which sensing, stimulation, implant-access, etc. may be implemented in a single body region (e.g. neck) instead of being dispersed among several body regions (e.g. neck and torso), thereby simplifying implantation and SDB care. For instance, the neck-located microstimulator devices may sense physiologic phenomenon (e.g. respiration, cardiac, etc.) which may sometimes primarily be associated with a different region of the body (e.g. chest) while simultaneously conveniently placing a stimulation element in the neck region in which the microstimulator is located.

FIG. 26 is a block diagram schematically representing an example processing portion 2200, which may form part of and/or be in communication with at least sensing portion 2000 (FIG. 24A). In general terms, the processing portion processes signals and/or information obtained by a single sensor, single sensor type, or multiple types of sensors as described in association with at least FIG. 24. As shown in FIG. 26, processing portion 2200 may comprise a filtering function 2210 to filter the sensed signals to exclude noise, non-relevant information, etc. In some examples, the processing portion 2200 may comprise interpretation function 2212, which may interpret the information sensed via sensing portion 2000 in light of sensed physiologic information present in typical sleep patterns. In some such examples, the interpretation may be performed, at least partially with respect to information associated with a reference parameter 2220. In some such examples, the information available via reference parameter 2220 (for interpreting sensed information) may comprise a respiratory rate and/or respiratory signal morphology and/or may comprise a cardiac rate and cardiac signal morphology. Normalization may or may not be utilized.

In some examples, the sensing portion 2000 (FIG. 24) and/or processing portion 2200 (FIG. 26) may be employed in methods to extract important features from sensor signals. Such feature extraction may comprise band-pass filtering, frequency analysis, power spectral analysis, signal amplitude analysis, derivative signal analysis, use of thresholds, and differential signal analysis. Moreover, in some examples, such feature extraction also may comprise amplification and gain control, outlier rejection methods, be based on physiological rates, and/or wavelet analysis, as well as combinations of the preceding parameters. In some examples, the feature extraction may relate to and/or be performed to enable analysis of periods of periodic behavior. For instance, feature extraction may be performed on the sensed signal and analyzing the extracted feature as a moving average or in discrete time chunks as a distribution to determine if the particular extracted feature (e.g. heart rate, heart rate variability, respiratory rate, etc.) has reached a threshold of stability or exhibits a change from the previous behavior.

In some examples, at least a part of processing portion 2200 may comprise, and/or be implemented, at least some of the features and attributes described in association with FIGS. 33-36.

In some examples, all or a portion of processing portion 2200 may be incorporated within sensing portion 2510 or other portions of care engine 2500 in FIG. 27A and/or may be incorporated within control portion 4000 (FIG. 28A).

FIG. 27A is a block diagram schematically representing an example care engine 2500. In some examples, the care engine 2500 may form part of a control portion 4000, as later described in association with at least FIG. 28A, such as but not limited to comprising at least part of the instructions 4011 and/or information 4012. In some examples, the care engine 2500 may be used to implement at least some of the various example devices and/or example methods of the present disclosure as previously described in association with FIGS. 1-26 and/or as later described in association with FIGS. 27B-36. In some examples, the care engine 2500 (FIG. 27A) and/or control portion 4000 (FIG. 28A) may form part of, and/or be in communication with, a pulse generator (e.g. 2133 in FIG. 25A-25B) whether such elements comprise a microstimulator or other arrangement.

In one aspect, at least the sensing portion 2510 of care engine 2500 in FIG. 27A directs the sensing of information, and/or receives, tracks, and/or evaluates sensed information obtained via one or more of the sensing modalities, sensing elements, etc. of sensing portion 2000 (FIG. 24), with care engine 2500 employing such information to determine sleep-wake status, among other actions, functions, etc. as further described below.

As shown in FIG. 27A, in some examples the care engine 2500 comprises a sensing portion 2510, a sleep state portion 2650, a sleep disordered breathing (SDB) parameters portion 2800, and/or a stimulation portion 2900. In some examples, the sensing portion 2510 may comprise an EEG parameter 2512 to sense EEG information, such as a single channel (2514) or multiple channels of EEG signals. Such sensed EEG information may be obtained via EEG sensor 2012 (FIG. 24) or derived from information sensed via another sensing modality. In some examples, the EEG information sensed per parameter 2512 comprises sleep state information. In some such examples, the sleep state information may comprise the parameters provided in the later described sleep state portion 2650 of care engine 2500.

In some examples, the sensing portion 2510 may comprise an electroocoulogram (EOG) parameter 2524, which relates to receiving, tracking, evaluating, and/or directing sensing of eye movement, eye position, etc., such as via an EOG sensor (e.g. 2024 in FIG. 24). In some such examples, the sensing element may comprise an optical sensor.

In some examples, this EOG information may be used as part of determining and/or confirming sleep state information, among other CNS information (2532) which may be used to sense, diagnose, and/or treat sleep disordered breathing (SDB) behavior. For instance, in some such examples, this EOG information may comprise detection and/or tracking of rapid eye movement (REM) per parameter 2668 (FIG. 27A) during sleep, which in turn may be used in differentiating between an awake state, REM state, and/or other sleep states, including various sleep stages.

As further shown in FIG. 27A, the care engine 2500 may comprise a sleep state portion 2650 to sense and/or track sleep state information, which may be obtained via the EEG information parameter 2512, in some examples. In some examples, the sleep state portion 2650 may identify and/or track onset (2660) of sleep and/or offset (2662) of sleep, as well as identify and/or track sleep stages once the patient is asleep. Accordingly, in some examples, the sleep state portion 2650 comprises sleep stage parameter 2666 to identify and/or track various sleep stages (e.g. REM and N1, N2, N3 or S1, S2, S3, S4) of the patient during a treatment portion or during longer periods of time. In some instances, the various stages (e.g. N1-N3 or S1-S4) other than REM sleep may sometimes be referred to as non-REM sleep. The sleep state portion 2650 also may comprise, in some examples, a separate rapid eye movement (REM) parameter 2668 to sense and/or track REM information in association with various aspects of sleep disordered breathing (SDB) care, as further described below and throughout various examples of the present disclosure. In some examples, the REM parameter 2668 may form part of, or be used with, the sleep stage parameter 2666.

In some examples, the sleep state portion 2650 may comprise an wakefulness parameter 2664 to direct sensing of, and/or to receive, track, evaluate, etc. sensing a wakeful state of the patient. An awake state of a patient may be indicative of general non-sleep periods (e.g. day time) and/or of interrupted sleep events, such as macro-arousals (per parameter 2672) associated with a patient waking up to use the restroom (e.g. urinate, etc.), rolling over in bed, waking up in the morning to turn off their alarm, and the like.

Conversely, in some examples, the sleep state portion 2650 may comprise a micro-arousal parameter 2674, by which one may detect and/or track neurological arousals associated with sleep disordered breathing (SDB) events in which a patient experiences a short neurological arousal due to sleep apnea, such as but not limited to obstructive sleep apnea, central sleep apnea, and/or hypopneas. Such SDB-related micro-arousals typically do not result in the patient waking up, in the traditional sense familiar to a lay person. In at least some examples, the stimulation intensity within a treatment period is not varied in response to such SDB-related micro-arousals as one goal of the therapy is for the electrical stimulation to prevent or substantially reduce sleep disordered breathing, which in turn would lessen the frequency and volume of such SDB-related micro-arousals.

In some examples, via at least the sleep state portion 2650 of care engine 2500, the sleep detection method/device may differentiate between wakefulness and sleep disordered breathing (SDB), which occurs during sleep. Among other situations, this differentiation may enable effective neurostimulation therapy such as when a patient is in a sleep position (e.g. laying horizontally or incline position) and the sleep detection arrangement detects a change in sensed data which could possibly be interpreted as a rolling over (e.g. from a supine position onto their side (e.g. lateral decubitus) or vice versa) or as consistent with a SDB behavior. In the case of a bona fide rollover by the patient, such as when getting out of bed, the system will pause the neurostimulation therapy. However, if the detected change may be confirmed as legitimate SDB behavior, then the system/method does not pause the neurostimulation therapy in at least some examples.

With this in mind, in some examples the device/method may differentiate between REM sleep (even where no sleep disordered breathing (SDB) is present) and wakefulness at least because if the patient is in REM sleep, the system avoids pausing neurostimulation therapy for sleep disordered breathing. Conversely, if the patient is in an actual wakeful state, the system should not initiate neurostimulation therapy or may act to pause or to terminate neurostimulation therapy. In some examples, one characteristic feature associated with REM is a lack of body motion, which may sometimes be referred to as paralysis or at least partial paralysis of voluntary muscle control.

In some examples, sleep disordered breathing may occur during REM sleep, such that at least some example device/methods may differentiate sleep disordered breathing from wakefulness and/or differentiate REM sleep from wakefulness. For instance, in some such examples, sensing a lack of body motion may prevent a false positive if/when other parameters (e.g. HR) might otherwise be indicative of wakefulness. For example, during REM sleep stage, sensed information may indicate increased variability in the respiratory period and/or in the heart rate (HR) of the patient.

In some such examples and as previously described, the sleep state information (per sleep state portion 2650) may be used to direct, receive, track, evaluate, diagnose, etc. sleep disordered breathing (SDB) behavior. In some such examples and as previously described, the sleep state information may be used in a closed-loop manner to initiate, terminate, and/or adjust stimulation therapy to treat sleep disordered breathing (SDB) behavior to enhance device efficacy. At least some example closed-loop implementations are further described later in association with at least parameter 2910 in FIG. 27A.

For instance, in some examples via sensing wakefulness (2664 in a sleep state portion 2650), stimulation therapy may be terminated automatically. In some examples, via sensing commencement of particular sleep stages (2666), stimulation therapy may be initiated automatically. In some examples, the intensity of stimulation therapy may be adjusted and implemented according to a particular sleep stage and/or particular characteristics within a sleep stage. In some examples, a lower stimulation intensity level may be implemented upon detecting a REM sleep stage. In some examples, stimulation intensity may be decreased in some sleep stages to conserve power and battery life as well as to improve patient comfort and/or therapy utilization.

In some examples, in cooperation with at least sleep stage parameter 2666 of care engine 2500, delivery of a stimulation signal may be toggled among different predetermined intensity levels for each different sleep stage (e.g. N1, N2, N3 or S1, S2, S3, S4, REM).

In addition to the above described sensing parameters, modalities, etc. described in association with FIG. 27A, in some examples, the sensing portion 2510 of care engine 2500 comprises an ECG parameter 2520, EMG parameter 2522, accelerometer parameter 2526, pressure parameter 2537, temperature parameter 2538, acoustic parameter 2539 to direct sensing of, and/or to receive, track, evaluate, etc. sensing signals from the previously described ECG sensor 2020, EMG sensor 2022, accelerometer 2026, pressure parameter 2037, temperature sensor 2038, and/or acoustic sensor 2039 in association with FIG. 24. In some examples, the EMG parameter 2522 may comprise detecting muscle activity and/or motion at interscostal muscles, the upper airway, and/or the tongue, such as described in association with at least FIG. 25A and other examples throughout the present disclosure.

In some examples, the sensing portion 2510 of care engine 2500 (FIG. 27A) comprises an impedance parameter 2536 to sense and/or track sensing of impedance within the patient's body to sense motion at (or of) the chest and/or neck and/or other parameters in order to determine sleep-wake status. In addition to or instead of being used to determine sleep-wake status, the impedance parameter 2536 also may be used to sense respiratory information, and/or other information in association with sleep disordered breathing (SDB) care. The impedance parameter 2536 may obtain impedance information from impedance sensor 2036 in FIG. 24 and/or other sensors.

In some examples, sensing portion 2510 of care engine 2500 may comprise a posture parameter 2540 to direct sensing of, and/or to receive, track, evaluate, etc. sensing signals from the previously described posture sensor 2040 in FIG. 24 or other posture, body-position sensor, etc. Like the other parameters of sensing portion 2510, the posture parameter 2540 may be used alone or in combination with other parameters to determine a sleep-wake state of the patient. As previously noted, however, in some example methods (and/or devices) a determination of sleep-wake status may be made without (or independent of) posture information.

In some examples, sensing portion 2510 of care engine 2500 comprises a snoring parameter 2545 to direct sensing of, and/or to receive, track, evaluate, etc. snoring information, which in some examples may be detected and obtained via motion sensing. This sensed snoring information may be used, in some examples, to at least partially determine a sleep-wake status. In one aspect, snoring may be defined as noise associated with each exhalation when respiratory periods are relatively stable and with stable frequency content. Conversely, talking lacks stable respiratory periods and frequency content, and therefore would not be detected as snoring. As noted elsewhere, in some examples the snoring is sensed via acoustic sensor 2039 (FIG. 24) and/or acoustic parameter 2539 (FIG. 27A).

In some examples, sensing portion 2510 of care engine 2500 may comprise a history parameter 2542 by which a history of sensed physiologic information is maintained, and which may be used via comparison parameter 2544 to compare recent sensed physiologic information with older sensed physiologic information. At least some example implementations of using such history parameter 2542 and comparison parameter 2544 are described in association with at least FIGS. 15A-15B.

In some examples, via care engine 2500, at least some example methods to determine a sleep-wake status may comprise identifying sleep via trends (including variability) in a respiratory rate and/or in a heart rate. In some examples, determination of the sleep-wake status may comprise identifying sleep via a morphology of respiratory cycles, via stability of a respiratory rate, and/or stability in the respiratory morphology. At least some of these examples are further described below in association with at least respiration portion 2580 of care engine 2500.

As shown in FIG. 27A, in some examples, care engine 2500 may comprise a respiration portion 2580. In at least some examples, in general terms respiration portion 2580 may direct sensing of, and/or receive, track, and/or evaluate respiratory morphology, including general patterns and/or specific fiducials within a respiratory signal. In some examples, the respiration portion 2580 may operate in cooperation with, or as part of, sensing portion 2510 of care engine 2500 in FIG. 27A and/or sensing portion 2000 (FIG. 24). At least some aspects of such respiratory morphology managed via respiration portion 2580 may comprise inspiration morphology (parameter 2582) and/or expiration morphology (parameter 2584). In some examples, the respective inspiration morphology parameter 2582 and/or expiration morphology parameter 2584 may comprise amplitude, duration, peak (2586), onset (2588), and/or offset (2590) of the respective inspiratory and/or expiratory phases of the patient's respiratory cycle. In some examples, the detected respiration morphology may comprise transition morphology (2592) such as an inspiration-to-expiration transition and/or an expiration-to-inspiration transition. In some examples, any one or more of these aspects (e.g. peak, onset, offset, magnitude, etc.) of the respective inspiratory and expiratory phases may be used to at least partially determine sleep and/or wakefulness.

For example, the inspiration-to-expiration transition associated with respiration portion 2580 of care engine 2500 may be used as a fiducial to detect and/or track a respiratory rate (and respiratory rate variability), which may be indicative of a change in wake-sleep status. At least one such example inspiration-to-expiration transition 180 is shown in FIG. 27C. In some examples, changes in a duration of the inspiration-to-expiration transition, changes in peak-to-peak amplitude, and/or changes in the respiratory rate may be indicative of sleep and/or wakefulness, and therefore used to determine a sleep-wake status.

With regard to the sensing, tracking, etc. of respiratory morphologies described above, FIG. 27C is a diagram 150 schematically representing a respiratory cycle 150 which illustrates at least some aspects of respiratory morphology, with respiratory cycle 150 including an inspiratory phase 162 and an expiratory phase 170. The inspiratory phase 162 includes an initial portion 164 (e.g. onset), inspiratory peak 165, end portion 166 (e.g. offset), while expiratory phase 170 includes an initial portion 174 (e.g. onset), intermediate portion 175 (including expiratory peak 177), and end portion 176 (e.g. offset). The above-noted peak parameter 2586, onset parameter 2588, and offset parameter 2590 of the inspiration morphology 2582 (in sensing portion 2510 of care engine 2500) corresponds to the inspiration peak 165, inspiration onset 164, and inspiration offset 166 of the respiratory cycle diagram 150 in FIG. 27C, while the above-noted peak parameter 2586, onset parameter 2588, and offset parameter 2590 of the expiration morphology parameter 2584 ( in sensing portion 2510 of care engine 2500) corresponds to the expiratory peak 177, expiratory onset 164, and expiratory offset 166 of the respiratory cycle diagram 150 in FIG. 27C.

In the respiratory cycle diagram 150 in FIG. 27C, a first transition 180 occurs at a junction between the end inspiratory portion 166 and the initial expiratory portion 174. In some instances, this transition 180 may sometimes be referred to as an inspiration-to-expiration transition 180, which as noted above may be used to determine a sleep-wake status per parameter 2592 of respiration portion 2580 of care engine 2500 in FIG. 27A. A second transition 182 occurs at a junction between the end expiratory portion 176 and the initial inspiratory portion 164. In some instances, this transition 182 may sometimes be referred to as an expiration-to-inspiration transition 182, which as noted above may be used to determine a sleep-wake status per parameter 2592 of respiration portion 2580 of care engine 2500 in FIG. 27A.

In some examples, as shown in FIG. 27A the respiration portion 2580 may comprise a chest wall parameter 2594 to direct sensing of and/or receive, track, evaluate, etc. chest wall behavior of the patient. In some such examples, the chest behavior may comprise chest wall motion (e.g. ribcage motion). In some examples, the sensed chest wall motion (e.g. used in determining sleep-wake status) may comprise general motion (e.g. rise and fall) of the chest wall associated with inspiration and expiration of a respiratory cycle as the patient breathes. In some instances, this chest wall motion may comprise intercostal muscle contraction. In some examples, this sensed general chest wall motion (e.g. used in determining sleep-wake status) does not include characteristics such as pectoral muscle contraction and/or signal information (which may be unrelated to breathing and/or cardiac function). Among other uses, the sensed chest motion may be used to determine respiratory information, cardiac information and/or other physiologic information in order to determine a sleep-wake status, as further described throughout various examples of the present disclosure. For instance, one use of the sensed chest motion is to at least partially determine whether respiration is passive or active (e.g. forced), which in turn may be used to determine a sleep-wake state. As just one example aspect of passive respiration, normal exhalation occurs without direct muscular effort, as during normal tidal breathing when air may be expelled from the lungs as a result of the recoil effect of elastic tissues in the chest, lungs, and diaphragm. This behavior would be expected in a sleep state. In contrast, one example of active respiration, which may be associated with an awake state, includes forced exhalation which involves contraction of the abdominal wall, internal intercostal muscles, and diaphragm.

In some examples, as shown in FIG. 27A the respiration portion 2580 may comprise a neck parameter 2595 to direct sensing of and/or receive, track, evaluate, etc. neck movement of the patient, which may be indicative of respiratory information and/or cardiac information regarding the patient, which may be used to determine a sleep-wake state. As previously described, such sensed movement of the neck and/or at the neck may comprise movement such as (but not limited to) motion from the airway and/or blood vessels, impedance, and/or other physiologic phenomenon. For instance, at least some sensed impedance vectors may be measured across the airway, across a vessel, and/or across both.

In some examples, the respiratory portion 2580 may comprise a respiratory rate parameter 2596 to direct sensing of, and/or receive, track, evaluate, etc. respiratory rate information including a respiratory rate, respiratory rate variability 2597, etc., which may be used to determine a sleep-wake status or change in sleep-wake status. In some examples, sensing the respiratory rate (and any associated variability, trends, etc.) may be implemented via sensing and tracking one of the above-noted identifiable parameters (e.g. peak, onset, offset, transition) of respiration morphology per respiratory portion 2580 of care engine 2500.

As shown in FIG. 27A, in some examples the care engine 2500 may comprise a cardiac portion 2600. In some examples, in general terms the cardiac portion 2600 may be employed to sense, track, determine, etc. cardiac information, which may be indicative of a sleep-wake status, among other information pertinent to SDB care. In some examples, the cardiac portion 2600 may operate in cooperation with, or as part of, sensing portion 2510 of care engine 2500 (FIG. 27A) and/or sensing portion 2000 (FIG. 24). The cardiac portion 2600 may be employed, alone or in combination with, other elements, modalities, etc. of the care engine 2500. In some examples, the cardiac portion 2600 may employ a single type of sensing or multiple types of sensing in sensing portion 2510, and in some examples, the cardiac portion 2600 may employ other sensing types, modalities, etc. in addition to, or as an alternative to, the particular sensing types, modalities of sensing portion 2510. Moreover, the cardiac portion 2600 may determine, track, etc. a sleep-wake status in cooperation with, or independent of, the respiration portion 2580 of care engine 2500.

In some examples, in general terms the cardiac portion 2600 may direct sensing of, and/or receive, track, evaluate, etc. cardiac signal morphology to at least determine a sleep-wake status. As shown in FIG. 27A, in some examples the cardiac portion 2600 comprises an atrial morphology parameter 2610 and/or a ventricular morphology parameter 2612, which may be employed alone, or in combination, to determine a sleep-wake status. In some examples, at least some aspects of the respective atrial and ventricular morphologies (2610, 2612) may comprise detecting contraction (parameter 2620) and/or relaxation (parameter 2622) of the atria and ventricles, respectively. In some such examples, the tracking of the respective contraction and/or relaxation may facilitate determining a sleep-wake status by providing a readily identifiable portion of a cardiac waveform by which heart rate (HR) and/or heart rate variability (HRV) may be detected, tracked, and from which values, trends, etc. of the heart rate or heart rate variability may indicate sleep or wakefulness.

In some examples, at least some aspects of the respective atrial and ventricular morphologies (2610, 2612) may comprise a peak (2630) of an atrial or ventricular contraction, which may be used to determine a sleep-wake status.

In some examples, at least some aspects of the respective atrial and ventricular morphologies (2610, 2612) may comprise an onset (e.g. start) 2632 of an atrial contraction, of an atrial relaxation, of a ventricular contraction, or of a ventricular relaxation. In some examples, at least some aspects of the respective atrial and ventricular morphologies (2610, 2612) may comprise an offset (e.g. termination, end) 2634 of an atrial contraction, an atrial relaxation, a ventricular contraction, or ventricular relaxation.

In some examples, at least some aspects of the respective atrial and ventricular morphologies (2610, 2612) by which a sleep-wake status may be detected may comprise a combination of atrial and ventricular contraction.

In some examples, at least some aspects of the respective atrial and ventricular morphologies (2610, 2612) may comprise a transition (2640), such as a transition between different phases of the cardiac cycle.

In some examples, at least some aspects of the cardiac information (by which a sleep-wake status may be determined) may comprise opening or closing of a heart valve per parameter 2642. In some examples, such detection of opening and/or closing of a heart valve (per parameter 2642) also may be used to help determine the timing and/or occurrence of an onset and/or offset of a contraction (or relaxation) of an atria or ventricles in association with parameters 2610, 2612, 2620, 2622, 2630, 2632, 2634.

In some examples, cardiac information may comprise heart motion 2644, from which the above-described cardiac morphology parameters may be determined. The heart motion 2644 may be obtained via one or more of the various sensing modalities (e.g. accelerometer, EMG, etc.) described in association with at least FIG. 24.

As further shown in FIG. 27A, in some examples, cardiac information may comprise heart rate parameter 2645 to direct sensing of, and/or receive, track, evaluate, etc. heart rate information including a heart rate (HR), heart rate variability (HRV) 2646, etc., which may be used to determine a sleep-wake status or change in sleep-wake status. In some examples, sensing the heart rate (and any associated variability, trends, etc.) may be implemented via sensing and tracking one of the above-noted identifiable parameters (e.g. peak, onset, offset, transition) of cardiac morphology per cardiac portion 2600.

In some examples, at least some of the above-described cardiac information may be determined, at least partially, according to heart sounds (e.g. S1, S2, etc. in FIG. 27B), which may be sensed acoustically (e.g. 2039 in FIG. 24; 2539 in FIG. 27A).

In some examples, sleep-wake status may be determined via a combination of sensed respiratory features and sensed cardiac features. At least some aspects of use of this combination of information are previously described in association with at least FIGS. 5-9 and 10-13, and elsewhere throughout examples of the present disclosure.

As further shown in FIG. 27A, in some examples the care engine 2500 comprises a SDB parameters portion 2800 to direct sensing of, and/or receive, track, evaluate, etc. parameters particularly associated with sleep disordered breathing (SDB) care. For instance, in some examples, the SDB parameters portion 2800 may comprise a sleep quality portion 2810 to sense and/or track sleep quality of the patient in particular relation to the sleep disordered breathing behavior of the patient. Accordingly, in some examples the sleep quality portion 2810 comprises an arousals parameter 2812 to sense and/or track arousals caused by sleep disordered breathing (SDB) events with the number, frequency, duration, etc. of such arousals being indicative of sleep quality (or lack thereof). In some such examples, such arousals may correspond to micro-arousals as described in association with at least parameter 2674 in sleep state portion 2650 of care engine 2500 in FIG. 27A.

In some examples, the sleep quality portion 2810 comprises a state parameter 2814 to sense and/or track the occurrence of various sleep states (including sleep stages) of a patient during a treatment period or over a longer period of time. In some such examples, the state parameter 2814 may cooperate with, form part of, and/or comprise at least some of substantially the same features and attributes as sleep state portion 2650 of care engine 2500.

In some examples, the SDB parameters portion 2800 comprises an AHI parameter 2830 to sense and/or track apnea-hypopnea index (AHI) information, which may be indicative of the patient's sleep quality. In some examples, AHI information is sensed throughout each of the different sleep stages experienced by a patient, with such sensed AHI information being at least partially indicative of a degree of sleep disordered breathing (SDB) behavior. In some examples, the AHI information is obtained via a sensing element, such as one or more of the various sensing types, modalities, etc. in association with at least sensing portion 2000 (FIG. 24) and/or sensing portion 2510 (FIG. 27A), which may be implemented as described in various examples of the present disclosure. In some examples, AHI information may be sensed via a sensing element, such as an accelerometer located in either the torso or chin/neck region with the sensing element locatable and implemented as described in various examples of the present disclosure. In some examples, a combination of accelerometer-based sensing and other types of sensing may be employed to sense and/or track AHI information. In some examples, the AHI information is obtained via sensing modalities (e.g. ECG, impedance, EMG, etc.) other than via an accelerometer.

In some examples, determination of sleep-wake status may be implemented via a probability portion 3200 of care engine 2500 in FIG. 27A. In some such examples, via a selection parameter 3210, the probability portion 3200 may enable selective inclusion or selective exclusion of at least some sleep-wake determination parameters without directly affecting the general operation of determining sleep-wake status. In some examples, via the probability function 3200, a sensitivity parameter 3220 may be adjusted by a patient, clinician, caregiver to increase or decrease a sensitivity of determining the sleep-wake status via a particular parameter. In some examples, a machine-learning parameter 3230 may be implemented to assess and modify adjustments to probabilistic determinations of the sleep-wake status, including but not limited to, adjustments to any amplitude thresholds, duration thresholds, etc. associated with a probabilistic determination of sleep-wake status. In some examples, employing such probabilistic determinations may permit more granular controls of a patient's individual signals (used in combination to make the determination of sleep-wake status), which in turn, may enable balancing simple control with the capability of complex control and sensor flexibility when desired.

In some examples, via at least the machine-learning parameter 3230, the care engine 2500 may comprise and/or access a neural network resource (e.g. deep learning, convolutional neural networks, etc.) to identify patterns indicative of sleep from a single sensor of multiple sensors. In some examples, a decision tree-based expert resource also could be used to combine sensors or neural network output with other signals such as time of day or remote inputs/usage. One example implementation of machine learning, such as via parameter 3230, is further described later in association with at least FIGS. 33-36. At least some other example implementations are described throughout the present disclosure.

In some examples, via probability portion 3200, care engine 2500 may assign and apply a weight (parameter 3240) to be associated with each signal in order to increase (or decrease) the relative importance of a particular sensor signal in determining sleep-wake status.

In some examples, via a temporal emphasis parameter 3250 different thresholds may be selected for different times of a 24 hour daily period. For instance, during a first period (e.g. daytime such as Noon) some parameters may be de-emphasized and/or other parameters emphasized, while during a second period (e.g. late evening such as 10pm), some parameters may be emphasized in determining sleep-wake status while other parameters are de-emphasized. Alternatively, during the first period, the sensitivity of most or all parameters (for determining sleep-wake status) may be decreased and during the second period, the sensitivity of some or all parameters (for determining sleep-wake status) may be increased.

In some such examples, this adjustability via the temporal emphasis parameter 3250 may enhance sleep-wake determinations for a patient having non-standard sleep periods, such as a graveyard shift worker (e.g. works 11pm-7 am), because their intended sleep period (e.g. 8 am - 3pm) conflicts with a conventional sleep period (e.g. 10pm - 6 am).

In some examples, the probability function 3200 of care engine 2500 may implement a probabilistic determination of sleep-wake status based on sensing motion at (or of) the chest, neck, and/or head. In some such examples, an accelerometer and/or other sensors (e.g. impedance, EMG, etc.) may be employed to sense motion at (or of) the chest, neck, and/or head. In some such examples, per a differentiation parameter 3260, where sensing is performed via a sensor (e.g. accelerometer) with multiple signal components (e.g. a multiple axis accelerometer) or captures a signal (e.g. ECG) from which multiple different signals may be derived, an example method may comprise dividing a signal associated with sensing the physiologic information into a plurality of different signals with each respective signal representing a different sleep-wake determination parameter. Stated differently, multiple components within a signal are differentiated into distinct and separate signals, each of which may be indicative of sleep-wake status. A probability of sleep-wake status is then determined based on assessing the respective different signals associated with the respective different sleep-wake determination parameters. As noted above, in some examples each respective different signal may comprise one axis of a multiple axis accelerometer (e.g. in which each axis is orthogonal to other axes) or may comprise a single axis accelerometer (when multiple single-axis accelerometers are employed). In some such examples, a different processing method or technique may be applied to at least some of the signal components (e.g. sleep determination parameters).

At least one example implementation of a dividing a signal in the above-described manner is provided later in the example method/device 7000 described in association with FIGS. 33-36.

As shown in FIG. 27A, in some examples the care engine 2500 may comprise an activation portion 3000, which in general terms may control activation of an implantable medical device, such as an IPG. In some such examples, nerve stimulation delivery via the implantable medical device may be activated and terminated automatically (3010), with such activation and termination based on sleep-wake status. In some such examples, the sleep-wake status is determined automatically via care engine 2500.

Accordingly, in some examples, via automatic parameter 3010, at least some example methods and/or devices for determining sleep-wake status may be used to automatically initiate a treatment period (e.g. upon automatically detecting sleep) and to automatically terminate a treatment period (e.g. upon automatically detecting wakefulness).

In some examples in which the automatic determination of a sleep-wake status is unavailable or deactivated by the patient (or clinician or caregiver), then per remote parameter 3012 the treatment period may comprise a period of time beginning with the patient using a remote control to turn on the therapy device and ending with the patient turning off the device via the remote control. In some examples, per remote parameter 3012, a treatment period may be initiated and/or terminated based on at least one a degree of ambient light sensed via a remote control, a degree or type of motion sensed by the remote control, and/or the above-described therapy activation (e.g. on, off) implemented via the remote control. In some examples, the remote control may comprise the remote control 4340 shown in FIG. 30. It will understood that, in some examples, detecting the degree of ambient light and/or the degree or type of motion of the remote control may be used as part of other features described herein to perform an automatic determination of sleep-wake status, which in turn may determine automatic initiation, termination, pause, adjustment, etc. of a treatment period in which neurostimulation therapy is applied. In some examples, remote control parameter 4340 may be implemented in association with method 788 in FIG. 20I.

In some examples in which the automatic determination of a sleep-wake status is unavailable or deactivated by the patient (or clinician or caregiver), then per app parameter 3013 the treatment period may comprise a period of time beginning with the patient using an app to turn on the therapy device and ending with the patient turning off the device via the app. In some examples, per app parameter 3013, a treatment period may be initiated and/or terminated based on at least one a degree of ambient light sensed via app, a degree or type of motion sensed by the mobile device, and/or the above-described therapy activation (e.g. on, off) implemented via the app on the mobile device. In some examples, the app may comprise the app 4330 shown in FIG. 30, which may be implemented via a mobile device 4320 (FIG. 30), such as a mobile smart phone, tablet, phablet, smart watch, etc. The mobile device may comprise a control portion, user interface (e.g. display) operate the app, and the mobile device may comprise sensor(s) to sense the above-described features (e.g. motion, ambient light, sounds, etc.) in manner to enable the app to perform sleep-wake determination at least partially based on the use (or non-use) of the mobile device.

In some examples, the sensor(s) of a remote control and/or mobile device may comprise an accelerometer, gyroscope, and/or other motion detector.

However, in some examples where automatic determination of sleep-wake status is unavailable (or deactivated), via the temporal parameter 3014 the treatment period may begin automatically at a selectable, predetermined start time (e.g. 10 p.m.) and may terminate at a selectable, predetermined stop time (e.g. 6 a.m.)

In one aspect, the treatment period corresponds to a period during which a patient is sleeping such that the stimulation of the upper airway patency-related nerve and/or central sleep apnea-related nerve is generally not perceived by the patient and so that the stimulation coincides with the patient behavior (e.g. sleeping) during which the sleep disordered breathing behavior (e.g. central or obstructive sleep apnea) would be expected to occur. Accordingly, to avoid enabling stimulation prior to the patient falling asleep, in some examples stimulation can be enabled during the treatment period after expiration of a timer started upon the automatic sleep detection. To avoid continuing stimulation after the patient wakes, stimulation can be disabled upon the automatic detection of wakefulness. Accordingly, in at least some examples, these periods may be considered to be outside of the treatment period or may be considered as a startup portion and wind down portion, respectively, of a treatment period.

In some examples, via a boundary parameter 3016 a selectable, predetermined first time marker (e.g. 10 pm) may be used as a limit or boundary to prevent automatic initiation of a treatment period (based on automatic detection of sleep) before the first time marker, and a selectable, predetermined second time marker (e.g. 6 am) may be used as a limit or boundary to ensure automatic termination of a treatment period to prevent continuance of a treatment period after the second time marker. Via such example arrangements, the treatment period may be initiated automatically via automatic sleep detection and/or may be terminated automatically via automatic wakefulness detection, while providing assurance to the patient of a treatment period not being initiated during normally wakeful periods, or not extending beyond their normal sleep period.

In some examples, determining sleep-wake status in association with boundary parameter 3016 may comprise and/or be combined with at least the features and attributes as previously described in association with method 780 in FIG. 20A, as well as in association with temperature parameter 2038 (FIG. 24) as previously described.

However, in some instances, via a physical parameter 3018 a user may take physical steps to cause activation (or deactivation) of a treatment period for the implantable medical device. For instance, via the activation portion 3000 and physical parameter 3018, the care engine 2500 may receive physical input such as tapping of the chest (or neck or head) or tapping over the implant to activate or deactivate the device. Alternatively, a user may use a patient remote control function 3012 to activate or deactivate the implantable medical device, which in turn may activate or deactivate delivery of nerve stimulation. In some such examples, activation or deactivation of the treatment period (in which nerve stimulation is applied) may be implemented via physical motion of a remote control or a mobile device (e.g. hosting an app). In some instances, via a clinician programmer or remote control, this physical feature (3018) may be activated or deactivated at the discretion of the clinician or user.

As further shown in FIG. 27A, in some examples care engine 2500 comprises a stimulation portion 2900 to control stimulation of target tissues, such as but not limited to an upper airway patency nerve, to treat sleep disordered breathing (SDB) behavior. In some examples, the stimulation portion 2900 comprises a closed loop parameter 2910 to deliver stimulation therapy in a closed loop manner such that the delivered stimulation is in response to and/or based on sensed patient physiologic information.

In some examples, the closed loop parameter 2910 may be implemented as using the sensed information to control the particular timing of the stimulation according to respiratory information, in which the stimulation pulses are triggered by or synchronized with specific portions (e.g. inspiratory phase) of the patient's respiratory cycle(s). In some such examples and as previously described, this respiratory information may be determined via a single type of sensing or multiple types of sensing via sensing portion 2000 (FIG. 24) and sensing portion 2510 (FIG. 27A).

In some examples in which the sensed physiologic information enables determining (at least) a sleep-wake state, the closed loop parameter 2910 may be implemented to initiate, maintain, pause, adjust, and/or terminate stimulation therapy based on the determined sleep-wake state (including particular sleep stages).

As further shown in FIG. 27A, in some examples the stimulation portion 2900 comprises an open loop parameter 2925 by which stimulation therapy is applied without a feedback loop of sensed physiologic information. In some such examples, in an open loop mode the stimulation therapy is applied during a treatment period without (e.g. independent of) information sensed regarding the patient's sleep quality, sleep state, respiratory phase, AHI, etc. In some such examples, in an open loop mode the stimulation therapy is applied during a treatment period without (i.e. independent of) particular knowledge of the patient's respiratory cycle information.

However, in some such examples, some sensory feedback may be utilized to determine, in general, whether the patient should receive stimulation based on a severity of sleep apnea behavior.

As further shown in FIG. 27A, in some examples the stimulation portion 2900 comprises an auto-titration parameter 2920 by which an intensity of stimulation therapy can be automatically titrated (i.e. adjusted) to be more intense (e.g. higher amplitude, greater frequency, and/or greater pulse width) or to be less intense (e.g. lower amplitude, lower frequency, and/or lower pulse width) within a treatment period.

In some such examples and as previously described, such auto-titration may be implemented based on sleep quality and/or sleep state information, which may be obtained via sensed physiologic information, in some examples. It will be understood that such examples may be employed with synchronizing stimulation to sensed respiratory information (i.e. closed loop stimulation) or may be employed without synchronizing stimulation to sensed respiratory information (i.e. open loop stimulation).

In some examples, at least some aspects of the auto-titration parameter 2920 may comprise, and/or may be implemented, via at least some of substantially the same features and attributes as described in Christopherson et al., SYSTEM FOR TREATING SLEEP DISORDERED BREATHING, issued as U.S. 8,938,299 on January 20, 2015, and which is hereby incorporated by reference in its entirety.

With regard to the various examples of the present disclosure, in some examples, delivering stimulation to an upper airway patency nerve is to cause contraction of upper airway patency-related muscles. In some such examples, the contraction comprises a suprathreshold stimulation, which is in contrast to a subthreshold stimulation (e.g. mere tone) of such muscles. In one aspect, a suprathreshold intensity level corresponds to a stimulation energy greater than the nerve excitation threshold, such that the suprathreshold stimulation may provide for maximum upper-airway clearance (i.e. patency) and obstructive sleep apnea therapy efficacy.

In some examples, at least some example methods may comprise identifying, maintaining, and/or optimizing a target stimulation intensity (e.g. therapy level) without intentionally identifying a stimulation discomfort threshold at the time of implantation or at a later point in time after implantation.

In some examples, upon determining sleep according to a minimum predetermined confidence level, an amplitude (e.g. intensity) of the stimulation signal may start a lower value and then be increased to higher values in a ramped manner. In some such examples, the increases in amplitude (up to a desired/target value) may be made dependent on additional or further predetermined confidence levels. However, if it is later determined that sleep is not occurring but rather that the patient is in a quiet, restful awake state, then the stimulation may be terminated or ramped down while still in the ramping phase, prior to reaching a target stimulation amplitude. Among other applications, this example method may be beneficial for patients with cardiac or respiratory disorders at least because the cardiac morphologies and/or respiratory morphologies (from which sleep may be detected) may be more complex such that accurate detection of actual sleep may be more challenging in such patients.

As noted above in association with the boundary parameter 3016 of the activation portion 3000, a clock or time keeping element within an implantable medical device (e.g. IPG 2133) may be used to implement boundaries or limit for when stimulation therapy (within a treatment period) may be automatically initiated or terminated via automatic sleep detection (or wake detection) per determining a sleep-wake status. In some examples, the time-based boundaries may be based on patient behaviors and/or direct clinician programming. In some examples, such tracked patient behavior may be used as input to a probabilistic model of determining a sleep-wake status. In some examples, the time-based boundaries also may be based, at least in part, on a history of patient activities.

In some examples, the time-based boundaries may account for daylight savings time and travel (e.g. different time zones), and may be adjusted via a patient remote control or physical tapping on the chest. In some such examples, a time-based boundary parameter may comprise one of multiple inputs used to determine sleep-wake status, and which may increase reliability in determining sleep-wake status in a variety of environments (rather than a single time-place environment such as only a patient bedroom).

In some examples, boundary parameter 3016 of activation portion 3000 in FIG. 27A may comprise criteria which are not strictly time-based (e.g. time of day). For instance, in some examples the boundary parameter 3016 may be implemented based on a number, type, and/or duration of various sleep stages associated with a single treatment period (e.g. a night's sleep). For instance, an example method may determine a boundary or an end limit of a treatment period according to observing a certain number (e.g. 4 or 5) of REM sleep periods, stage 4 sleep periods, or stage 3 sleep periods, etc. In some such examples, the number of particular sleep stage periods may be selectable. In some examples, the boundary may be based on a selectable percentage that a patient spends in one or more particular sleep stages.

In some examples, upon detecting a sleep state (per a sleep-wake status) a neurostimulation signal may be applied to a phrenic nerve, in order to treat central sleep apnea. In some examples, determining a sleep-wake status may be used to control initiation and/or termination of stimulation of both an upper airway patency nerve (e.g. hypoglossal nerve) and a diaphragm control nerve (in a manner coordinated relative to each other) to treat sleep disordered breathing.

In some examples, the stimulation portion 2900 may operate cooperatively with at least the respiration portion 2580 and/or the sensing portion 2510 of care engine 2500 (e.g. such as in association with sensing portion 2000 in FIG. 24) to determine efficacy of stimulation, and/or whether a flow limitation exists, by evaluating a flow response within a single respiratory cycle. Such evaluation stands in contrast to performing such evaluation on a cycle-to-cycle basis, such as looking at the respiratory signal from a peak of an inspiratory phase of one cycle to a peak of an inspiratory phase of another cycle.

For instance, in the example method, if the stimulation portion 2900 were to cause a change in the stimulation intensity level (e.g. increase or decrease) during the inspiratory phase, one feature of the care engine 2500 may comprise determining whether a substantial change (e.g. 10%, 15%, 20%, or more) in the flow response occurred.

In some examples, this feature also may be implemented as at least a portion of method as shown at 3600 in FIG. 27D, which may be performed with, or as a part of other example methods described herein. As shown in FIG. 27D, method 3600 may comprise, upon causing a change in stimulation intensity level during an inspiratory phase of a single respiratory cycle, determining if a change in flow response occurs during the inspiratory phase. For instance, if such a substantial change is determined, then it may provide confirmation of a flow limitation in the upper airway, such as obstructive sleep apnea was occurring. Upon such confirmation, then the method and/or device may further determine whether or not the current level of stimulation intensity is effective. For example, if no substantial change in the flow response were to occur upon a change in the stimulation intensity made during the inspiratory phase of a single respiratory cycle, then the method and/or device may determine that no change in the stimulation intensity is warranted or that the stimulation intensity may be decreased somewhat without diminishing efficacy of the therapy. However, if a substantial change in the flow response were to occur upon a change in the stimulation intensity made during the inspiratory phase of a single respiratory cycle, then the method and/or device may determine that a change (e.g. increase) in the stimulation intensity is warranted. Such determinations and/or implementations of a change in stimulation intensity may be implemented via at least the stimulation portion 2900 of care engine 2500. Some such sensing of the flow response, such as an aspect of a sensed respiratory waveform, may be implemented via sensing portion 2000 (FIG. 24), via sensing portion 2510 of care engine 2500 (FIG. 27A), and/or via respiratory portion 2580 of care engine 2500 (FIG. 27A).

In some such examples of stimulation portion 2900 in evaluating whether stimulation therapy is efficacious (based on a flow response of the inspiratory phase within a single respiratory cycle versus from cycle-to-cycle), some example methods may comprise determining whether a change (e.g. a substantial change) in the flow response were to occur upon a complete termination of stimulation or upon initiation of stimulation (such as when no stimulation was previously occurring) during an inspiratory phase of a single respiratory cycle, such as shown at 3610 in FIG. 27E.

In some examples associated with FIG. 27D and/or FIG. 27E, depending upon the determined flow response (such as whether a flow limitation is present) the stimulation portion 2900 may implement changes in the stimulation intensity via at least closed loop parameter 2910 and/or via auto-titrate parameter 2920 in FIG. 27A.

In some examples, care engine 2500 in FIG. 27A may comprise an initial use function 3100, which in some examples may automatically enhance determination of sleep-wake status. In some such examples, via initial use function 3100 a method and/or device for SDB care may omit a manual training period and instead automatically "normalize" use of the method and/or device for a particular patient. For instance, in some examples, determination of a sleep-wake status may begin with default parameters or may begin with parameters collected at the time of implant of a SDB care device in the patient. In some examples, determination of a sleep-wake status may be performed initially with no default parameters. In some such examples, when wakefulness is detected, sensing portion 2000 (FIG. 24) and/or care engine 2500 (FIG. 27A) may collect respiratory information, motion information, and/or posture information, etc. associated with wakefulness, which in turn may to allow for more sensitive detection of sleep in determining a sleep-wake status. In some examples, detection of wakefulness may comprise detecting gross body motion, such as but not limited to walking, swallowing, torso motion, etc. In some examples, a gravity vector is established at the time of implanting the SDB care device.

With this in mind, per the initial use function 3100, such automatic normalization may comprise omitting the use of absolute thresholds and instead perform determination of sleep-wake status (e.g. detection of onset of sleep) on the basis of percentage change in sensed values. Moreover, in some examples, sensing of various physiologic phenomenon (e.g. respiration, cardiac, etc.) may be used to determine a highest value or lowest value of such physiologic phenomenon and then use such end-of-the-range values to adjust thresholds accordingly.

It will be understood that the various parameters, functions, portions, etc. shown and described in association with FIG. 27A are not limited to the particular groupings, relationships, etc. shown in FIG. 27A, but may be arranged in groupings, relationships, etc. other than shown in FIG. 27A. Moreover, it will be understood that the care engine 2500 (or portions thereof) in FIG. 27A may be implemented with just some (i.e. not all) of the portions, elements, parameters, etc. shown in FIG. 27A.

With reference to at least care engine 2500 in FIG. 27A and the example methods and/or devices described throughout the present disclosure, it will be understood that such engines, methods, and/or devices (and components, portions, etc. thereof) for determining a sleep-wake status also may be used for quantifying activity levels and assessing related health parameters.

In some examples, as shown in FIG. 27B a cardiac morphology diagram 3500 schematically represents various cardiac morphological features, at least some of which may be employed to determine a sleep-wake status according to at least some of the example methods and/or example devices of the present disclosure, such as but not limited to those previously described in at least FIGS. 10-13, FIG. 24 (e.g. 2020, 2036), and 27A (e.g. 2520, 2600) For instance, diagram 3500 provides a graphic illustration of at least some of the various parameters and features (e.g. onset, peak, contraction, relaxation, valve closure, etc.) of at least the cardiac portion 2600 of care engine 2500 in FIG. 27A. As shown in FIG. 27B, cardiac morphology diagram 3500 comprises an aortic pressure signal 3502, an atrial pressure signal 3504, a ventricular pressure signal 3506, a ventricular volume signal 3510, an ECG signal 3520, and phonocardiogram (e.g. heart sounds) signal 3530. It will be understood the cardiac morphology diagram 3500 also may comprise a ballistocardiogram or seismocardiogram. These signals are plotted relative to time to demonstrate various morphological features, actions, etc. of a cardiac cycle with each beat of the heart. For instance, diagram 3500 depicts points in time along the atrial pressure and ventricular pressure signals 3504, 3506 at which the opening and closing of different valves may be identified, such as AV valves closure 3550, AV valves opening 3552, semilunar valves opening 3554, semilunar valves closure 3556. As further described in association with at least cardiac portion 2600 (FIG. 27A), these valve openings and closings may act as fiducials or parameters to track heart rate (and heart rate variability), and by which a determination of sleep-wake status may be performed. Diagram 3500 also depicts points in time along the atrial and ventricular pressure signals 3502, 3504 at which various portions of the cardiac cycle may be identified, such as ventricular contraction 3560 and ventricular relaxation 3562, and from which a determination of sleep-wake status may be performed upon tracking such contractions as a reliable identifier of a heart rate (and/or heart rate variability). Similarly, diagram 3500 depicts heart sounds S1, S2, S3, etc., whose features may correspond to the timing of the contraction and relaxation of the ventricles (3560, 3562 in FIG. 27A), valve opening and closure (3550, 3552, 2554, 3556 in FIG. 27A), etc. as shown in diagram 3500.

FIG. 28A is a block diagram schematically representing an example control portion 4000. In some examples, control portion 4000 provides one example implementation of a control portion forming a part of, implementing, and/or generally managing stimulation elements, power/control elements (e.g. pulse generators, microstimulators), sensors, and related elements, devices, user interfaces, instructions, information, engines, elements, functions, actions, and/or methods, as described throughout examples of the present disclosure in association with FIGS. 1-27B and 28B-36.

In some examples, control portion 4000 includes a controller 4002 and a memory 4010. In general terms, controller 4002 of control portion 4000 comprises at least one processor 4004 and associated memories. The controller 4002 is electrically couplable to, and in communication with, memory 4010 to generate control signals to direct operation of at least some of the stimulation elements, power/control elements (e.g. pulse generators, microstimulators) sensors, and related elements, devices, user interfaces, instructions, information, engines, elements, functions, actions, and/or methods, as described throughout examples of the present disclosure. In some examples, these generated control signals include, but are not limited to, employing instructions 4011 and/or information 4012 stored in memory 4010 to at least determining sleep-wake status of a patient, including particular sleep stages. Such sleep-wake determination may comprise part of directing and managing treatment of sleep disordered breathing such as obstructive sleep apnea, hypopnea, and/or central sleep apnea, with such sleep-wake determination also comprising sensing physiologic information including but not limited to electrical brain activity, respiratory information, heart rate, and/or monitoring sleep disordered breathing, etc. as described throughout the examples of the present disclosure in association with FIGS. 1-27E and 28B-36. In some instances, the controller 4002 or control portion 4000 may sometimes be referred to as being programmed to perform the above-identified actions, functions, etc. such that the controller 4002, control portion 4000 and any associated processors may sometimes be referred to as being a special purpose computer, control portion, controller, or processor. In some examples, at least some of the stored instructions 4011 are implemented as, or may be referred to as, a care engine, a sensing engine, monitoring engine, and/or treatment engine. In some examples, at least some of the stored instructions 4011 and/or information 4012 may form at least part of, and/or, may be referred to as a care engine, sensing engine, monitoring engine, and/or treatment engine.

In response to or based upon commands received via a user interface (e.g. user interface 4040 in FIG. 29) and/or via machine readable instructions, controller 4002 generates control signals as described above in accordance with at least some of the examples of the present disclosure. In some examples, controller 4002 is embodied in a general purpose computing device while in some examples, controller 4002 is incorporated into or associated with at least some of the stimulation elements, power/control elements (e.g. pulse generators, microstimulators), sensors, and related elements, devices, user interfaces, instructions, information, engines, functions, actions, and/or method, etc. as described throughout examples of the present disclosure.

For purposes of this application, in reference to the controller 4002, the term "processor" shall mean a presently developed or future developed processor (or processing resources) that executes machine readable instructions contained in a memory. In some examples, execution of the machine readable instructions, such as those provided via memory 4010 of control portion 4000 cause the processor to perform the above-identified actions, such as operating controller 4002 to implement the sensing, monitoring, determining, treatment, etc. as generally described in (or consistent with) at least some examples of the present disclosure. The machine readable instructions may be loaded in a random access memory (RAM) for execution by the processor from their stored location in a read only memory (ROM), a mass storage device, or some other persistent storage (e.g., non-transitory tangible medium or non-volatile tangible medium), as represented by memory 4010. In some examples, the machine readable instructions may comprise a sequence of instructions, a processor-executable machine learning model, or the like. In some examples, memory 4010 comprises a computer readable tangible medium providing non-volatile storage of the machine readable instructions executable by a process of controller 4002. In some examples, the computer readable tangible medium may sometimes be referred to as, and/or comprise at least a portion of, a computer program product. In other examples, hard wired circuitry may be used in place of or in combination with machine readable instructions to implement the functions described. For example, controller 4002 may be embodied as part of at least one application-specific integrated circuit (ASIC), at least one field-programmable gate array (FPGA), and/or the like. In at least some examples, the controller 4002 is not limited to any specific combination of hardware circuitry and machine readable instructions, nor limited to any particular source for the machine readable instructions executed by the controller 3002.

In some examples, control portion 4000 may be entirely implemented within or by a stand-alone device.

In some examples, the control portion 4000 may be partially implemented in one of the sensing devices, monitoring devices, stimulation devices, apnea treatment devices (or portions thereof), etc. and partially implemented in a computing resource separate from, and independent of, the apnea treatment devices (or portions thereof) but in communication with the apnea treatment devices (or portions thereof). For instance, in some examples control portion 4000 may be implemented via a server accessible via the cloud and/or other network pathways. In some examples, the control portion 4000 may be distributed or apportioned among multiple devices or resources such as among a server, an apnea treatment device (or portion thereof), and/or a user interface.

In some examples, control portion 4000 includes, and/or is in communication with, a user interface 4040 as shown in FIG. 29.

Figure 28B is a diagram schematically illustrating at least some example implementations of a control portion 4020 by which the control portion 4000 (FIG. 28A) can be implemented, according to one example of the present disclosure. In some examples, control portion 4020 is entirely implemented within or by an IPG assembly 4025, which has at least some of substantially the same features and attributes as a pulse generator (e.g. power/control element, microstimulator) as previously described throughout the present disclosure. In some examples, control portion 4020 is entirely implemented within or by a remote control 4030 (e.g. a programmer) external to the patient's body, such as a patient control 4032 and/or a physician control 4034. In some examples, the control portion 4000 is partially implemented in the IPG assembly 4025 and partially implemented in the remote control 4030 (at least one of patient control 4032 and physician control 4034).

FIG. 29 is a block diagram schematically representing user interface 4040, according to one example of the present disclosure. In some examples, user interface 4040 forms part or and/or is accessible via a device external to the patient and by which the therapy system may be at least partially controlled and/or monitored. The external device which hosts user interface 4040 may be a patient remote (e.g. 4032 in FIG. 28B), a physician remote (e.g. 4034 in FIG. 28B) and/or a clinician portal. In some examples, user interface 4040 comprises a user interface or other display that provides for the simultaneous display, activation, and/or operation of at least some of the stimulation elements, power/control elements (e.g. pulse generators, microstimulators), sensors, and related elements, devices, user interfaces, instructions, information, engines, functions, actions, and/or method, etc., as described in association with FIGS. 1-36. In some examples, at least some portions or aspects of the user interface 4040 are provided via a graphical user interface (GUI), and may comprise a display 4044 and input 4042.

FIG. 30 is a block diagram 4300 which schematically represents some example implementations by which an implantable device (IMD) 4310, such as an implantable pulse generator and/or implantable sensing monitor, may communicate wirelessly with external devices outside the patient. As shown in FIG. 30, in some examples, the IMD 4310 may communicate with at least one of patient app 4330 on a mobile device 4320, a patient remote control 4340, a clinician programmer 4350, and a patient management tool 4336. The patient management tool 4336 may be implemented via a cloud-based portal 4362, the patient app 4330, and/or the patient remote control 4340. Among other types of data, these communication arrangements enable the IMD 4310 to communicate, display, manage, etc. sleep/wake data for patient management as well as to allow for adjustment to the detection algorithm if/where needed.

It will be understood that at least some of the various devices/elements 4320, 4340, 4350, patient management tool 4360 also may communicate with each other, with or without communicating with the implantable device 4310.

FIG. 31A is diagram schematically representing one example user interface 5000, which may comprise just one example implementation of many example implementations user interface 4040 in FIG. 29, with user interface 5000 being displayable, in whole or part, on at least one of the mobile device 4320, remote control 4340, clinician programmer 4350, and/or patient management tool 4360 in FIG. 30 or other devices. However, it will be understood from the description associated with at least FIG. 29, a user interface 4040 may comprise or display many other parameters, functions, relationships, sensed information, etc. (from examples of the present disclosure) other than (or in addition to) the information shown in the user interface 5000 in FIG. 31A.

In some examples, a user interface (e.g. 4040 in FIG. 29) may display just a portion or some portions of user interface 5000 on a single display screen or a series of display screens. In some examples, a user interface (e.g. 4040) may display all of the portions of user interface 5000 shown in FIG. 31A.

In some examples, the user interface 5000 may comprise a nightly utilization portion 5010. In some examples, the nightly utilization portion 5010 comprises a wakefulness parameter 5050, elective start parameter 5060, elective stop parameter 5062, automatic start parameter 5070, automatic stop parameter 5072, on parameter 5075, pause (or non-use) parameter 5080, and/or a non-applicable parameter 5090. In some examples, each of the various parameters 5050-5090 are associated with a display element having a distinctive shape, color, pattern, and/or size, etc. to enable differentiating the different parameters. It will be understood that the example methods and/or devices relating to user interface 5000 are not limited to the particular shapes, color, sizes, etc. of display elements shown in FIG. 31A, which are provided for illustrative purposes. For instance, the wakefulness parameter 5050 is represented via a grid pattern in a columnar shape, which is shown in FIG. 31A as extending within a portion of a 24 hour daily period corresponding to period(s) in which the patient is in a wakeful state. Accordingly, as shown in FIG. 31A, the wakefulness parameter 5050 represents hours of the 24 hour period in which the patient is awake. Similarly, the other parameters 5060, 5062, 5070, 5072, 5075, 5080, 5090 are represented by their own distinctive shape, color, and/or pattern, etc.

The automatic start parameter 5070 (striped triangle) represents a point in time in which a treatment period is automatically initiated, which in turn in some examples may be driven by automatic determination of a sleep-wake status (e.g. detecting sleep). Following such an automatic start, the on parameter 5075 (solid color elongate rectangle) represents the number of hours (within the 24 hour period) for which the treatment period extends, and in which stimulation may be delivered. The pause parameter 5080 (white bar) represents a period of time in which a treatment period is paused or suspended, such as when a patient is temporarily awake to use the lavatory, get a snack, etc. In some examples, as shown in FIG. 31A, after such a pause (5080), the treatment period resumes in the on mode (5075). The treatment period may be resumed via automatic initiation upon detecting sleep by the patient or by the patient re-initiating the treatment period via remote control or other means (e.g. physical control).

In some examples, the pause parameter 5080 may be graphically represented via one type (e.g. color, pattern, shape, size) of indicator for automatic pause and another, different type (e.g. color, pattern, shape, size, etc.) of indicator for elective pause.

In some examples, an end of the treatment period may be represented via automatic stop parameter 5072 (short black bar).

In some examples, instead of an automatic initiation (e.g. start) of a treatment period, in some instances a patient may electively initiate a treatment period, such as represented by elective start parameter 5060 (speckled bar), as shown in FIG. 31A.

In some examples, instead of an automatic termination (e.g. stop), in some instances a patient may electively terminate a treatment period, such as represented by elective stop parameter 5062 (speckled isosceles triangle), as shown in FIG. 31A.

In some examples, as shown in FIG. 31A, the SDB care device may not be capable of entering a treatment period, either at the discretion of the patient, clinician, etc. In such situations, the non-applicable parameter 5090 may fill the entire 24 hour period.

In some examples, one or more of the parameters may be presented in an altered shape, pattern, etc. in the example nightly utilization portion 5010 of FIG. 31A in order to represent a change in a quantity, value, or other aspect of a sleep quality parameter. In one non-limiting example, such as when the SDB care device is active in a short treatment period A during the evening, but the patient then enters a wakeful state B (e.g. 19 hours to midnight (00 hours) before the patient sleeps without a treatment period being activated (white bar from midnight to 4 am) as shown at C. This lack of treatment may be due to manual control or other reasons. As shown on Wednesday, Nov 30, as represented by the narrow column (grid pattern) D representing a wakeful state, the patient exhibits one or more sleep quality parameters, which are indicative of poor sleep quality during the preceding nighttime period (Tuesday Nov 29) in which no treatment period was employed from midnight to 4 am, and the patient otherwise did not sleep much. In some such examples, at least some of the sleep quality parameters exhibited by the patient during their wakeful state (e.g. on Wed Nov 30) may be sensed via sensing portion 2000 (FIG. 24) and/or tracked via care engine 2500 (FIG. 27A), and may be displayed per a wakefulness-sourced sleep quality portion 5100 of user interface 5000.

As shown in FIG. 31A, in some examples the wakefulness-sourced sleep quality portion 5100 may comprise a date parameter 5102 (e.g. day of month), average parameter 5104 (e.g. average hours sleep), trend parameter 5106, heart rate (HR) parameter 5108, heart rate variability (HRV) parameter 5110, body movement parameter 5112, blood pressure parameter 5114, and/or other parameter 5116 (e.g. posture, position, etc.).

In some examples, the user interface 5000 may comprise a sleep-sourced sleep quality portion 5200, which may track and display substantially the same parameters (e.g. 5202-5216) as wakefulness-sourced sleep quality portion, except for the parameters being sensed during periods of sleep instead of during periods of wakefulness. In addition, the sleep-sourced sleep quality portion 5200 comprises an AHI parameter 5218 to track a hypopnea-apnea index (AHI).

In instances in which the patient is receiving therapeutic treatment on a consistent basis, at least some of the parameters of the wakefulness-sourced sleep quality parameters portion 5100 may be used to better characterize a wakeful state and at least some of the parameters of the wakefulness-sourced sleep quality parameters portion 5100 may be used to better characterize a sleep state. Accordingly, a comparison of the wakefulness-sourced sleep quality parameters (per portion 5100) with the sleep-sourced sleep quality parameters may be used to enhance determination of sleep-wake status (e.g. detecting sleep, detecting wakefulness) by learning how a particular patient may behave physiologically during a wakeful state and during a sleep state over a range of conditions, days, etc.

As further shown in FIG. 31A, user interface 5000 may comprise a user metrics portion 5300, which in at least some examples may display and/or otherwise communicate to a user information regarding automatic and/or elective treatment. For instance, in some examples the user metrics portion 5300 may comprise an automatic element 5310 to track and/or report instances in which the treatment is automatically started (5312), automatically stopped (5314), and automatically paused 5316. In some examples, these automatic implementations (e.g. start, stop, pause) are triggered or caused by an automatic determination of a sleep-wake status as described throughout the present disclosure. For instance, in some examples the user metrics portion 5300 may comprise an elective element 5320 to track and/or report instances in which the treatment is electively started (5322), electively stopped (5324), and electively paused 5326. In some examples, these elective implementations (e.g. start, stop, pause) are caused by a user operating a remote control, app on a mobile device, etc. Each of the respective automatic and elective functions (e.g. start, stop, pause) may be tracked and/or reported according to observable patterns (5330), averages (5340), and trends (5350).

By tracking and/or reporting the absolute and/or relative amount of automatic implementations and elective implementations via the use metrics portion 5300 and/or other displayable/reportable formats, a clinician or patient may determine the relative effectiveness (5342 in FIG. 31A) of automatic determination of a sleep-wake status according to examples of the present disclosure, which in turn may be used in determining the relative effectiveness of SDB care (e.g. electrical stimulation to treat obstructive sleep apnea, etc.).

It will be understood that the various parameters, functions, portions, etc. shown and described in association with FIG. 31A are not limited to the particular groupings, relationships, etc. shown in FIG. 31A, but may be arranged in groupings, relationships, etc. other than shown in FIG. 31A. Moreover, it will be understood that the user interface (or portions thereof) in FIG. 31A may be implemented with just some (i.e. not all) of the portions, elements, parameters, etc. shown in FIG. 31A.

As shown in FIG. 31B, in some examples the user interface 5000 of FIG. 31A also may display and/or report the use of ramped initiation, ramped transitions in and out of a pause in therapy, and/or a ramped termination of stimulation for a given treatment period. For example, as shown in the schematic representation in FIG. 31B, a daily display portion 5400 may comprise various graphic identifiers similar to those shown in FIG. 31A, such as a wakefulness period 5050, automatic start (e.g. auto-start) instances 5070, on period 5075, etc. It will be understood that display portion 5400 in FIG. 31B may comprise at least some of substantially the same features and attributes as one of the daily display portions 5030 shown in FIG. 31A and/or that at least some of substantially the same features and attributes of daily display portion 5400 (FIG. 31B) may be implemented in or as at least one of the daily display portions 5030 in FIG. 31A.

In some example methods, at least some of the start, stop, pauses of stimulation within a treatment period may be implemented in a ramped manner with the display portion 5400 in FIG. 31B schematically representing these implementations. For instance, an automatic start of stimulation may comprise a ramped increase (from zero) to a target stimulation intensity as represented via the triangular shaped ramp symbol shown at 5070 (also shown in FIG. 31A). This representation immediately indicates to a viewer the ramped manner in which the stimulation intensity was implemented. The ramped increase may occur at the beginning of a treatment period (e.g. 5405). Similarly, the triangular-shaped ramp symbol 5410 represents a ramped decrease of stimulation intensity from the target level (or another non-zero level) to zero, such as when stimulation is terminated (e.g. at 5406) or when stimulation is to be paused (e.g. at 5080). It will be understood that the representation of a ramped increase or decrease of stimulation intensity may be implemented via shapes other than a triangle.

The gradual ramped initiation or termination of stimulation therapy may enhance a patient's comfort by avoiding abrupt initiation, pause, or cessation of stimulation therapy. Among other features, the ramped implementation may increase the likelihood of patient compliance and appreciation for SDB care.

In some examples, at least some of the features and attributes associated with at least the methods and/or devices represented via FIGS. 31A-31B may be implemented via at least some features and attributes of the example methods described hereafter in association with FIGS. 31C-31H. In some examples, the methods described in association with FIGS. 31C-31H may be implemented via devices and elements other than those shown in at least FIGS. 31A-31B.

It will be understood that FIGS. 31A, 31B schematically represent at least some aspects of patient's experience of, operation of a device, and/or a method of treating a patient for sleep apnea. Accordingly, at least some aspects of at least FIGS. 31A-31B schematically represent via a method such as example method, as shown at 5500 in FIG. 31C, which comprises automatically taking an action when a probability of sleep, according to the sleep-wake status determination, exceeds a sleep-detection threshold or a probability of wakefulness, according to the sleep-wake status determination, exceeds a wake-detection threshold. In some examples, as shown at 5510 in FIG. 31D, automatically taking an action comprises at least one of automatically starting a stimulation treatment period and automatically stopping the stimulation treatment period. In some such examples, the term "non-sleep" may correspond to a probability of sleep remaining below a sleep detection threshold, while in some such examples, the term "non-wake" may correspond to a probability of wakefulness remaining below a wake detection threshold.

In some such examples (at 5510 in FIG. 31D), an example method may further comprise, as shown at 5520 in FIG. 31E, receiving input to electively start a treatment period and/or to electively stop a treatment period; and upon reception of input to electively start, suspending the automatically start and upon reception of input to electively stop, suspending the automatically terminating.

In some examples, as shown at 5530 in FIG. 31F, a method (associated with the actions/methods in FIGS. 31C-31E) may further comprise tracking information, for a plurality of nightly utilization periods, of at least one of a pattern, trend, and average of at least one of: automatic starts; automatic stops; elective starts; and elective stops. It will be understood that other (or additional) nightly utilization parameters described in association with FIGS. 31A-31B may be tracked per method 5530 in FIG. 31F.

In some examples, as shown at 5540 in FIG. 31G, a method comprises determining a quantitative effectiveness of the automatic sleep-wake determination via determining, from at least the tracked information, a first proportion of automatically starting relative to elective starts and/or a second proportion of automatically stopping relative to elective stops.

In some examples, as shown at 5550 in FIG. 31H, a method comprises displaying, via a graphical user interface, a plurality of separate nightly utilization periods, each which symbolically illustrate with respect to a treatment period within each nightly utilization period at least one of: an automatic start; an automatic stop; an elective start; and an elective stop. As apparent from FIGS. 31A-31B, at least some features of method at 5550 in FIG. 31H may be implemented via the graphical user interface illustrated and described in association with FIGS. 31A-31B.

FIG. 32 is a diagram schematically representing a timeline 6010 of sleep-wake-related events according to an example method 6000 of sleep-wake determination, such as may occur during sleep disordered breathing (SDB) care (e.g. monitoring, diagnosis, treatment, etc.). In some examples, the example SDB care may comprise at least some of substantially the same features and attributes as the example SDB care methods and/or devices (including sleep-wake detection) as described in association with FIGS. 1-31H and 33-36.

As shown in FIG. 32, the timeline 6010 includes a series of wake and sleep periods with wake period 6020 occurring just before a first sleep stage period 6040 (e.g. stage 1). The wake period 6020 in FIG. 32 may represent an end portion of a wake period extending since the end of a prior night's sleep or may represent another wake period.

As further represented by indicator 6035, a real physiologic transition occurs between the wake period 6020 and the first sleep stage 6040 and indicator 6043 represents a detection of sleep according to examples of the present disclosure. As shown in FIG. 32, the detection of sleep (6043) may occur just after the physiologic transition 6035.

In some examples, the detection of sleep 6043 may trigger a delay period 6045 prior to a start of therapy (e.g. electrical stimulation). In some such examples, the duration of the delay generally corresponds to an amount of time sufficient for a patient to experience sufficiently sound sleep such that the patient will not be awakened by the onset of stimulation. Moreover, in some examples, once stimulation begins it may be implemented in a ramped manner (6046) with an initial lower stimulation intensity which is gradually increased until a target stimulation intensity (6047) is achieved to therapeutically provide electrical stimulation to an upper airway patency related tissue.

As previously noted in the present disclosure, at least some example implementations of method 6000 in FIG. 32 may comprise identifying, maintaining, and/or optimizing a target stimulation intensity (e.g. therapy level) without intentionally identifying a stimulation discomfort threshold at the time of implantation or at a later point in time after implantation.

As further shown in FIG. 32, once the target stimulation intensity is achieved, it may be maintained throughout the treatment period.

In some examples, the target stimulation intensity may be automatically adjusted (e.g. auto-titrated) during the treatment period. In some such examples, the automatic adjustment of the target stimulation intensity may be implemented according to at least some of substantially the same features and/or attributes as described in association with at least auto-titration parameter 2920 in FIG. 27A.

As further shown in FIG. 32, after some period of time (which may vary from night to night) the patient may sometimes experience a wake period 6060 during a treatment period, which interrupts a sleep stage (e.g. a second sleep stage (S2) 6050 in this example). The example method 6000 detects wakefulness (6062), which may extend for a period of time (W1), before the patient goes back to sleep, such as represented by sleep stage 6070 and transition 6065 between the respective wake period 6060 and sleep stage 6070.

In some examples, method 600 may completely pause stimulation during the wakeful period 6060 or instead in some examples, method 6000 may implement a reduced therapy 6064 during the wakeful period 6060 because of the expectation of the patient going back to sleep and a full stimulation therapy being resumed. In some such examples, the reduced therapy at 6064 may comprise providing stimulation at a functional threshold (FT), which corresponds to a minimum amplitude at which the stimulation will cause the tongue to protrude at least part way past the lower teeth and at which a therapeutic outcome (e.g. reduction in apneas) may be achieved. However, in some such examples, the reduced therapy at 6064 may comprise providing stimulation at a sensation threshold (ST), which involves a stimulation intensity less than the stimulation intensity to reach the functional threshold (FT). The sensation threshold (ST) may correspond to a minimum amplitude at which the patient can sense stimulation.

As indicated at 6072 in FIG. 32, therapy may be resumed automatically. It will be understood that, in at least some examples, the resumption of therapy 6072 may comprise substantially the same features and attributes as the initiation of therapy as previously described in relation to indicators 6043, 6046, 6047 in FIG. 32, including detection of sleep 6043 according to at least some of the examples of the present disclosure to determine a sleep-wake status.

In some examples, in general terms the beginning of the first sleep stage 6040 generally corresponds to a beginning of a treatment period during which a patient may be treated for sleep disordered breathing and/or the method (and/or device) may monitor for or diagnose sleep disordered breathing.

FIG. 33 is a block diagram schematically representing an example arrangement 7000 comprising an example method (and/or example device) for determining a sleep-wake status. In some examples, method 7000 may be implemented in a complementary manner with, and/or comprise, at least some of substantially the same features as previously described in association with at least probability function 3200 of care engine 2500 in FIG. 27A (including machine learning parameter 3230), processing portion 2600 in FIG. 26, and/or method 800 in FIG. 21. In some examples, at least some aspects of the example arrangement 7000 may be implemented more generally via the control portion 4000 (FIG. 28A), such as but not limited to being one example implementation of at least some of stored executable instructions 4011 and/or 4012 (FIG. 28A).

With further reference to FIG. 33, one example single sensor 2010 (or type of single sensor) may comprise an accelerometer, which may produce a signal of sensed physiologic information from which multiple physiologic phenomenon may be extracted, such as but not limited to cardiac, respiratory, motion, activity, etc.

As shown in FIG. 33, method 7000 comprises sensing physiologic phenomenon (at 7005) via sensor 7010 to produce a signal 7012, which is processed (7020) to divide signal 7012 into different/individual components such as at 7062A-7062N.

In some examples, each individual component 7062A-7062N may sometimes be referred to as a sleep-wake determination parameter, such as (but not limited to) previously described in association with FIG. 21. It will be understood that throughout the entirety of FIG. 33, the identifier "N" represents the "nth" signal component, such as an unspecified total number of signal components, and not a particular limit on the number of components into which signal 7012 may be divided. As previously noted, at least some example signal components may comprise cardiac, respiratory, motion, activity, posture, etc.

As further shown in FIG. 33, in some examples the activity and/or elements represented via indicators (e.g. 7062A-N, 7072A-N, 7082A-N) may sometimes be referred to as a processing pathway or processing portion 7050. The divided signals 7062A-7062N may be referred to as a group 7060 of signal components of signal 7012, the individual feature enhancements 7072A-7072N may be referred to collectively as a group 7070 of feature enhancements, and/or the individual sleep-wake determinations 7072A-7072N may be referred to as a group 7070 of sleep-wake determinations.

As further shown in FIG. 33, each respective signal component 7062A-7062N (e.g. each sleep-wake determination parameter) is further processed to produce a respective feature enhancement 7072A-7072N (group 7070), which in turn may be used and/or further processed to yield a respective sleep-wake determination result 7082A-7082N (group 7080). In some examples, any one of the various sleep-wake determination results 7082A-7082N may be sufficient to effectively determine a sleep-wake status. However, in some examples at least two sleep-wake determination results (e.g. 7802A, 7802C) may be considered together as part of a combined sleep-wake determination at 7300.

As further shown at 7350, in some examples a patient and/or clinician may provide information (e.g. input) regarding a patient's experience related to sleep and wakefulness, and transitions therebetween, which may be used to enhance or support the combined sleep-wake determination. In some examples, this input may be implemented or expressed via applying different weights (e.g. 3240 in FIG. 27A) to the above-described various sleep-wake determination parameters (e.g. 7062A-7062N in FIG. 33, FIG. 20A).

In some examples, instead of or in addition to the processing pathway 7050 shown in FIG. 33, method 7000 may comprise further processing via pattern matching at 7200. In some such examples, the pattern matching comprises accessing stored signal patterns of a sleep behavior (e.g. sleep behavior pattern), stored signal patterns of wakefulness behavior (e.g. wakefulness pattern), and/or stored signal sleep-related behavior. In some examples, the stored signal patterns, etc. may be stored in memory 4010 (FIG. 28A) and/or other accessible database, which may be external to the patient.

As further shown in FIG. 33, the output of stored patterns from the pattern matching (7200) may be submitted to and used by a deep learning element 7250 in analyzing sensed physiologic phenomenon (e.g. respiratory signals, cardiac signals, etc.) to determine a pattern(s) indicative of a sleep state (e.g. onset, offset, various sleep stages) and/or pattern(s) indicative of wakefulness (e.g. onset, offset various sleep stages). In some examples, at least part of this analysis may comprise comparing stored signal patterns with current or recent signal patterns.

In some examples, the deep learning element 7250 may comprise a convolutional neural network, deep neural network, deep neural learning, and the like. It will be understood that in some examples the deep learning element 7250 may be implemented via other forms of artificial intelligence tools. The deep learning element 7250 may be implemented as part of, or in a complementary manner with, machine learning parameter 3230 in FIG. 27A.

The output of the deep learning element 7250 is provided to, or as, the comprehensive sleep-wake determination at 7300. It will be understood that in some examples, the output of the deep learning element 7250 may be the sole basis on which a comprehensive sleep-wake determination 7300 is implemented. However, in some examples, the output of the deep learning element 7250 may comprise just one input in a comprehensive sleep-wake determination 7300. In some such examples, other inputs may comprise one of the sleep-wake determination results 7082A-7082N and/or may comprise the patient/clinician input 7350.

In some examples, the deep learning element 7250 as represented in FIG. 33 may comprise a trained machine learning model (e.g. trained deep learning model), which may be trained (i.e. constructed) prior to operation of the example arrangement 7000. As further shown in the example arrangement (e.g. example method or device) in FIG. 34, in some examples the training may be performed at least partially via an external resource 7410 external to patient's body and external to an implantable medical device 7420. The implantable medical device 7420 may comprise an implantable sensor (e.g. 7010 in FIG. 33) and control portion 4000 (FIG. 28A), among other components, features, etc. After such training, the trained deep learning model may be imported into the implantable medical device 7420 for use in determining a sleep-wake state, such as by acting as a deep learning element 7250 in the example arrangement 7000 in FIG. 33.

In some examples, the external resource(s) 7410 (FIG. 34) may comprise a computing resource 7414 sized and scaled to perform deep learning. In some examples, the external resource(s) 7410 may comprise a data store 7412, such as but not limited to a large data set of stored sleep information for many patients, which may comprise acceleration signal component information, etc. relating to different non-physiologic parameters and physiologic parameters, such as but not limited to cardiac information, respiratory information, motion/activity information, posture information, etc. In some examples, the stored sleep-related data may be specific to the patient in which the trained deep learning model may be imported, such as being imported into or as element 7250 of an example arrangement 7000 within an implantable medical device.

With this in mind, in some examples the deep learning element 7250 may be trained (i.e. constructed) via the external resource 7410 according to the example arrangement (e.g. method and/or device) 7500 in FIG. 34. As shown in FIG. 34, known inputs 7510 sensed via at least an implanted accelerometer (e.g. 7010 in FIG. 33) and a known output 7540 are both provided to a trainable machine learning model 7530. In some examples, the known output 7540 may comprise an externally determined sleep-wake state, which may comprise any number of externally measurable physiologic parameters for determining a sleep-wake state, such as but not limited to any one of (or combinations of) EEG, EOG, EMG, ECG, cardiac information, respiratory information, motion/activity, posture, etc.

As further shown in FIG. 34, in some examples at least some known inputs (obtained via the implanted accelerometer or other implantable sensor) may comprise cardiac information 7512, respiratory information 7514, motion/activity information 7516, posture information 7518, and/or other information. It will be understood that these inputs are mere examples, and that the known inputs (from the implanted accelerometer signal or other implantable sensor) may comprise any sensed physiologic information pertinent to determining a sleep-wake state.

By providing such known inputs (7510) and known outputs (7540) to the trainable machine learning model 7530, a trained machine learning model 7631 (FIG. 35) may be obtained. In some examples, just one or some of the known inputs 7510 may be used, while all of the known inputs 7510 may be used in some examples. As noted elsewhere, the trainable/trained machine learning model (7530, 7631) may comprise a deep learning model.

FIG. 35 is a diagram schematically representing an example method 7600 (and/or example device) for using a trained machine learning model 7631 for determining sleep-wake state using internal measurements, such as via an implanted accelerometer in some examples. As shown in FIG. 35, currently sensed inputs 7611 are fed into the trained machine learning model 7631, which then produces a determinable output 7641, such as a current sleep-wake state determination 7643, which is based on the current inputs 7611. In some examples, the current inputs 7611 correspond to the same type and/or number of known inputs 7510 (FIG. 34) used to train the machine learning model. In some examples, just one or some of the current inputs 7611 may be used, while all of the current inputs 7611 may be used in some examples.

As previously noted, once the trained machine learning model 7631 is obtained, it is imported into and/or otherwise forms part of control portion 4000 (and/or care engine 2500 in FIG. 27A) such that the trained machine learning model 7631 may act as the deep learning element 7250 in the example arrangement 7000 of FIG. 33.

With further reference to FIG. 33, in some examples, method/device 7000 may comprise an other input element 7260 by which other information which is sensed via an implantable sensor (e.g. within or connected to implantable pulse generator) as internal input 7264 or otherwise received from an external sensor as external input 7262 may be used as part of a comprehensive sleep-wake determination 7300. In some examples, a first output 7266 of the other input element 7260 may be combined with output 7090 from the processing pathway 7050 as part of the comprehensive sleep-wake determination 3000. In some examples, a second output 7267 of the other input element 7260 may be fed into deep learning element 7250 prior to, or as part of, performing the comprehensive sleep-wake determination 3000.

In some examples, the external input 7262 may comprise input such as from external sensors associated with a remote control 4340, an app 4330 on mobile consumer device 4320, etc. (as shown in FIG. 30 and FIG. 28B) and/or associated with remote, app, physical parameters 3012, 3013, 3018 in FIG. 27A. The external sensors/input may comprise ambient light, movement/operation of the remote control or of the app/mobile consumer device, etc. Other input may comprise time of day, time zone, geographic latitude, etc. as previously described in association with at least FIGS. 20E-20F, temporal parameter 3014 (FIG. 27A), boundary parameter 3016 (FIG. 27A), and the like regarding input used to at least partially determine sleep-wake status according to detecting a probability of sleep and/or a probability of wakefulness.

In some examples, the internal sensors/input 7264 in FIG. 33 may comprise any other signals or input (e.g. stored information, command, etc.) available via the implantable device (e.g. IPG) which may complement the information being processed (e.g. pathway 7005/7012/7020/7200/7250 OR in pathway 7005/7012/7020/7050).

Although specific examples have been illustrated and described herein, a variety of alternate and/or equivalent implementations may be substituted for the specific examples shown and described without departing from the scope of the present disclosure. This application is intended to cover any adaptations or variations of the specific examples discussed herein. The scope of the invention is defined in claim 1.

## Claims

1. A sleep disordered breathing (SDB) care device comprising:
an implantable first sensor (7010) comprising an accelerometer (2026, 2526) to sense physiologic information to determine a sleep-wake status via the sensed physiologic information;
wherein the SDB care device is to:
automatically take an action when at least one of:
a probability of sleep, according to the sleep-wake status determination, exceeds a sleep-detection threshold; or
a probability of wakefulness, according to the sleep-wake status determination, exceeds a wake-detection threshold,
wherein the automatically taking an action comprises at least one of automatically starting a stimulation treatment period and automatically stopping the stimulation treatment period,
wherein the device is to:
receive input to electively start a treatment period and/or to electively stop a treatment period; and
upon reception of input to electively start, suspend the automatically start and upon reception of input to electively stop, suspending the automatically terminating; and
wherein the SBD care device is **characterized in that** it comprises a graphical user interface (4040) to display a plurality of separate nightly utilization periods, each which symbolically illustrate with respect to a treatment period within each nightly utilization period at least one of:
an automatic start;
an automatic stop;
an elective start; and
an elective stop.

2. The SDB care device of claim 1, wherein the determination of the sleep-wake status comprises detecting an onset of sleep.

3. The SDB care device according to one of the claims 1 or 2, wherein the implantable first sensor (7010) is configured to sense the physiologic information as motion at at least one of a chest and a neck, and
wherein the sensed motion comprises at least one of:
sensed respiration information;
sensed cardiac information including heart rate; and
sensed gross body motion.

4. The SDB care device of claim 3, wherein the sensed respiration information is based on sensing motion of a chest wall, particularly
wherein the implantable first sensor (7010) is configured to differentiate, based on an amplitude of the sensed chest wall motion, between active respiration indicative of a wake state and passive respiration indicative of a sleep state.

5. The SDB care device according to one of the claims 3 or 4, comprising:
a second sensor to perform electrocardiogram sensing, in addition to the sensing of motion via the first sensor (7010), to further determine the cardiac information.

6. The SDB care device according to one of the claims 3 to 5, wherein sensing the motion comprises:
performing the sensing via the implantable first sensor (7010) implanted in the neck region (2103, 2302).

7. The SDB care device of claim 6, comprising:
an implantable microstimulator (2133, 2355), including the implantable first sensor (7010), for implantation within the head-and-neck region (2103, 2302).

8. The SDB care device of claim 7, wherein the microstimulator (2133, 2355) including an exterior housing on which is mounted at least one first electrode (2135, 2404), and the SDB care device comprising an implantable second electrode (2120, 2130, 2431, 2432) spaced apart from the first electrode (2135, 2404), wherein the respective first (2135) and second electrodes (2120, 2130) are configured to obtain the cardiac information via electrocardiogram sensing, in addition to the sensing of motion via the first sensor (7010).

9. The SDB care device of claim 8, comprising:
a lead (2117) extending from a housing of the microstimulator (2133) and including the second electrode, with the lead (2117) having a length to maintain the second electrode (2120) to be spaced apart from the first electrode (2135).

10. The SDB care device of one of the claims 3 to 9, comprising:
an implantable pulse generator (2133) including an exterior housing on which is located at least one first electrode (2135), and the SDB care device comprising an implantable second electrode (2120, 2130) spaced apart from the first electrode (2135), wherein the respective first and second electrodes (2120, 2130, 2135) are configured to obtain the cardiac information via electrocardiogram sensing, in addition to the sensing of motion via the first sensor (7010).

11. The SDB care device of one of the claims 1 to 10, wherein the implantable first sensor (7010) to sense the physiologic information as sensed body motion to determine the sleep-wake status via detecting sleep upon a time of day and a lack of sensed body motion for a predetermined period of time, particularly wherein the SBD care device comprises
a stimulation element to implement, upon detection of sleep, stimulation, wherein the stimulation is maintained until at least one of:
the first sensor (7010) senses physiologic information indicative of wakefulness; and
manual termination of stimulation.

12. The SDB care device of claim 11, wherein the stimulation element is to implement stimulation via increasing stimulation from a lower intensity level to a target intensity level.

13. The SDB care device of one of the claims 11 or 12, wherein the implantable first sensor (7010) is to sense physiologic information to include as at least one of sensed respiratory information and sensed cardiac information, wherein the SDB care device is to determine the sleep-wake status via at least one of the sensed respiratory information and sensed cardiac information, and
optionally wherein the implantable first sensor (7010) is to sense the physiologic information to include posture information, and wherein the SDB care device is to determine the sleep-wake status via at least one of the sensed posture information, the sensed respiratory information, and the sensed cardiac information.

14. The SDB care device of one of the claims 1 to 13, wherein the implantable first sensor (7010) is to sense the physiologic information via sensing at least variability in at least one of a respiratory information including a respiratory rate, cardiac information including a heart rate, posture, and body movement; and
wherein the SDB care device is to determine the sleep-wake status via determining the variability in at least one of the respective sensed respiratory rate, sensed cardiac information including the heart rate, sensed posture, and sensed body movement.

15. The SDB care device of claim 14, wherein the SDB care device is to process the sensed physiologic information to distinguish between:
a first variability in the respiratory information and the cardiac information which is characteristic of sleep disordered breathing; and
a second variability in the respiratory information and the cardiac information which is other than the first variability characteristic of sleep disordered breathing.

## Patentansprüche

1. Vorrichtung zum Behandeln von Schlafatmungsstörungen (sleep disordered breathing, SDB), umfassend:
einen implantierbaren ersten Sensor (7010), umfassend einen Beschleunigungsmesser (2026, 2526) zum Erfassen physiologischer Informationen zum Bestimmen eines Schlaf-Wach-Status anhand der erfassten physiologischen Informationen;
wobei die SDB-Behandlungsvorrichtung dient zum:
automatischen Ausführen einer Maßnahme bei mindestens einem der Folgenden:
eine Schlafwahrscheinlichkeit überschreitet gemäß der Bestimmung des Schlaf-Wach-Status einen Schlaferkennungsschwellenwert oder
eine Wachzustandswahrscheinlichkeit überschreitet gemäß der Bestimmung des Schlaf-Wach-Status einen Wachzustandserkennungsschwellenwert,
wobei das automatische Ausführen einer Maßnahme mindestens eines umfasst von einem automatischen Starten einer Stimulationsbehandlungsperiode und einem automatischen Stoppen einer Stimulationsbehandlungsperiode,
wobei die Vorrichtung dient zum:
Empfangen einer Eingabe zum wahlweisen Starten einer Behandlungsperiode und/oder wahlweisen Stoppen einer Behandlungsperiode; und
bei Empfang einer Eingabe zum wahlweisen Starten Aussetzen des automatischen Starts und bei Empfang einer Eingabe zum wahlweisen Stoppen Aussetzen des automatischen Stoppens; und
wobei die SDB-Behandlungsvorrichtung **dadurch gekennzeichnet ist, dass** sie umfasst
eine grafische Benutzeroberfläche (4040) zum Anzeigen einer Vielzahl von separaten nächtlichen Nutzungsperioden, die jeweils symbolisch in Bezug auf eine Behandlungsperiode innerhalb jeder nächtlichen Nutzungsperiode mindestens eines der Folgenden veranschaulichen:
einen automatischen Start;
einen automatischen Stopp;
einen wahlweisen Start und
einen wahlweisen Stopp.

2. SDB-Behandlungsvorrichtung nach Anspruch 1, wobei das Bestimmen des Schlaf-Wach-Status das Erkennen eines Schlafbeginns umfasst.

3. SDB-Behandlungsvorrichtung nach einem der Ansprüche 1 oder 2, wobei der implantierbare erste Sensor (7010) konfiguriert ist, um die physiologischen Informationen als Bewegung an mindestens einem von Brust und Hals zu erfassen, und
wobei die erfasste Bewegung mindestens eines der Folgenden umfasst:
erfasste Atmungsinformationen;
erfasste kardiale Informationen einschließlich Herzfrequenz und
erfasste grobe Körperbewegung.

4. SDB-Behandlungsvorrichtung nach Anspruch 3, wobei die erfassten Atmungsinformationen auf dem Erfassen der Bewegung einer Brustwand basieren, insbesondere
wobei der implantierbare erste Sensor (7010) konfiguriert ist, um, basierend auf einer Amplitude der erfassten Brustwandbewegung, zwischen aktiver Atmung, die einen Wachzustand anzeigt, und passiver Atmung, die einen Schlafzustand anzeigt, zu unterscheiden.

5. SDB-Behandlungsvorrichtung nach einem der Ansprüche 3 oder 4, umfassend:
einen zweiten Sensor zum Durchführen einer Elektrokardiogrammerfassung zusätzlich zum Erfassen von Bewegung über den ersten Sensor (7010), um die kardialen Informationen weiter zu bestimmen.

6. SDB-Behandlungsvorrichtung nach einem der Ansprüche 3 bis 5, wobei das Erfassen der Bewegung umfasst:
Durchführen der Erfassung über den implantierbaren ersten Sensor (7010), der im Halsbereich (2103, 2302) implantiert ist.

7. SDB-Behandlungsvorrichtung nach Anspruch 6, umfassend:
einen implantierbaren Mikrostimulator (2133, 2355), einschließlich des implantierbaren ersten Sensors (7010), zur Implantation in den Kopf-HalsBereich (2103, 2302).

8. SDB-Behandlungsvorrichtung nach Anspruch 7, wobei der Mikrostimulator (2133, 2355) ein Außengehäuse umfasst, an dem mindestens eine erste Elektrode (2135, 2404) angebracht ist, und die SDB-Behandlungsvorrichtung eine implantierbare zweite
Elektrode (2120, 2130, 2431, 2432) umfasst, die von der ersten Elektrode (2135, 2404) beabstandet ist, wobei die jeweilige erste (2135) und zweite Elektrode (2120, 2130) konfiguriert sind, um die kardialen Informationen über die Elektrokardiogrammerfassung zusätzlich zum Erfassen von Bewegung über den ersten Sensor (7010) zu erhalten.

9. SDB-Behandlungsvorrichtung nach Anspruch 8, umfassend:
eine Leitung (2117), die sich aus einem Gehäuse des Mikrostimulators (2133) erstreckt und die zweite Elektrode umfasst, wobei die Leitung (2117) eine solche Länge aufweist, dass die zweite Elektrode (2120) von der ersten Elektrode (2135) beabstandet bleibt.

10. SDB-Behandlungsvorrichtung nach einem der Ansprüche 3 bis 9, umfassend:
einen implantierbaren Impulsgenerator (2133), der eine Außengehäuse aufweist, auf dem sich mindestens eine erste Elektrode (2135) befindet, und wobei die SDB-Behandlungsvorrichtung eine implantierbare zweite Elektrode (2120, 2130) umfasst, die von der ersten Elektrode (2135) beabstandet ist, wobei die jeweilige erste und zweite Elektrode (2120, 2130, 2135) konfiguriert sind, um die kardialen Informationen über eine Elektrokardiogrammerfassung zusätzlich zum Erfassen von Bewegung über den ersten Sensor (7010) zu erhalten.

11. SDB-Behandlungsvorrichtung nach einem der Ansprüche 1 bis 10, wobei der implantierbare erste Sensor (7010) die physiologischen Informationen als erfasste Körperbewegung zum Bestimmen des Schlaf-Wach-Status durch das Erkennen von Schlaf zu einer Tageszeit und ein Fehlen erfasster Körperbewegung für einen vorbestimmten Zeitraum erfassen soll, insbesondere wobei die SDB-Behandlungsvorrichtung umfasst
ein Stimulationselement zum Durchführen einer Stimulation bei Erkennen von Schlaf, wobei die Stimulation aufrechterhalten wird, bis zu mindestens einem der Folgenden:
der erste Sensor (7010) erfasst physiologische Informationen, die einen Wachzustand anzeigen; und
manuelles Beenden der Stimulation.

12. SDB-Behandlungsvorrichtung nach Anspruch 11, wobei das Stimulationselement die Stimulation durch Erhöhen der Stimulation von einem niedrigeren Intensitätsniveau auf ein Zielintensitätsniveau durchführen soll.

13. SDB-Behandlungsvorrichtung nach einem der Ansprüche 11 oder 12, wobei der implantierbare erste Sensor (7010) physiologische Informationen erfassen soll, sodass sie mindestens eines von den erfassten Atmungsinformationen und den erfassten kardialen Informationen umfassen, wobei die SDB-Behandlungsvorrichtung den Schlaf-Wach-Status über mindestens eines von den erfassten Atmungsinformationen und den erfassten kardialen Informationen bestimmen soll, und
wobei optional der implantierbare erste Sensor (7010) die physiologischen Informationen erfassen soll, sodass sie Körperhaltungsinformationen umfassen, und wobei die SDB-Behandlungsvorrichtung den Schlaf-Wach-Status über mindestens eines von den erfassten Körperhaltungsinformationen, den erfassten Atmungsinformationen und den erfassten kardialen Informationen bestimmen soll.

14. SDB-Behandlungsvorrichtung nach einem der Ansprüche 1 bis 13, wobei der implantierbare erste Sensor (7010) die physiologischen Informationen durch Erfassen mindestens einer Variabilität in mindestens einem von einer Atmungsinformation einschließlich einer Atemfrequenz, einer kardialen Information einschließlich einer Herzfrequenz, einer Körperhaltung und Körperbewegung erfassen soll; und
wobei die SDB-Behandlungsvorrichtung den Schlaf-Wach-Status durch Ermitteln der Variabilität in mindestens einem von der jeweils erfassten Atemfrequenz, der erfassten kardialen Informationen einschließlich der Herzfrequenz, der erfassten Körperhaltung und der erfassten Körperbewegung bestimmen soll.

15. SDB-Behandlungsvorrichtung nach Anspruch 14, wobei die SDB-Behandlungsvorrichtung die erfassten physiologischen Informationen verarbeiten soll zum Unterscheiden zwischen:
einer ersten Variabilität in den Atmungsinformationen und den kardialen Informationen, die für Schlafatmungsstörungen charakteristisch ist; und
einer zweite Variabilität in den Atmungsinformationen und den kardialen Informationen, die sich von der ersten, für Schlafatmungsstörungen charakteristischen Variabilität unterscheidet.

## Revendications

1. Dispositif de soins pour les troubles respiratoires du sommeil (TRS) comprenant :
un premier capteur implantable (7010) comprenant un accéléromètre (2026, 2526) pour détecter des informations physiologiques afin de déterminer un état de sommeil-veille via les informations physiologiques détectées ;
le dispositif de soins TRS étant destiné à :
effectuer automatiquement une action lorsqu'au moins l'un parmi :
une probabilité de sommeil, en fonction de la détermination de l'état sommeil-veille, dépasse un seuil de détection de sommeil ; ou
une probabilité d'éveil, en fonction de la détermination de l'état sommeil-veille, dépasse un seuil de détection d'éveil,
dans lequel l'exécution automatique d'une action comprend au moins l'un parmi le démarrage automatique d'une période de traitement de stimulation et l'arrêt automatique de la période de traitement de stimulation,
le dispositif étant destiné à :
recevoir des informations permettant de démarrer ou d'arrêter une période de traitement de manière élective ; et
à la réception d'une entrée pour démarrer de manière élective, suspendre le démarrage automatique et à la réception d'une entrée pour arrêter de manière élective, suspendre l'arrêt automatique ; et
le dispositif de soins TRS étant **caractérisé en ce qu'**il comprend
une interface utilisateur graphique (4040) pour afficher une pluralité de périodes d'utilisation nocturnes distinctes, chacune illustrant symboliquement, pour une période de traitement dans chaque période d'utilisation nocturne, au moins l'un parmi :
un démarrage automatique ;
un arrêt automatique ;
un démarrage électif ; et
un arrêt électif.

2. Dispositif de soins TRS selon la revendication 1, dans lequel la détermination de l'état de sommeil-veille comprend la détection d'un début de sommeil.

3. Dispositif de soins TRS selon l'une des revendications 1 ou 2, dans lequel le premier capteur implantable (7010) est configuré pour détecter les informations physiologiques sous forme de mouvement au niveau d'au moins l'un de la poitrine et du cou, et
dans lequel le mouvement détecté comprend au moins l'un parmi :
des informations respiratoires détectées ;
des informations cardiaques détectées, notamment la fréquence cardiaque ; et
un mouvement corporel général détecté.

4. Dispositif de soins TRS selon la revendication 3, dans lequel les informations respiratoires détectées sont basées sur la détection du mouvement d'une paroi thoracique, en particulier
dans lequel le premier capteur implantable (7010) est configuré pour différencier, en fonction d'une amplitude du mouvement de paroi thoracique détecté, entre une respiration active indiquant un état de veille et une respiration passive indiquant un état de sommeil.

5. Dispositif de soins TRS selon l'une des revendications 3 ou 4, comprenant :
un deuxième capteur pour effectuer une détection d'électrocardiogramme, en plus de la détection de mouvement via le premier capteur (7010), pour déterminer davantage les informations cardiaques.

6. Dispositif de soins TRS selon l'une des revendications 3 à 5, dans lequel la détection du mouvement comprend :
la réalisation de la détection via le premier capteur implantable (7010) implanté dans la région du cou (2103, 2302).

7. Dispositif de soins TRS selon la revendication 6, comprenant :
un microstimulateur implantable (2133, 2355), comprenant le premier capteur implantable (7010), destiné à être implanté dans la région de la tête et du cou (2103, 2302).

8. Dispositif de soins TRS selon la revendication 7, dans lequel le microstimulateur (2133, 2355) comprend un boîtier extérieur sur lequel est montée au moins une première électrode (2135, 2404), et le dispositif de soins TRS comprend une deuxième électrode implantable (2120, 2130, 2431, 2432) espacée de la première électrode (2135, 2404), dans lequel les première (2135) et deuxième électrodes (2120, 2130) respectives sont configurées pour obtenir les informations cardiaques via une détection d'électrocardiogramme, en plus de la détection de mouvement via le premier capteur (7010).

9. Dispositif de soins TRS selon la revendication 8, comprenant :
une dérivation (2117) s'étendant depuis un boîtier du microstimulateur (2133) et comprenant la deuxième électrode, la dérivation (2117) ayant une longueur pour maintenir la deuxième électrode (2120) espacée de la première électrode (2135).

10. Dispositif de soins TRS selon l'une des revendications 3 à 9, comprenant :
un générateur d'impulsions implantable (2133) comprenant un boîtier extérieur sur lequel est située au moins une première électrode (2135), et le dispositif de soins TRS comprenant une deuxième électrode implantable (2120, 2130) espacée de la première électrode (2135), les première et deuxième électrodes respectives (2120, 2130, 2135) étant configurées pour obtenir les informations cardiaques via une détection d'électrocardiogramme, en plus de la détection de mouvement via le premier capteur (7010).

11. Dispositif de soins TRS selon l'une des revendications 1 à 10, dans lequel le premier capteur implantable (7010) détecte les informations physiologiques telles que les mouvements corporels détectés pour déterminer l'état sommeil-veille via la détection du sommeil à un moment de la journée et une absence de mouvement corporel détecté pendant une période de temps prédéterminée, en particulier dans lequel le dispositif de soins TRS comprend
un élément de stimulation pour mettre en œuvre, lors de la détection du sommeil, une stimulation, la stimulation étant maintenue jusqu'à au moins l'un parmi :
le premier capteur (7010) détecte des informations physiologiques indiquant un état de veille ; et
l'arrêt manuel de la stimulation.

12. Dispositif de soins TRS selon la revendication 11, dans lequel l'élément de stimulation est destiné à mettre en œuvre une stimulation par augmentation de la stimulation d'un niveau d'intensité inférieur à un niveau d'intensité cible.

13. Dispositif de soins TRS selon l'une des revendications 11 ou 12, dans lequel le premier capteur implantable (7010) est destiné à détecter des informations physiologiques, notamment des informations respiratoires détectées et des informations cardiaques détectées, le dispositif de soins TRS étant destiné à déterminer l'état sommeil-veille via au moins l'une parmi des informations respiratoires détectées et des informations cardiaques détectées, et
éventuellement dans lequel le premier capteur implantable (7010) est destiné à détecter les informations physiologiques pour inclure des informations de posture, et le dispositif de soins TRS étant destiné à déterminer l'état de sommeil-veille via au moins l'une parmi les informations de posture détectées, les informations respiratoires détectées et les informations cardiaques détectées.

14. Dispositif de soins TRS selon l'une des revendications 1 à 13, dans lequel le premier capteur implantable (7010) est destiné à détecter les informations physiologiques via la détection d'au moins la variabilité d'au moins l'une parmi les informations respiratoires comprenant une fréquence respiratoire, les informations cardiaques comprenant une fréquence cardiaque, une posture et un mouvement corporel ; et
le dispositif de soins TRS étant destiné à déterminer l'état de sommeil-veille via la détermination de la variabilité d'au moins l'une parmi la fréquence respiratoire détectée respective, les informations cardiaques détectées comprenant la fréquence cardiaque, la posture détectée et le mouvement corporel détecté.

15. Dispositif de soins TRS selon la revendication 14, le dispositif de soins TRS étant destiné à traiter les informations physiologiques détectées pour faire la distinction entre :
une première variabilité des informations respiratoires et des informations cardiaques qui est caractéristique des troubles respiratoires du sommeil ; et
une deuxième variabilité des informations respiratoires et des informations cardiaques qui est différente de la première variabilité caractéristique des troubles respiratoires du sommeil.
